(19) Europäisches Patentamt
European Patent Office
Office européen des brevets

(11) EP 1 688 496 A2

(12) # EUROPEAN PATENT APPLICATION

(43) Date of publication:
**09.08.2006 Bulletin 2006/32**

(21) Application number: **06000083.3**

(22) Date of filing: **10.08.2001**

(51) Int Cl.:
***C12N 15/12*** *(2006.01)* ***C12N 15/63*** *(2006.01)*
***C12N 1/19*** *(2006.01)* ***C12N 15/11*** *(2006.01)*
***C12N 15/62*** *(2006.01)* ***C07K 14/705*** *(2006.01)*
***C07K 16/28*** *(2006.01)* ***C12Q 1/68*** *(2006.01)*
***G01N 33/68*** *(2006.01)* ***A61K 31/7088*** *(2006.01)*
***A61K 38/17*** *(2006.01)* ***A61K 39/395*** *(2006.01)*
***A61K 48/00*** *(2006.01)*

(84) Designated Contracting States:
**AT BE CH CY DE DK ES FI FR GB GR IE IT LI LU MC NL PT SE TR**

(30) Priority: **14.08.2000 US 224989 P**

(62) Document number(s) of the earlier application(s) in accordance with Art. 76 EPC:
**01976066.9 / 1 311 677**

(71) Applicant: **Actimis Pharmaceuticals, Inc.,**
**La Jolla CA 92037 (US)**

(72) Inventor: **Ramakrishnan, Shyam**
**Brighton, MA 02135 (US)**

(74) Representative: **Jones Day**
**Rechtsanwälte, Attorneys-at-Law, Patentanwälte**
**Prinzregentenstrasse 11**
**80538 München (DE)**

Remarks:
This application was filed on 03 - 01 - 2006 as a divisional application to the application mentioned under INID code 62.

(54) **Regulation of human P2Y1-like G protein-coupled receptor**

(57) Use of a screening method, said method for the identification of compounds useful in the treatment of chronic obstructive pulmonary or airway disease ("COPD")comprising the step of contacting a test compound with a polypeptide.

EP 1 688 496 A2

**Description**

## TECHNICAL FIELD OF THE INVENTION

The invention relates to the area of G protein-coupled receptors. More particularly, it relates to the area of P2X1-like G protein-coupled receptors and their regulation.

## BACKGROUND OF THE INVENTION

### *G Protein-Coupled Receptors*

Many medically significant biological processes are mediated by signal transduction pathways that involve G proteins (Lefkowitz, *Nature 351,* 353-354, 1991). The family of G protein-coupled receptors (GPCR) includes receptors for hormones, neurotransmitters, growth factors, and viruses. Specific examples of GPCRs include receptors for such diverse agents as dopamine, calcitonin, adrenergic hormones, endothelin, cAMP, adenosine, acetylcholine, serotonin, histamine, thrombin, kinin, follicle stimulating hormone, opsins, endothelial differentiation gene-1, rhodopsins, odorants, cytomegalovirus, G proteins themselves, effector proteins such as phospholipase C, adenyl cyclase, and phosphodiesterase, and actuator proteins such as protein kinase A and protein kinase C.

GPCRs possess seven conserved membrane-spanning domains connecting at least eight divergent hydrophilic loops. GPCRs (also known as 7TM receptors) have been characterized as including these seven conserved hydrophobic stretches of about 20 to 30 amino acids, connecting at least eight divergent hydrophilic loops. Most GPCRs have single conserved cysteine residues in each of the first two extracellular loops, which form disulfide bonds that are believed to stabilize functional protein structure. The seven transmembrane regions are designated as TM1, TM2, TM3, TM4, TM5, TM6, and TM7. TM3 has been implicated in signal transduction.

Phosphorylation and lipidation (palmitylation or farnesylation) of cysteine residues can influence signal transduction of some GPCRs. Most GPCRs contain potential phosphorylation sites within the third cytoplasmic loop and/or the carboxy terminus. For several GPCRs, such as the β-adrenergic receptor, phosphorylation by protein kinase A and/or specific receptor kinases mediates receptor desensitization.

For some receptors, the ligand binding sites of GPCRs are believed to comprise hydrophilic sockets formed by several GPCR transmembrane domains. The hydrophilic sockets are surrounded by hydrophobic residues of the GPCRs. The hydrophilic side of each GPCR transmembrane helix is postulated to face inward and form a polar ligand binding site. TM3 has been implicated in several GPCRs as having a ligand binding site, such as the TM3 aspartate residue. TM5 serines, a TM6 asparagine, and TM6 or TM7 phenylalanines or tyrosines also are implicated in ligand binding.

GPCRs are coupled inside the cell by heterotrimeric G-proteins to various intracellular enzymes, ion channels, and transporters *(see* Johnson *et al., Endoc. Rev.* 10, 317-331, 1989). Different G-protein alpha-subunits preferentially stimulate particular effectors to modulate various biological functions in a cell. Phosphorylation of cytoplasmic residues of GPCRs is an important mechanism for the regulation of some GPCRs. For example, in one form of signal transduction, the effect of hormone binding is the activation inside the cell of the enzyme, adenylate cyclase. Enzyme activation by hormones is dependent on the presence of the nucleotide GTP. GTP also influences hormone binding. A G protein connects the hormone receptor to adenylate cyclase. G protein exchanges GTP for bound GDP when activated by a hormone receptor. The GTP-carrying form then binds to activated adenylate cyclase. Hydrolysis of GTP to GDP, catalyzed by the G protein itself, returns the G protein to its basal, inactive form. Thus, the G protein serves a dual role, as an intermediate that relays the signal from receptor to effector, and as a clock that controls the duration of the signal.

Over the past 15 years, nearly 350 therapeutic agents targeting GPCRs have been successfully introduced onto the market. This indicates that these receptors have an established, proven history as therapeutic targets. Clearly, there is an on-going need for identification and characterization of further GPCRs which can play a role in preventing, ameliorating, or correcting dysfunctions or diseases including, but not limited to, infections such as bacterial, fungal, protozoan, and viral infections, particularly those caused by HIV viruses, pain, cancers, anorexia, bulimia, asthma, Parkinson's diseases, acute heart failure, hypotension, hypertension, urinary retention, osteoporosis, angina pectoris, myocardial infarction, ulcers, asthma, allergies, multiple sclerosis, benign prostatic hypertrophy, and psychotic and neurological disorders, including anxiety, schizophrenia, manic depression, delirium, dementia, several mental retardation, and dyskinesias, such as Huntington's disease and Tourett's syndrome.

### *P2Y Receptors*

Adenosine 5'-triphosphate (ATP) has many different physiological functions in the cell. For example, ATP is the energy source for many biochemical reactions, a precursor for ribonucleic acid (RNA) synthesis, the precursor for cyclic AMP synthesis, etc. ATP also functions as an extracellular messenger in neuronal and non-neuronal tissues. Extracellular ATP exerts its effects on these tissues by acting through membrane-associated purinoreceptors (Burnstock, G. Ann. NY Acad. Sci. (1990) 603:1-17). The purinoreceptors can be either ligand-gated ion channels (Bean, B. P. (1992) Trends Pharmac. Sci. 12:87-90; Bean, B. P. and Fried, D. D. (1990) Ion Channels 2:169-203) that are generally referred to as P2X receptors, (but also known as: purinergic channels, P2X R-channels, and ATP-gated channels) or G-protein-coupled (P2Y or P2V) receptors (Barnard, E. A. et al. (1994) Trends Pharmac. Sci. 15:67-70). See U.S. Patent 5,856,129.

P2Y1 receptors are abundant in brain (Filippov *et al., Br. J. Pharmacol.* 129, 1063-66, 2000) and are found in a variety of other locations, including vascular smooth muscle (Erlinge *et al., Biochem. Biophys. Res. Commun. 248,* 864-70, 2998), neonatal rat cardiac fibroblasts *(Zheng et al., Cardiovasc. Res. 37,* 718-28, 1998), and neonatal and adult cardiac myocytes (Webb *et al., J. Auton. Pharmacol. 16,* 303-07, 1996).

Because of the wide-spread distribution of GPCRs with diverse biological effects, including P2Y receptors, there is a need in the art to identify additional members of the GPCR family whose activity can be regulated to provide therapeutic effects.

## SUMMARY OF THE INVENTION

It is an object of the invention to provide reagents and methods of regulating a human P2Y1-like G protein-coupled receptor. This and other objects of the invention are provided by one or more of the embodiments described below.

One embodiment of the invention is a P2Y1-like GPCR polypeptide comprising an amino acid sequence selected from the group consisting of:

amino acid sequences which are at least about 50% identical to the amino acid sequence shown in SEQ ID NO: 2; and
the amino acid sequence shown in SEQ ID NO: 2.

Yet another embodiment of the invention is a method of screening for agents which decrease extracellular matrix degradation. A test compound is contacted with a P2Y1-like GPCR polypeptide comprising an amino acid sequence selected from the group consisting of:

amino acid sequences which are at least about 50% identical to the amino acid sequence shown in SEQ ID NO: 2; and
the amino acid sequence shown in SEQ ID NO: 2.

Binding between the test compound and the P2Y1-like GPCR polypeptide is detected. A test compound which binds to the P2Y1-like GPCR polypeptide is thereby identified as a potential agent for decreasing extracellular matrix degradation.
The agent can work by decreasing the activity of the P2Y1-like GPCR.

Another embodiment of the invention is a method of screening for agents which decrease extracellular matrix degradation. A test compound is contacted with a polynucleotide encoding a P2Y1-like GPCR polypeptide, wherein the polynucleotide comprises a nucleotide sequence selected from the group consisting of:

nucleotide sequences which are at least about 50% identical to the nucleotide sequence shown in SEQ ID NO: 1;
the nucleotide sequence shown in SEQ ID NO: 1;
nucleotide sequences which are at least about 50% identical to the nucleotide sequence shown in SEQ ID NO: 3;
the nucleotide sequence shown in SEQ ID NO: 3;
nucleotide sequences which are at least about 50% identical to the nucleotide sequence shown in SEQ ID NO: 4;
the nucleotide sequence shown in SEQ ID NO: 4;
nucleotide sequences which are at least about 50% identical to the nucleotide sequence shown in SEQ ID NO: 5; and
the nucleotide sequence shown in SEQ ID NO:5.

Binding of the test compound to the polynucleotide is detected. A test compound which binds to the polynucleotide is identified as a potential agent for decreasing extracellular matrix degradation. The agent can work by decreasing the amount of the P2Y1-like GPCR through interacting with the P2Y1-like GPCR mRNA.

Another embodiment of the invention is a method of screening for agents which regulate extracellular matrix degradation. A test compound is contacted with a P2Y1-like GPCR polypeptide comprising an amino acid sequence selected from the group consisting of:

amino acid sequences which are at least about 50% identical to the amino acid sequence shown in SEQ ID NO: 2; and
the amino acid sequence shown in SEQ ID NO: 2.

A P2Y1-like GPCR activity of the polypeptide is detected. A test compound which increases P2Y1-like GPCR activity of the polypeptide relative to P2Y1-like GPCR activity in the absence of the test compound is thereby identified as a potential agent for increasing extracellular matrix degradation. A test compound which decreases P2Y1-like GPCR activity of the polypeptide relative to P2Y1-like GPCR activity in the absence of the test compound is thereby identified as a potential agent for decreasing extracellular matrix degradation.

Even another embodiment of the invention is a method of screening for agents which decrease extracellular matrix degradation. A test compound is contacted with a P2Y1-like GPCR product of a polynucleotide which comprises a nucleotide sequence selected from the group consisting of:

nucleotide sequences which are at least about 50% identical to the nucleotide sequence shown in SEQ ID NO: 1;
the nucleotide sequence shown in SEQ ID NO: 1;
nucleotide sequences which are at least about 50% identical to the nucleotide sequence shown in SEQ ID NO: 3;
the nucleotide sequence shown in SEQ ID NO: 3;
nucleotide sequences which are at least about 50% identical to the nucleotide sequence shown in SEQ ID NO: 4;
the nucleotide sequence shown in SEQ ID NO: 4;
nucleotide sequences which are at least about 50% identical to the nucleotide sequence shown in SEQ ID NO: 5; and
the nucleotide sequence shown in SEQ ID NO:5.

Binding of the test compound to the P2Y1-like GPCR product is detected. A test compound which binds to the P2Y1-like GPCR product is thereby identified as a potential agent for decreasing extracellular matrix degradation.

Still another embodiment of the invention is a method of reducing extracellular matrix degradation. A cell is contacted with a reagent which specifically binds to a polynucleotide encoding a P2Yl-like GPCR polypeptide or the product encoded by the polynucleotide, wherein the polynucleotide comprises a nucleotide sequence selected from the group consisting of:

nucleotide sequences which are at least about 50% identical to the nucleotide sequence shown in SEQ ID NO: 1;
the nucleotide sequence shown in SEQ ID NO: 1;
nucleotide sequences which are at least about 50% identical to the nucleotide sequence shown in SEQ ID NO: 3;
the nucleotide sequence shown in SEQ ID NO: 3;
nucleotide sequences which are at least about 50% identical to the nucleotide sequence shown in SEQ ID NO: 4;
the nucleotide sequence shown in SEQ ID NO: 4;
nucleotide sequences which are at least about 50% identical to the nucleotide sequence shown in SEQ ID NO: 5; and
the nucleotide sequence shown in SEQ ID NO:5.

P2Y1-like GPCR activity in the cell is thereby decreased.

The invention thus provides a P2Y1-like G protein-coupled receptor which can be used to identify test compounds which may act as agonists or antagonists at the receptor site and which can be regulated to provide therapeutic effects.

## BRIEF DESCRIPTION OF THE DRAWING

Fig. 1 shows the DNA-sequence encoding a P2Y1-like GPCR polypeptide (SEQ ID NO: 1).

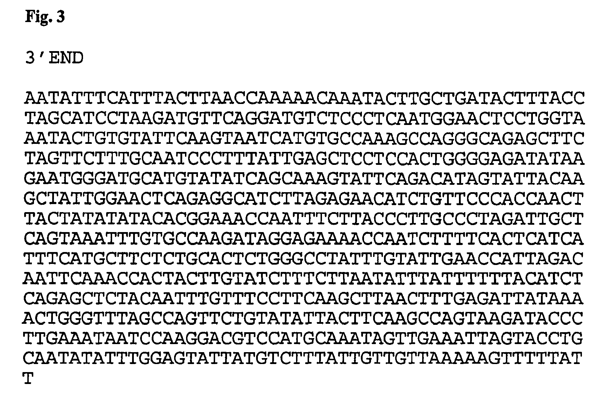

Fig. 2 shows the amino acid sequence deduced from the DNA-sequence of Fig.1 (SEQ ID NO:2).
Fig. 3 shows the DNA-sequence encoding a P2Y1-like GPCR polypeptide (SEQ ID NO:3).

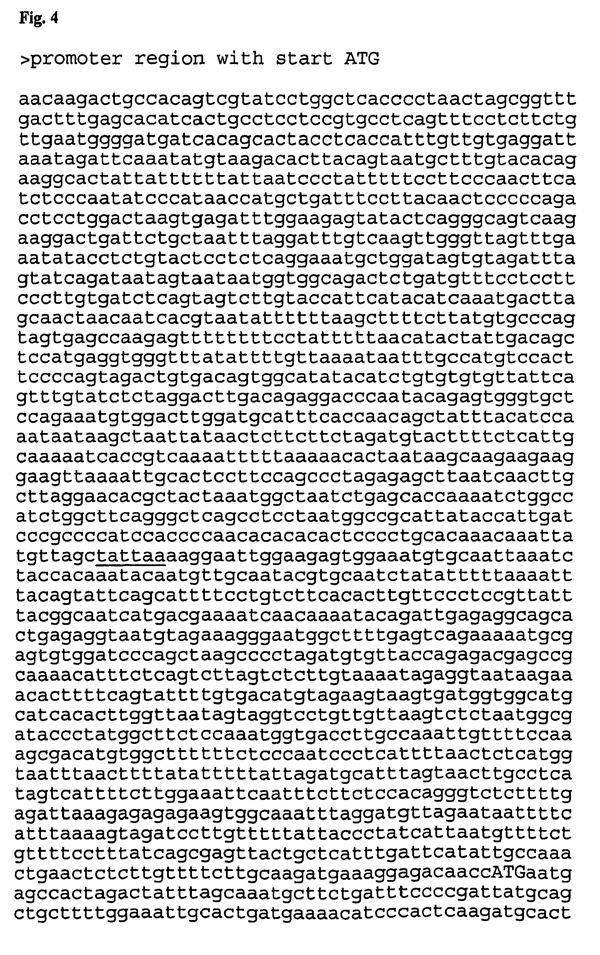

Fig. 4 shows the DNA-sequence encoding a P2Y1-like GPCR polypeptide (SEQ ID NO:4).

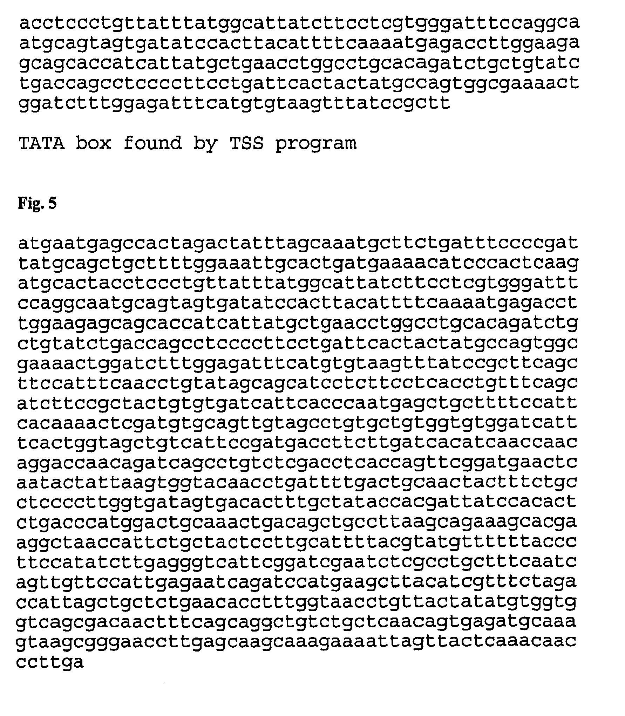

Fig. 5 shows the DNA-sequence encoding a P2Y1-like GPCR polypeptide (SEQ ID NO:5).

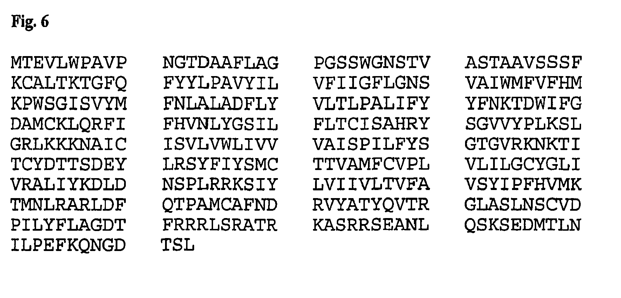

Fig. 6 shows the amino acid sequence of the protein identified by SwissProt Accession No. P49650 (SEQ ID NO:6).

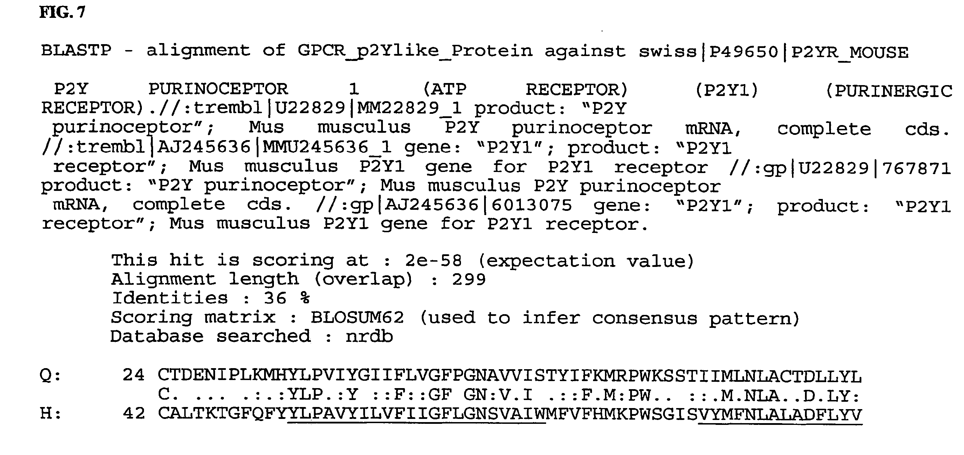

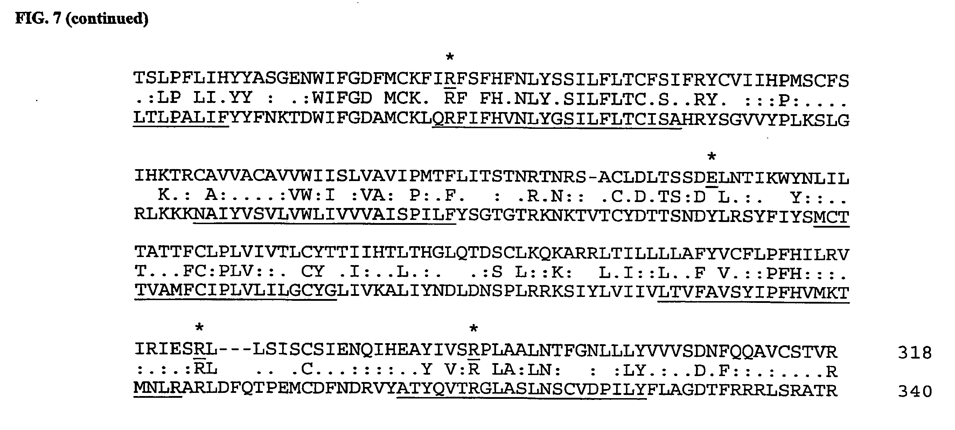

Fig. 7 shows the BLASTP alignment of human P2Y1-like GPCR (SEQ ID NO:2) and the protein identified by SwissProt Accession No. P49650 (SEQ ID NO:6). Transmembrane domains on the sequence are highlited in bold and underlined. P2Y1 receptors having F226A, K280A, or Q307A mutations, do not bind the antagonist, 2'-deoxy-N6-methyladenosine 3', 5'-bisphosphate (MRS 2179), indicating that these residues are critical for the binding of the antagonist molecule. P2Y1-like GPCR is missing these three sites. Three sites which are critical for binding of ligands in human, two are conserved as shown by bold, underlined (no italics) residues on the query sequence. Mutations in each of these residues individually leads to loss of binding. Thus residues on the exofacial side of TM3 and TM7 are critical determinants of the ATP binding pocket. ATP may be docked in the receptor, both within the previously defined TM cleft and within two other regions of the receptor, termed meta-binding sites, defined by the extracellular loops. The first meta-binding site is located outside of the TM bundle, between EL2 and EL3, and the second higher energy site is positioned immediately underneath EL2. Binding at both the principal TM binding site and the lower energy meta-binding sites potentially affects the ligand potency. In meta-binding site I, the side chain of Glu (EL2) (Bold,Italic,Underlined, between TM domain 4 and 5) is within hydrogen-bonding distance(2.8 A) of the ribose O3', and Arg (EL3) (Bold,Italic,Underlined, between TM domain 6 and 7) coordinate both alpha- and beta-phosphates of the triphosphate chain.

Fig. 8 shows the relative expression of human P2Y1-like G protein-coupled receptor in various human tissues and the neutrophil-like cell line HL60.

Fig. 9 shows the relative expression of human P2Y1-like G protein-coupled receptor in respiratory cells and tissues.

Fig. 10 shows the relative expression of human P2Y1-like GPCR in various human tissues.

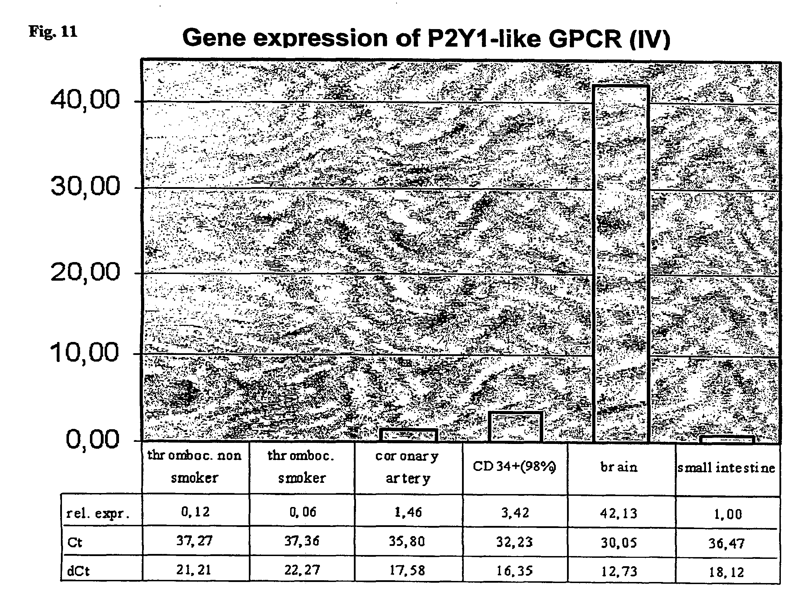

Fig. 11 shows the relative expression of human P2Y1-like GPCR in human thrombocytes of non-smokers and smokers, in coronary arteries, in brain and in small instestine.

## DETAILED DESCRIPTION OF THE INVENTION

The invention relates to an isolated polynucleotide encoding a P2Y1-like GPCR polypeptide and being selected from the group consisting of:

a) a polynucleotide encoding a P2Y1-like GPCR polypeptide comprising an amino acid sequence selected from the group consisting of:

amino acid sequences which are at least about 50% identical to
the amino acid sequence shown in SEQ ID NO: 2; and
the amino acid sequence shown in SEQ ID NO: 2.

b) a polynucleotide comprising the sequence of SEQ ID NOS: 1, 3, 4 or 5;
c) a polynucleotide which hybridizes under stringent conditions to a polynucleotide specified in (a) and (b);
d) a polynucleotide the sequence of which deviates from the polynucleotide sequences specified in (a) to (c) due to the degeneration of the genetic code; and
e) a polynucleotide which represents a fragment, derivative or allelic variation of a polynucleotide sequence specified in (a) to (d).

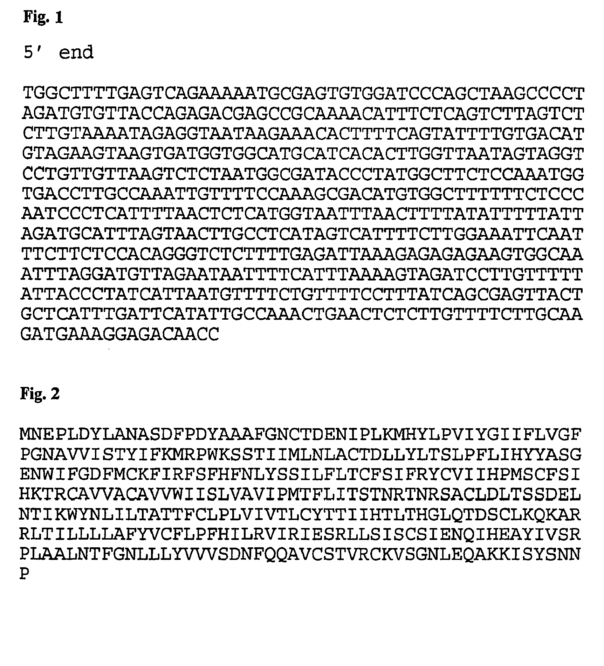

Furthermore, it has been discovered by the present applicant that a novel P2Y1-like GPCR, particularly a human P2Y1-like GPCR, is a discovery of the present invention. Human P2Y1-like GPCR has the amino acid sequence shown in SEQ ID NO:2. Using the BLASTP alignment program, this amino acid sequence is 36% identical over 299 amino acids to the mouse protein identified by SwissProt Accession No. P49650 (SEQ ID NO:6) and annotated as a "P2Y purinoceptor 1 (ATP receptor) (P2Y1)" (FIG. 1). Human P2Y1-like GPCR is therefore expected to bind a ligand to produce a biological effect or activity, such as cyclic AMP formation, mobilization of intracellular calcium, or phosphoinositide metabolism. Transmembrane domains of human P2Y1-like GPCR are shown in bold in FIG. 1.

Disorders such as bacterial, fungal, protozoan, and viral infections, particularly those caused by HIV viruses, pain, cancers, anorexia, bulimia, asthma, cardiovascular diseases such as acute heart failure, hypotension, hypertension, angina pectoris, and myocardial infarction, urinary retention, osteoporosis, diabetes, COPD, inflammation, ulcers, asthma, allergies, multiple sclerosis, benign prostatic hypertrophy, and psychotic and neurological disorders, including anxiety, schizophrenia, manic depression, delirium, dementia, several mental retardation, and dyskinesias, such as Parkinson's disease, Huntington's disease, and Tourett's syndrome can be treated by regulating human P2Yl-like GPCR Human P2Y1-like GPCR also can be used to screen for human P2Y1-like GPCR agonists and antagonists.

*Polypeptides*

P2Y1-like GPCR polypeptides according to the invention comprise at least 10, 12, 15, 20, 24, 30, 40, 50, 75, 100, 125, 150, 175, 200, 225, 250, 275, 300, 325, or 350 contiguous amino acids selected from the amino acid sequence shown in SEQ ID NO:2 or a biologically active variant of that sequence, as defined below. A P2Y1-like GPCR polypeptide of the invention therefore can be a portion of a P2Y1-like GPCR, a full-length P2Y1-like GPCR, or a fusion protein comprising all or a portion of a P2Y1-like GPCR.

*Biologically Active Variants*

P2Y1-like GPCR polypeptide variants which are biologically active, *i.e.*, retain the ability to bind a ligand to produce a biological effect, such as cyclic AMP formation, mobilization of intracellular calcium, or phosphoinositide metabolism, also are P2Y1-like GPCR polypeptides. Preferably, naturally or non-naturally occurring P2Y1-like GPCR polypeptide variants have amino acid sequences which are at least about 50, 55, 60, 65, 70, more preferably about 75, 90, 96, or 98% identical to an amino acid sequence shown in SEQ ID NO:2 or a fragment thereof. Percent identity between a putative P2Y1-like GPCR polypeptide variant and an amino acid sequence of SEQ ID NO:2 is determined using the Blast2 alignment program.

Variations in percent identity can be due, for example, to amino acid substitutions, insertions, or deletions. Amino acid substitutions are defined as one for one amino acid replacements. They are conservative in nature when the substituted amino acid has similar structural and/or chemical properties. Examples of conservative replacements are substitution of a leucine with an isoleucine or valine, an aspartate with a glutamate, or a threonine with a serine.

Amino acid insertions or deletions are changes to or within an amino acid sequence. They typically fall in the range of about 1 to 5 amino acids. Guidance in determining which amino acid residues can be substituted, inserted, or deleted without abolishing biological or immunological activity of a P2Y1-like GPCR polypeptide can be found using computer programs well known in the art, such as DNASTAR software. Whether an amino acid change results in a biologically active P2Y1-like GPCR polypeptide can readily be determined by assaying for binding to a ligand or by conducting a functional assay, as described for example, in the specific Examples, below.

*Fusion Proteins*

Fusion proteins are useful for generating antibodies against P2Y1-like GPCR polypeptide amino acid sequences and for use in various assay systems. For example, fusion proteins can be used to identify proteins which interact with portions of a P2Y1-like GPCR polypeptide. Protein affinity chromatography or library-based assays for protein-protein interactions, such as the yeast two-hybrid or phage display systems, can be used for this purpose. Such methods are well known in the art and also can be used as drug screens.

A P2Y1-like GPCR polypeptide fusion protein comprises two polypeptide segments fused together by means of a peptide bond. The first polypeptide segment comprises at least 10, 12, 15, 20, 24, 30, 40, 50, 75, 100, 125, 150, 175, 200, 225, 250, 275, 300, 325, or 350 contiguous amino acids of SEQ ID NO:2 or a biologically active variant of SEQ ID NO:2. Contiguous amino acids for use in a fusion protein can be selected from the amino acid sequence shown in SEQ ID NO:2 or from a biologically active variant of those sequences, such as those described above. The first polypeptide segment also can comprise full-length P2Y1-like G protein-coupled receptor.

The second polypeptide segment can be a full-length protein or a protein fragment. Proteins commonly used in fusion protein construction include β-galactosidase, β-glucuronidase, green fluorescent protein (GFP), autofluorescent proteins, including blue fluorescent protein (BFP), glutathione-S-transferase (GST), luciferase, horseradish peroxidase (HRP), and chloramphenicol acetyltransferase (CAT). Additionally, epitope tags are used in fusion protein constructions, including histidine (His) tags, FLAG tags, influenza hemagglutinin (HA) tags, Myc tags, VSV-G tags, and thioredoxin (Trx) tags. Other fusion constructions can include maltose binding protein (MBP), S-tag, Lex a DNA binding domain (DBD) fusions, GAL4 DNA binding domain fusions, and herpes simplex virus (HSV) BP16 protein fusions. A fusion protein also can be engineered to contain a cleavage site located between the P2Y1-like GPCR polypeptide-encoding sequence and the heterologous protein sequence, so that the P2Y1-like GPCR polypeptide can be cleaved and purified away from the heterologous moiety.

A fusion protein can be synthesized chemically, as is known in the art. Preferably, a fusion protein is produced by covalently linking two polypeptide segments or by standard procedures in the art of molecular biology. Recombinant DNA methods can be used to prepare fusion proteins, for example, by making a DNA construct which comprises coding sequences selected from SEQ ID NO:7 in proper reading frame with nucleotides encoding the second polypeptide segment and expressing the DNA construct in a host cell, as is known in the art. Many kits for constructing fusion proteins are available from companies such as Promega Corporation (Madison, WI), Stratagene (La Jolla, CA), CLONTECH (Mountain View, CA), Santa Cruz Biotechnology (Santa Cruz, CA), MBL International Corporation (MIC; Watertown, MA), and Quantum Biotechnologies (Montreal, Canada; 1-888-DNA-KITS).

*Identification of Species Homologs*

Species homologs of human P2Y1-like GPCR polypeptide can be obtained using P2Y1-like GPCR polynucleotides (described below) to make suitable probes or primers for screening cDNA expression libraries from other species, such as mice, monkeys, or yeast, identifying cDNAs which encode homologs of P2Y1-like GPCR polypeptide, and expressing the cDNAs as is known in the art.

*Polynucleotides*

A P2Y1-like GPCR polynucleotide can be single- or double-stranded and comprises a coding sequence or the complement of a coding sequence for a P2Y1-like GPCR polypeptide. A nucleotide sequence encoding SEQ ID NO:2 is shown in SEQ ID NO:5. The 5' and 3' ends of the human P2Y1-like GPCR gene are shown in SEQ ID NOS:1 and 3, respectively. The promoter region with the start ATG is shown in SEQ ID NO:4.

Degenerate nucleotide sequences encoding human P2Y1-like GPCR polypeptides, as well as homologous nucleotide sequences which are at least about 50, 55, 60, 65, or 70, more preferably about 75, 90, 96, or 98% identical to a nucleotide sequence shown in SEQ ID NOS:1, 3, 4, or 5 or its complement also are P2Y1-like GPCR polynucleotides. Percent sequence identity between the sequences of two polynucleotides is determined using computer programs such as ALIGN which employ the FASTA algorithm, using an affine gap search with a gap open penalty of -12 and a gap extension penalty of -2. Complementary DNA (cDNA) molecules, species homologs, and variants of P2Y1-like GPCR polynucleotides which encode biologically active P2Y1-like GPCR polypeptides also are P2Y1-like GPCR polynucleotides.

*Identification of Polynucleotide Variants and Homologs*

Variants and homologs of the P2Y1-like GPCR polynucleotides described above also are C\P2Y1-like GPCR polynucleotides. Typically, homologous P2Y1-like GPCR polynucleotide sequences can be identified by hybridization of candidate polynucleotides to known P2Y1-like GPCR polynucleotides under stringent conditions, as is known in the art. For example, using the following wash conditions--2X SSC (0.3 M NaCl, 0.03 M sodium citrate, pH 7.0), 0.1% SDS, room temperature twice, 30 minutes each; then 2X SSC, 0.1% SDS, 50 °C once, 30 minutes; then 2X SSC, room temperature twice, 10 minutes each--homologous sequences can be identified which contain at most about 25-30% basepair mismatches. More preferably, homologous nucleic acid strands contain 15-25% basepair mismatches, even more preferably 5-15% basepair mismatches.

Species homologs of the P2Yl-like GPCR polynucleotides disclosed herein also can be identified by making suitable probes or primers and screening cDNA expression libraries from other species, such as mice, monkeys, or yeast. Human variants of P2Y1-like GPCR polynucleotides can be identified, for example, by screening human cDNA expression libraries. It is well known that the $T_m$ of a double-stranded DNA decreases by 1-1.5 °C with every 1% decrease in homology (Bonner *et al., J. Mol. Biol. 81,* 123 (1973). Variants of human P2Y1-like GPCR polynucleotides or P2Y1-like GPCR polynucleotides of other species can therefore be identified by hybridizing a putative homologous P2Y1-like GPCR polynucleotide with a polynucleotide having a nucleotide sequence of SEQ ID NO:1 or 7 or the complement thereof to form a test hybrid. The melting temperature of the test hybrid is compared with the melting temperature of a hybrid comprising polynucleotides having perfectly complementary nucleotide sequences, and the number or percent of basepair mismatches within the test hybrid is calculated.

Nucleotide sequences which hybridize to P2Y1-like GPCR polynucleotides or their complements following stringent hybridization and/or wash conditions also are P2Y1-like GPCR polynucleotides. Stringent wash conditions are well known and understood in the art and are disclosed, for example, in Sambrook *et al.*, MOLECULAR CLONING: A LABORATORY MANUAL, 2d ed., 1989, at pages 9.50-9.51.

Typically, for stringent hybridization conditions a combination of temperature and salt concentration should be chosen that is approximately 12-20 °C below the calculated $T_m$ of the hybrid under study. The $T_m$ of a hybrid between a P2Y1-like GPCR polynucleotide having a nucleotide sequence shown in SEQ ID NO:1, 3, 4, or 5 or the complement thereof and a polynucleotide sequence which is at least about 50, 55, 60, 65, 70, preferably about 75, 90, 96, or 98% identical to one of those nucleotide sequences can be calculated, for example, using the equation of Bolton and McCarthy, *Proc. Natl. Acad. Sci. U.S.A. 48,* 1390 (1962):

$$T_m = 81.5\ ^{o}C - 16.6(\log_{10}[Na^+]) + 0.41(\%G + C) - 0.63(\%formamide) - 600/l),$$

where *l* = the length of the hybrid in basepairs.

Stringent wash conditions include, for example, 4X SSC at 65 °C, or 50% formamide, 4X SSC at 42 °C, or 0.5X SSC, 0.1% SDS at 65 °C. Highly stringent wash conditions include, for example, 0.2X SSC at 65 °C.

*Preparation of Polynucleotides*

A P2Y1-like GPCR polynucleotide can be isolated free of other cellular components such as membrane components, proteins, and lipids. Polynucleotides can be made by a cell and isolated using standard nucleic acid purification techniques, or synthesized using an amplification technique, such as the polymerase chain reaction (PCR), or by using an automatic synthesizer. Methods for isolating polynucleotides are routine and are known in the art. Any such technique for obtaining a polynucleotide can be used to obtain isolated P2Y1-like GPCR polynucleotides. For example, restriction enzymes and probes can be used to isolate polynucleotide fragments which comprises P2Y1-like GPCR nucleotide sequences. Isolated polynucleotides are in preparations which are free or at least 70, 80, or 90% free of other molecules.

P2Y1-like GPCR cDNA molecules can be made with standard molecular biology techniques, using P2Y1-like GPCR mRNA as a template. P2Y1-like GPCR cDNA molecules can thereafter be replicated using molecular biology techniques known in the art and disclosed in manuals such as Sambrook *et al.* (1989). An amplification technique, such as PCR, can be used to obtain additional copies of polynucleotides of the invention, using either human genomic DNA or cDNA as a template.

Alternatively, synthetic chemistry techniques can be used to synthesizes P2Y1-like GPCR polynucleotides. The degeneracy of the genetic code allows alternate nucleotide sequences to be synthesized which will encode a P2Y1-like GPCR polypeptide having, for example, the amino acid sequence shown in SEQ ID NO:2 or a biologically active variant thereof.

*Extending Polynucleotides*

Various PCR-based methods can be used to extend the nucleic acid sequences encoding the disclosed portions of human P2Y1-like GPCR polypeptide to detect upstream sequences such as promoters and regulatory elements. For example, restriction-site PCR uses universal primers to retrieve unknown sequence adjacent to a known locus (Sarkar, *PCR Methods Applic.* 2, 318-322, 1993). Genomic DNA is first amplified in the presence of a primer to a linker sequence and a primer specific to the known region. The amplified sequences are then subjected to a second round of PCR with the same linker primer and another specific primer internal to the first one. Products of each round of PCR are transcribed with an appropriate RNA polymerase and sequenced using reverse transcriptase.

Inverse PCR also can be used to amplify or extend sequences using divergent primers based on a known region (Triglia *et al., Nucleic Acids Res. 16, 8186,* 1988). Primers can be designed using commercially available software, such as OLIGO 4.06 Primer Analysis software (National Biosciences Inc., Plymouth, Minn.), to be 22-30 nucleotides in length, to have a GC content of 50% or more, and to anneal to the target sequence at temperatures about 68-72 °C. The method uses several restriction enzymes to generate a suitable fragment in the known region of a gene. The fragment is then circularized by intramolecular ligation and used as a PCR template.

Another method which can be used is capture PCR, which involves PCR amplification of DNA fragments adjacent to a known sequence in human and yeast artificial chromosome DNA (Lagerstrom *et al., PCR Methods Applic. 1*, 111-119, 1991). In this method, multiple restriction enzyme digestions and ligations also can be used to place an engineered double-stranded sequence into an unknown fragment of the DNA molecule before performing PCR.

Another method which can be used to retrieve unknown sequences is that of Parker *et al., Nucleic Acids Res. 19*, 3055-3060, 1991). Additionally, PCR, nested primers, and PROMOTERFINDER libraries (CLONTECH, Palo Alto, Calif.) can be used to walk genomic DNA (CLONTECH, Palo Alto, Calif.). This process avoids the need to screen libraries and is useful in finding intron/exon junctions.

When screening for full-length cDNAs, it is preferable to use libraries that have been size-selected to include larger cDNAs. Randomly-primed libraries are preferable, in that they will contain more sequences which contain the 5' regions of genes. Use of a randomly primed library may be especially preferable for situations in which an oligo d(T) library does not yield a full-length cDNA. Genomic libraries can be useful for extension of sequence into 5' non-transcribed regulatory regions.

Commercially available capillary electrophoresis systems can be used to analyze the size or confirm the nucleotide sequence of PCR or sequencing products. For example, capillary sequencing can employ flowable polymers for electrophoretic separation, four different fluorescent dyes (one for each nucleotide) which are laser activated, and detection of the emitted wavelengths by a charge coupled device camera. Output/light intensity can be converted to electrical signal using appropriate software (e.g. GENOTYPER and Sequence NAVIGATOR, Perkin Elmer), and the entire process from loading of samples to computer analysis and electronic data display can be computer controlled. Capillary electrophoresis is especially preferable for the sequencing of small pieces of DNA which might be present in limited amounts in a particular sample.

*Obtaining Polypeptides*

P2Y1-like GPCR polypeptides can be obtained, for example, by purification from cells, by expression of P2Y1-like GPCR polynucleotides, or by direct chemical synthesis.

*Protein Purification*

P2Y1-like GPCR polypeptides can be purified from any cell which expresses the receptor, including host cells which have been transfected with P2Y1-like GPCR polynucleotides which express such polypeptides. A purified P2Y1-like GPCR polypeptide is separated from other compounds which normally associate with the P2Y1-like GPCR polypeptide in the cell, such as certain proteins, carbohydrates, or lipids, using methods well-known in the art. Such methods include, but are not limited to, size exclusion chromatography, ammonium sulfate fractionation, ion exchange chromatography, affinity chromatography, and preparative gel electrophoresis.

A P2Y1-like GPCR polypeptide can be conveniently isolated as a complex with its associated G protein, as described in the specific examples, below. A preparation of purified P2Y1-like GPCR polypeptides is at least 80% pure; preferably, the preparations are 90%, 95%, or 99% pure. Purity of the preparations can be assessed by any means known in the art, such as SDS-polyacrylamide gel electrophoresis.

*Expression of Polynucleotides*

To express a P2Y1-like GPCR polypeptide, a P2Y1-like GPCR polynucleotide can be inserted into an expression vector which contains the necessary elements for the transcription and translation of the inserted coding sequence. Methods which are well known to those skilled in the art can be used to construct expression vectors containing sequences encoding P2Y1-like GPCR polypeptides and appropriate transcriptional and translational control elements. These methods include *in vitro* recombinant DNA techniques, synthetic techniques, and *in vivo* genetic recombination. Such techniques are described, for example, in Sambrook *et al.* (1989) and in Ausubel *et al.*, CURRENT PROTOCOLS IN MOLECULAR BIOLOGY, John Wiley & Sons, New York, N.Y., 1989.

A variety of expression vector/host systems can be utilized to contain and express sequences encoding a P2Y1-like GPCR polypeptide. These include, but are not limited to, microorganisms, such as bacteria transformed with recombinant bacteriophage, plasmid, or cosmid DNA expression vectors; yeast transformed with yeast expression vectors, insect cell systems infected with virus expression vectors (e.g., baculovirus), plant cell systems transformed with virus expression vectors *(e.g.,* cauliflower mosaic virus, CaMV; tobacco mosaic virus, TMV) or with bacterial expression vectors (*e.g.*, Ti or pBR322 plasmids), or animal cell systems.

The control elements or regulatory sequences are those non-translated regions of the vector -- enhancers, promoters, 5' and 3' untranslated regions -- which interact with host cellular proteins to carry out transcription and translation. Such elements can vary in their strength and specificity. Depending on the vector system and host utilized, any number of suitable transcription and translation elements, including constitutive and inducible promoters, can be used. For example, when cloning in bacterial systems, inducible promoters such as the hybrid lacZ promoter of the BLUESCRIPT phagemid (Stratagene, LaJolla, Calif.) or pSPORT1 plasmid (Life Technologies) and the like can be used. The baculovirus polyhedrin promoter can be used in insect cells. Promoters or enhancers derived from the genomes of plant cells (*e.g.*, heat shock, RUBISCO, and storage protein genes) or from plant viruses (*e.g.*, viral promoters or leader sequences) can be cloned into the vector. In mammalian cell systems, promoters from mammalian genes or from mammalian viruses are preferable. If it is necessary to generate a cell line that contains multiple copies of a nucleotide sequence encoding a P2Y1-like GPCR polypeptide, vectors based on SV40 or EBV can be used with an appropriate selectable marker.

*Bacterial and Yeast Expression Systems*

In bacterial systems, a number of expression vectors can be selected depending upon the use intended for the P2Y1-like GPCR polypeptide. For example, when a large quantity of a P2Y1-like GPCR polypeptide is needed for the induction of antibodies, vectors which direct high level expression of fusion proteins that are readily purified can be used. Such vectors include, but are not limited to, multifunctional *E. coli* cloning and expression vectors such as BLUESCRIPT (Stratagene). In a BLUESCRIPT vector, a sequence encoding the P2Y1-like GPCR polypeptide can be ligated into the vector in frame with sequences for the amino-terminal Met and the subsequent 7 residues of β-galactosidase so that a hybrid protein is produced. pIN vectors (Van Heeke & Schuster, *J. Biol. Chem. 264,* 5503-5509, 1989) or pGEX vectors (Promega, Madison, Wis.) also can be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems can be designed to include heparin, thrombin, or factor Xa protease cleavage sites so that the cloned polypeptide of interest can be released from the GST moiety at will.

In the yeast *Saccharomyces cerevisiae*, a number of vectors containing constitutive or inducible promoters such as alpha factor, alcohol oxidase, and PGH can be used. For reviews, see *Ausubel et al.* (1989) and Grant *et al., Methods Enzymol. 153,* 516-544,1987.

*Plant and Insect Expression Systems*

If plant expression vectors are used, the expression of sequences encoding P2Y1-like GPCR polypeptides can be driven by any of a number of promoters. For example, viral promoters such as the 35S and 19S promoters of CaMV can be used alone or in combination with the omega leader sequence from TMV (Takamatsu, *EMBO J. 6,* 307-311, 1987). Alternatively, plant promoters such as the small subunit of RUBISCO or heat shock promoters can be used (Coruzzi *et al., EMBO J. 3,* 1671-1680, 1984; Broglie *et al., Science 224,* 838-843, 1984; Winter *et al., Results Probl. Cell Differ. 17*, 85-105, 1991). These constructs can be introduced into plant cells by direct DNA transformation or by pathogen-mediated transfection. Such techniques are described in a number of generally available reviews (e.g., Hobbs or Murray, in MCGRAW HILL YEARBOOK OF SCIENCE AND TECHNOLOGY, McGraw Hill, New York, N.Y., pp. 191-196, 1992).

An insect system also can be used to express a P2Y1-like GPCR polypeptide. For example, in one such system *Autographa californica* nuclear polyhedrosis virus (AcNPV) is used as a vector to express foreign genes in *Spodoptera frugiperda* cells or in *Trichoplusia* larvae. Sequences encoding P2Y1-like GPCR polypeptides can be cloned into a non-essential region of the virus, such as the polyhedrin gene, and placed under control of the polyhedrin promoter. Successful insertion of P2Y1-like GPCR polypeptides will render the polyhedrin gene inactive and produce recombinant virus lacking coat protein. The recombinant viruses can then be used to infect *S. frugiperda cells* or *Trichoplusia* larvae in which P2Y1-like GPCR polypeptides can be expressed (Engelhard *et al., Proc. Nat. Acad. Sci. 91,* 3224-3227, 1994).

*Mammalian Expression Systems*

A number of viral-based expression systems can be used to express P2Y1-like GPCR polypeptides in mammalian host cells. For example, if an adenovirus is used as an expression vector, sequences encoding P2Y1-like GPCR polypeptides can be ligated into an adenovirus transcription/translation complex comprising the late promoter and tripartite leader sequence. Insertion in a non-essential E1 or E3 region of the viral genome can be used to obtain a viable virus which is capable of expressing a P2Y1-like GPCR polypeptide in infected host cells (Logan & Shenk, *Proc. Natl. Acad. Sci.* 81, 3655-3659, 1984). If desired, transcription enhancers, such as the Rous sarcoma virus (RSV) enhancer, can be used to increase expression in mammalian host cells.

Human artificial chromosomes (HACs) also can be used to deliver larger fragments of DNA than can be contained and expressed in a plasmid. HACs of 6M to 10M are constructed and delivered to cells via conventional delivery methods (e.g., liposomes, polycationic amino polymers, or vesicles).

Specific initiation signals also can be used to achieve more efficient translation of sequences encoding P2Y1-like GPCR polypeptides. Such signals include the ATG initiation codon and adjacent sequences. In cases where sequences encoding a P2Y1-like GPCR polypeptide, its initiation codon, and upstream sequences are inserted into the appropriate expression vector, no additional transcriptional or translational control signals may be needed. However, in cases where only coding sequence, or a fragment thereof, is inserted, exogenous translational control signals (including the ATG initiation codon) should be provided. The initiation codon should be in the correct reading frame to ensure translation of the entire insert. Exogenous translational elements and initiation codons can be of various origins, both natural and synthetic. The efficiency of expression can be enhanced by the inclusion of enhancers which are appropriate for the particular cell system which is used (see Scharf *et al.*, *Results Probl. Cell Differ. 20,* 125-162, 1994).

*Host Cells*

A host cell strain can be chosen for its ability to modulate the expression of the inserted sequences or to process the expressed P2Y1-like GPCR polypeptide in the desired fashion. Such modifications of the polypeptide include, but are not limited to, acetylation, carboxylation, glycosylation, phosphorylation, lipidation, and acylation. Post-translational processing which cleaves a "prepro" form of the polypeptide also can be used to facilitate correct insertion, folding and/or function. Different host cells which have specific cellular machinery and characteristic mechanisms for post-translational activities (*e.g.*, CHO, HeLa, MDCK, HEK293, and WI38), are available from the American Type Culture Collection (ATCC; 10801 University Boulevard, Manassas, VA 20110-2209) and can be chosen to ensure the correct modification and processing of the foreign protein.

Stable expression is preferred for long-term, high-yield production of recombinant proteins. For example, cell lines which stably express P2Y1-like GPCR polypeptides can be transformed using expression vectors which can contain viral origins of replication and/or endogenous expression elements and a selectable marker gene on the same or on a separate vector. Following the introduction of the vector, cells can be allowed to grow for 1-2 days in an enriched medium before they are switched to a selective medium. The purpose of the selectable marker is to confer resistance to selection, and its presence allows growth and recovery of cells which successfully express the introduced P2Y1-like GPCR sequences. Resistant clones of stably transformed cells can be proliferated using tissue culture techniques appropriate to the cell type. See, for example, ANIMAL CELL CULTURE, R.I. Freshney, ed., 1986.

Any number of selection systems can be used to recover transformed cell lines. These include, but are not limited to, the herpes simplex virus thymidine kinase (Wigler *et al., Cell 11*, 223-32, 1977) and adenine phosphoribosyltransferase (Lowy *et al., Cell 22,* 817-23, 1980) genes which can be employed in *tk*- or *aprt*- cells, respectively. Also, antimetabolite, antibiotic, or herbicide resistance can be used as the basis for selection. For example, *dhfr* confers resistance to methotrexate (Wigler *et al., Proc. Natl. Acad. Sci. 77,* 3567-70, 1980), *npt* confers resistance to the aminoglycosides, neomycin and G-418 (Colbere-Garapin *et al., J Mol. Biol. 150,* 1-14, 1981), and *als* and *pat* confer resistance to chlorsulfuron and phosphinotricin acetyltransferase, respectively (Murray, 1992, *supra*). Additional selectable genes have been described. For example, *trpB* allows cells to utilize indole in place of tryptophan, or *hisD*, which allows cells to utilize histinol in place of histidine (Hartman & Mulligan, *Proc. Natl. Acad Sci. 85,* 8047-51, 1988). Visible markers such as anthocyanins, β-glucuronidase and its substrate GUS, and luciferase and its substrate luciferin, can be used to identify transformants and to quantify the amount of transient or stable protein expression attributable to a specific vector system (Rhodes *et al., Methods Mol. Biol. 55*, 121-131, 1995).

*Detecting Expression of Polypeptides*

Although the presence of marker gene expression suggests that the P2Y1-like GPCR polynucleotide is also present, its presence and expression may need to be confirmed. For example, if a sequence encoding a P2Y1-like GPCR polypeptide is inserted within a marker gene sequence, transformed cells containing sequences which encode a P2Y1-like GPCR polypeptide can be identified by the absence of marker gene function. Alternatively, a marker gene can be placed in tandem with a sequence encoding a P2Y1-like GPCR polypeptide under the control of a single promoter. Expression of the marker gene in response to induction or selection usually indicates expression of the P2Y1-like GPCR polynucleotide.

Alternatively, host cells which contain a P2Y1-like GPCR polynucleotide and which express a P2Y1-like GPCR polypeptide can be identified by a variety of procedures known to those of skill in the art. These procedures include, but are not limited to, DNA-DNA or DNA-RNA hybridizations and protein bioassay or immunoassay techniques which include membrane, solution, or chip-based technologies for the detection and/or quantification of nucleic acid or protein. For example, the presence of a polynucleotide sequence encoding a P2Y1-like GPCR polypeptide can be detected by DNA-DNA or DNA-RNA hybridization or amplification using probes or fragments or fragments of polynucleotides encoding a P2Y1-like GPCR polypeptide. Nucleic acid amplification-based assays involve the use of oligonucleotides selected from sequences encoding a P2Y1-like GPCR polypeptide to detect transformants which contain a P2Y1-like GPCR polynucleotide.

A variety of protocols for detecting and measuring the expression of a P2Y1-like GPCR polypeptide, using either polyclonal or monoclonal antibodies specific for the polypeptide, are known in the art. Examples include enzyme-linked immunosorbent assay (ELISA), radioimmunoassay (RIA), and fluorescence activated cell sorting (FACS). A two-site, monoclonal-based immunoassay using monoclonal antibodies reactive to two non-interfering epitopes on a P2Y1-like GPCR polypeptide can be used, or a competitive binding assay can be employed. These and other assays are described in Hampton *et al*., SEROLOGICAL METHODS: A LABORATORY MANUAL, APS Press, St. Paul, Minn., 1990) and Maddox *et al., J. Exp. Med. 158*, 1211-1216, 1983).

A wide variety of labels and conjugation techniques are known by those skilled in the art and can be used in various nucleic acid and amino acid assays. Means for producing labeled hybridization or PCR probes for detecting sequences related to polynucleotides encoding P2Y1-like GPCR polypeptides include oligolabeling, nick translation, end-labeling, or PCR amplification using a labeled nucleotide. Alternatively, sequences encoding a P2Y1-like GPCR polypeptide can be cloned into a vector for the production of an mRNA probe. Such vectors are known in the art, are commercially available, and can be used to synthesize RNA probes *in vitro* by addition of labeled nucleotides and an appropriate RNA polymerase such as T7, T3, or SP6. These procedures can be conducted using a variety of commercially available kits (Amersham Pharmacia Biotech, Promega, and US Biochemical). Suitable reporter molecules or labels which can be used for ease of detection include radionuclides, enzymes, and fluorescent, chemiluminescent, or chromogenic agents, as well as substrates, cofactors, inhibitors, magnetic particles, and the like.

*Expression and Purification of Polypeptides*

Host cells transformed with nucleotide sequences encoding a P2Y1-like GPCR polypeptide can be cultured under conditions suitable for the expression and recovery of the protein from cell culture. The polypeptide produced by a transformed cell can be secreted or contained intracellularly depending on the sequence and/or the vector used. As will be understood by those of skill in the art, expression vectors containing polynucleotides which encode P2Yl-like GPCR polypeptides can be designed to contain signal sequences which direct secretion of soluble P2Y1-like GPCR polypeptides through a prokaryotic or eukaryotic cell membrane or which direct the membrane insertion of membrane-bound P2Y1-like GPCR polypeptide.

As discussed above, other constructions can be used to join a sequence encoding a P2Y1-like GPCR polypeptide to a nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). Inclusion of cleavable linker sequences such as those specific for Factor Xa or enterokinase (Invitrogen, San Diego, CA) between the purification domain and the P2Y1-like GPCR polypeptide also can be used to facilitate purification. One such expression vector provides for expression of a fusion protein containing a P2Y1-like GPCR polypeptide and 6 histidine residues preceding a thioredoxin or an enterokinase cleavage site. The histidine residues facilitate purification by IMAC (immobilized metal ion affinity chromatography, as described in *Porath et al., Prot. Exp. Purif. 3,* 263-281, 1992), while the enterokinase cleavage site provides a means for purifying the P2Y1-like GPCR polypeptide from the fusion protein. Vectors which contain fusion proteins are disclosed in Kroll *et al., DNA Cell Biol. 12*, 441-453, 1993.

*Chemical Synthesis*

Sequences encoding a P2Y1-like GPCR polypeptide can be synthesized, in whole or in part, using chemical methods well known in the art (see Caruthers *et al., Nucl. Acids Res. Symp. Ser.* 215-223, 1980; Horn *et al. Nucl. Acids Res. Symp. Ser*. 225-232, 1980). Alternatively, a P2Y1-like GPCR polypeptide itself can be produced using chemical methods to synthesize its amino acid sequence, such as by direct peptide synthesis using solid-phase techniques (Merrifield, *J. Am. Chem. Soc. 85*, 2149-2154, 1963; Roberge *et al.*, *Science 269*, 202-204, 1995). Protein synthesis can be performed using manual techniques or by automation. Automated synthesis can be achieved, for example, using Applied Biosystems 431A Peptide Synthesizer (Perkin Elmer). Optionally, fragments of P2Y1-like GPCR polypeptides can be separately synthesized and combined using chemical methods to produce a full-length molecule.

The newly synthesized peptide can be substantially purified by preparative high performance liquid chromatography *(e.g.,* Creighton, PROTEINS: STRUCTURES AND MOLECULAR PRINCIPLES, WH Freeman and Co., New York, N.Y., 1983). The composition of a synthetic P2Y1-like GPCR polypeptide can be confirmed by amino acid analysis or sequencing (e.g., the Edman degradation procedure; *see* Creighton, *supra*). Additionally, any portion of the amino acid sequence of the P2Y1-like GPCR polypeptide can be altered during direct synthesis and/or combined using chemical methods with sequences from other proteins to produce a variant polypeptide or a fusion protein.

*Production of Altered Polypeptides*

As will be understood by those of skill in the art, it may be advantageous to produce P2Y1-like GPCR polypeptide-encoding nucleotide sequences possessing non-naturally occurring codons. For example, codons preferred by a particular prokaryotic or eukaryotic host can be selected to increase the rate of protein expression or to produce an RNA transcript having desirable properties, such as a half-life which is longer than that of a transcript generated from the naturally occurring sequence.

The nucleotide sequences disclosed herein can be engineered using methods generally known in the art to alter P2Y1-like GPCR polypeptide-encoding sequences for a variety of reasons, including but not limited to, alterations which modify the cloning, processing, and/or expression of the polypeptide or mRNA product. DNA shuffling by random fragmentation and PCR reassembly of gene fragments and synthetic oligonucleotides can be used to engineer the nucleotide sequences. For example, site-directed mutagenesis can be used to insert new restriction sites, alter glycosylation patterns, change codon preference, produce splice variants, introduce mutations, and so forth.

*Antibodies*

Any type of antibody known in the art can be generated to bind specifically to an epitope of a P2Y1-like GPCR polypeptide. "Antibody" as used herein includes intact immunoglobulin molecules, as well as fragments thereof, such as Fab, $F(ab')_z$, and Fv, which are capable of binding an epitope of a P2Y1-like GPCR polypeptide. Typically, at least 6, 8, 10, or 12 contiguous amino acids are required to form an epitope. However, epitopes which involve non-contiguous amino acids may require more, e.g., at least 15, 25, or 50 amino acids.

An antibody which specifically binds to an epitope of a P2Yl-like GPCR polypeptide can be used therapeutically, as well as in immunochemical assays, such as Western blots, ELISAs, radioimmunoassays, immunohistochemical assays, immunoprecipitations, or other immunochemical assays known in the art. Various immunoassays can be used to identify antibodies having the desired specificity. Numerous protocols for competitive binding or immunoradiometric assays are well known in the art. Such immunoassays typically involve the measurement of complex formation between an immunogen and an antibody which specifically binds to the immunogen.

Typically, an antibody which specifically binds to a P2Y1-like GPCR polypeptide provides a detection signal at least 5-, 10-, or 20-fold higher than a detection signal provided with other proteins when used in an immunochemical assay. Preferably, antibodies which specifically bind to P2Y1-like GPCR polypeptides do not detect other proteins in immunochemical assays and can immunoprecipitate a P2Y1-like GPCR polypeptide from solution.

P2Y1-like GPCR polypeptides can be used to immunize a mammal, such as a mouse, rat, rabbit, guinea pig, monkey, or human, to produce polyclonal antibodies. If desired, a P2Y1-like GPCR polypeptide can be conjugated to a carrier protein, such as bovine serum albumin, thyroglobulin, and keyhole limpet hemocyanin. Depending on the host species, various adjuvants can be used to increase the immunological response. Such adjuvants include, but are not limited to, Freund's adjuvant, mineral gels (e.g., aluminum hydroxide), and surface active substances (e.g. lysolecithin, pluronic polyols, polyanions, peptides, oil emulsions, keyhole limpet hemocyanin, and dinitrophenol). Among adjuvants used in humans, BCG *(bacilli Calmette-Guerin)* and *Corynebacterium parvum* are especially useful.

Monoclonal antibodies which specifically bind to a P2Y1-like GPCR polypeptide can be prepared using any technique which provides for the production of antibody molecules by continuous cell lines in culture. These techniques include, but are not limited to, the hybridoma technique, the human B-cell hybridoma technique, and the EBV-hybridoma technique (Kohler *et al.*, *Nature 256,* 495-497, 1985; Kozbor *et al., J. Immunol. Methods 81,* 31-42, 1985; Cote *et al., Proc. Natl. Acad Sci. 80,* 2026-2030, 1983; Cole *et al., Mol. Cell Biol. 62,* 109-120, 1984).

In addition, techniques developed for the production of "chimeric antibodies," the splicing of mouse antibody genes to human antibody genes to obtain a molecule with appropriate antigen specificity and biological activity, can be used (Morrison *et al.*, *Proc. Natl. Acad. Sci. 81, 6851-6855,* 1984; Neuberger *et al., Nature 312,* 604-608, 1984; Takeda *et al., Nature 314,* 452-454, 1985). Monoclonal and other antibodies also can be "humanized" to prevent a patient from mounting an immune response against the antibody when it is used therapeutically. Such antibodies may be sufficiently similar in sequence to human antibodies to be used directly in therapy or may require alteration of a few key residues. Sequence differences between rodent antibodies and human sequences can be minimized by replacing residues which differ from those in the human sequences by site directed mutagenesis of individual residues or by grating of entire complementarity determining regions. Alternatively, humanized antibodies can be produced using recombinant methods, as described in GB2188638B. Antibodies which specifically bind to a P2Y1-like GPCR polypeptide can contain antigen binding sites which are either partially or fully humanized, as disclosed in U.S. 5,565,332.

Alternatively, techniques described for the production of single chain antibodies can be adapted using methods known in the art to produce single chain antibodies which specifically bind to P2Y1-like GPCR polypeptides. Antibodies with related specificity, but of distinct idiotypic composition, can be generated by chain shuffling from random combinatorial immunoglobin libraries (Burton, *Proc. Natl. Acad Sci. 88*, 11120-23, 1991).

Single-chain antibodies also can be constructed using a DNA amplification method, such as PCR, using hybridoma cDNA as a template (Thirion *et al.*, 1996, *Eur. J. Cancer Prev. 5*, 507-11). Single-chain antibodies can be mono- or bispecific, and can be bivalent or tetravalent. Construction of tetravalent, bispecific single-chain antibodies is taught, for example, in Coloma & Morrison, 1997, *Nat. Biotechnol. 15*, 159-63. Construction of bivalent, bispecific single-chain antibodies is taught in Mallender & Voss, 1994, *J. Biol. Chem. 269,199-206.*

A nucleotide sequence encoding a single-chain antibody can be constructed using manual or automated nucleotide synthesis, cloned into an expression construct using standard recombinant DNA methods, and introduced into a cell to express the coding sequence, as described below. Alternatively, single-chain antibodies can be produced directly using, for example, filamentous phage technology (Verhaar *et al.*, 1995, *Int. J. Cancer 61,* 497-501; Nicholls *et al.*, 1993, *J. Immunol. Meth. 165,* 81-91).

Antibodies which specifically bind to P2Y1-like GPCR polypeptides also can be produced by inducing *in vivo* production in the lymphocyte population or by screening immunoglobulin libraries or panels of highly specific binding reagents as disclosed in the literature (Orlandi *et al., Proc. Natl. Acad. Sci. 86*, 3833-3837, 1989; Winter *et al., Nature 349*, 293-299, 1991).

Other types of antibodies can be constructed and used therapeutically in methods of the invention. For example, chimeric antibodies can be constructed as disclosed in WO 93/03151. Binding proteins which are derived from immunoglobulins and which are multivalent and multispecific, such as the "diabodies" described in WO 94/13804, also can be prepared.

Antibodies according to the invention can be purified by methods well known in the art. For example, antibodies can be affinity purified by passage over a column to which a P2Y1-like GPCR polypeptide is bound. The bound antibodies can then be eluted from the column using a buffer with a high salt concentration.

*Antisense Oligonucleotides*

Antisense oligonucleotides are nucleotide sequences which are complementary to a specific DNA or RNA sequence. Once introduced into a cell, the complementary nucleotides combine with natural sequences produced by the cell to form complexes and block either transcription or translation. Preferably, an antisense oligonucleotide is at least 11 nucleotides in length, but can be at least 12, 15, 20, 25, 30, 35, 40, 45, or 50 or more nucleotides long. Longer sequences also can be used. Antisense oligonucleotide molecules can be provided in a DNA construct and introduced into a cell as described above to decrease the level of P2Y1-like GPCR gene products in the cell.

Antisense oligonucleotides can be deoxyribonucleotides, ribonucleotides, or a combination of both. Oligonucleotides can be synthesized manually or by an automated synthesizer, by covalently linking the 5' end of one nucleotide with the 3' end of another nucleotide with non-phosphodiester internucleotide linkages such alkylphosphonates, phosphorothioates, phosphorodithioates, alkylphosphonothioates, alkylphosphonates, phosphoramidates, phosphate esters, carbamates, acetamidate, carboxymethyl esters, carbonates, and phosphate triesters. *See* Brown, *Meth. Mol. Biol. 20,* 1-8, 1994; Sonveaux, *Meth. Mol. Biol. 26,* 1-72, 1994; Uhlmann *et al., Chem. Rev. 90*, 543-583, 1990.

Modifications of P2Y1-like GPCR gene expression can be obtained by designing antisense oligonucleotides which will form duplexes to the control, 5', or regulatory regions of the P2Y1-like GPCR. Oligonucleotides derived from the transcription initiation site, e.g., between positions -10 and +10 from the start site, are preferred. Similarly, inhibition can be achieved using "triple helix" base-pairing methodology. Triple helix pairing is useful because it causes inhibition of the ability of the double helix to open sufficiently for the binding of polymerases, transcription factors, or chaperons. Therapeutic advances using triplex DNA have been described in the literature *(e.g.,* Gee *et al*., in Huber & Carr, MOLECULAR AND IMMUNOLOGIC APPROACHES, Futura Publishing Co., Mt. Kisco, N.Y., 1994). An antisense oligonucleotide also can be designed to block translation of mRNA by preventing the transcript from binding to ribosomes.

Precise complementarity is not required for successful complex formation between an antisense oligonucleotide and the complementary sequence of a P2Y1-like GPCR polynucleotide. Antisense oligonucleotides which comprise, for example, 2, 3, 4, or 5 or more stretches of contiguous nucleotides which are precisely complementary to a P2Y1-like GPCR polynucleotide, each separated by a stretch of contiguous nucleotides which are not complementary to adjacent P2Y1-like GPCR nucleotides, can provide sufficient targeting specificity for P2Y1-like GPCR mRNA. Preferably, each stretch of complementary contiguous nucleotides is at least 4, 5, 6, 7, or 8 or more nucleotides in length. Non-complementary intervening sequences are preferably 1, 2, 3, or 4 nucleotides in length. One skilled in the art can easily use the calculated melting point of an antisense-sense pair to determine the degree of mismatching which will be tolerated between a particular antisense oligonucleotide and a particular P2Y1-like GPCR polynucleotide sequence.

Antisense oligonucleotides can be modified without affecting their ability to hybridize to a P2Y1-like GPCR polynucleotide. These modifications can be internal or at one or both ends of the antisense molecule. For example, internucleoside phosphate linkages can be modified by adding cholesteryl or diamine moieties with varying numbers of carbon residues between the amino groups and terminal ribose. Modified bases and/or sugars, such as arabinose instead of ribose, or a 3', 5'-substituted oligonucleotide in which the 3' hydroxyl group or the 5' phosphate group are substituted, also can be employed in a modified antisense oligonucleotide. These modified oligonucleotides can be prepared by methods well known in the art. *See, e.g., Agrawal et al., Trends Biotechnol. 10,* 152-158, 1992; Uhlmann *et al*., *Chem. Rev. 90*, 543-584, 1990; Uhlmann *et al., Tetrahedron. Lett. 215,* 3539-3542, 1987.

*Ribozymes*

Ribozymes are RNA molecules with catalytic activity. *See, e.g.,* Cech, *Science 236,* 1532-1539; 1987; Cech, *Ann. Rev. Biochem. 59,* 543-568; 1990, Cech, *Curr. Opin. Struct. Biol. 2,* 605-609; 1992, Couture & Stinchcomb, *Trends Genet. 12,* 510-515, 1996. Ribozymes can be used to inhibit gene function by cleaving an RNA sequence, as is known in the art *(e.g.,* Haseloff *et al.,* U.S. Patent 5,641,673). The mechanism of ribozyme action involves sequence-specific hybridization of the ribozyme molecule to complementary target RNA, followed by endonucleolytic cleavage. Examples include engineered hammerhead motif ribozyme molecules that can specifically and efficiently catalyze endonucleolytic cleavage of specific nucleotide sequences.

The coding sequence of a P2Y1-like GPCR polynucleotide can be used to generate ribozymes which will specifically bind to mRNA transcribed from the P2Y1-like GPCR polynucleotide. Methods of designing and constructing ribozymes which can cleave other RNA molecules in trans in a highly sequence specific manner have been developed and described in the art *(see* Haseloff *et al. Nature 334,* 585-591, 1988). For example, the cleavage activity of ribozymes can be targeted to specific RNAs by engineering a discrete "hybridization" region into the ribozyme. The hybridization region contains a sequence complementary to the target RNA and thus specifically hybridizes with the target (see, for example, Gerlach *et al*., EP 321,201).

Specific ribozyme cleavage sites within a P2Y1-like GPCR RNA target can be identified by scanning the target molecule for ribozyme cleavage sites which include the following sequences: GUA, GUU, and GUC. Once identified, short RNA sequences of between 15 and 20 ribonucleotides corresponding to the region of the target RNA containing the cleavage site can be evaluated for secondary structural features which may render the target inoperable. Suitability of candidate P2Y1-like GPCR RNA targets also can be evaluated by testing accessibility to hybridization with complementary oligonucleotides using ribonuclease protection assays. Longer complementary sequences can be used to increase the affinity of the hybridization sequence for the target. The hybridizing and cleavage regions of the ribozyme can be integrally related such that upon hybridizing to the target RNA through the complementary regions, the catalytic region of the ribozyme can cleave the target.

Ribozymes can be introduced into cells as part of a DNA construct. Mechanical methods, such as microinjection, liposome-mediated transfection, electroporation, or calcium phosphate precipitation, can be used to introduce a ribozyme-containing DNA construct into cells in which it is desired to decrease P2Y1-like GPCR expression. Alternatively, if it is desired that the cells stably retain the DNA construct, the construct can be supplied on a plasmid and maintained as a separate element or integrated into the genome of the cells, as is known in the art. A ribozyme-encoding DNA construct can include transcriptional regulatory elements, such as a promoter element, an enhancer or UAS element, and a transcriptional terminator signal, for controlling transcription of ribozymes in the cells.

As taught in Haseloff *et al*., U.S. Patent 5,641,673, ribozymes can be engineered so that ribozyme expression will occur in response to factors which induce expression of a target gene. Ribozymes also can be engineered to provide an additional level of regulation, so that destruction of mRNA occurs only when both a ribozyme and a target gene are induced in the cells.

## *Differentially Expressed Genes*

Described herein are methods for the identification of genes whose products interact with human P2Y1-like G protein-coupled receptor. Such genes may represent genes that are differentially expressed in disorders including, but not limited to, CNS disorders, cardiovascular disorders, asthma, osteoporosis, diabetes, and COPD. Further, such genes may represent genes that are differentially regulated in response to manipulations relevant to the progression or treatment of such diseases. Additionally, such genes may have a temporally modulated expression, increased or decreased at different stages of tissue or organism development. A differentially expressed gene may also have its expression modulated under control versus experimental conditions. In addition, the human P2Y1-like G protein-coupled receptor gene or gene product may itself be tested for differential expression.

The degree to which expression differs in a normal versus a diseased state need only be large enough to be visualized via standard characterization techniques such as differential display techniques. Other such standard characterization techniques by which expression differences may be visualized include but are not limited to, quantitative RT (reverse transcriptase), PCR, and Northern analysis.

## *Identification of Differentially Expressed Genes*

To identify differentially expressed genes total RNA or, preferably, mRNA is isolated from tissues of interest. For example, RNA samples are obtained from tissues of experimental subjects and from corresponding tissues of control subjects. Any RNA isolation technique that does not select against the isolation of mRNA may be utilized for the purification of such RNA samples. See, for example, Ausubel *et al*., ed., CURRENT PROTOCOLS IN MOLECULAR BIOLOGY, John Wiley & Sons, Inc. New York, 1987-1993. Large numbers of tissue samples may readily be processed using techniques well known to those of skill in the art, such as, for example, the single-step RNA isolation process of Chomczynski, U.S. Patent 4,843,155.

Transcripts within the collected RNA samples that represent RNA produced by differentially expressed genes are identified by methods well known to those of skill in the art. They include, for example, differential screening (Tedder *et al., Proc. Natl. Acad. Sci. U.S.A. 85,* 208-12, 1988), subtractive hybridization (Hedrick *et al., Nature 308,* 149-53; Lee *et al., Proc. Natl. Acad. Sci. U.S.A. 88,* 2825, 1984), and differential display (Liang & Pardee, *Science 257*, 967-71, 1992; U.S. Patent 5,262,311), and microarrays.

The differential expression information may itself suggest relevant methods for the treatment of disorders involving the human P2Y1-like G protein-coupled receptor. For example, treatment may include a modulation of expression of the differentially expressed genes and/or the gene encoding the human P2Y1-like G protein-coupled receptor. The differential expression information may indicate whether the expression or activity of the differentially expressed gene or gene product or the human P2Y1-like G protein-coupled receptor gene or gene product are up-regulated or down-regulated.

## *Screening Methods*

The invention provides assays for screening test compounds which bind to or modulate the activity of a P2Y1-like GPCR polypeptide or a P2Y1-like GPCR polynucleotide. A test compound preferably binds to a P2Y1-like GPCR polypeptide or polynucleotide. More preferably, a test compound decreases or increases a biological effect mediated via human P2Y1-like GPCR by at least about 10, preferably about 50, more preferably about 75, 90, or 100% relative to the absence of the test compound.

*Test Compounds*

Test compounds can be pharmacologic agents already known in the art or can be compounds previously unknown to have any pharmacological activity. The compounds can be naturally occurring or designed in the laboratory. They can be isolated from microorganisms, animals, or plants, and can be produced recombinantly, or synthesized by chemical methods known in the art. If desired, test compounds can be obtained using any of the numerous combinatorial library methods known in the art, including but not limited to, biological libraries, spatially addressable parallel solid phase or solution phase libraries, synthetic library methods requiring deconvolution, the "one-bead one-compound" library method, and synthetic library methods using affinity chromatography selection. The biological library approach is limited to polypeptide libraries, while the other four approaches are applicable to polypeptide, non-peptide oligomer, or small molecule libraries of compounds. *See* Lam, *Anticancer Drug Des.* 12, 145, 1997.

Methods for the synthesis of molecular libraries are well known in the art *(see,* for example, DeWitt *et al., Proc. Natl. Acad Sci. U.S.A. 90, 6909, 1993; Erb et al. Proc. Natl. Acad. Sci. U.S.A. 91,* 11422, 1994; Zuckermann *et al., J. Med. Chem. 37,* 2678, 1994; Cho *et al., Science 261,* 1303, 1993; Carell *et al., Angew. Chem. Int. Ed. Engl. 33*, 2059, 1994; Carell *et al., Angew. Chem. Int. Ed. Engl. 33*, 2061; Gallop *et al., J. Med. Chem. 37*, 1233, 1994). Libraries of compounds can be presented in solution *(see, e.g.,* Houghten, *BioTechniques 13*, 412-421, 1992), or on beads (Lam, *Nature 354*, 82-84, 1991), chips (Fodor, *Nature 364,* 555-556, 1993), bacteria or spores (Ladner, U.S. Patent 5,223,409), plasmids (Cull *et al., Proc. Natl. Acad. Sci. U.S.A. 89,* 1865-1869, 1992), or phage (Scott & Smith, *Science 249,* 386-390, 1990; Devlin, *Science* 249, 404-406, 1990); Cwirla *et al., Proc. Natl. Acad Sci. 97*, 6378-6382, 1990; Felici, *J. Mol. Biol. 222*, 301-310, 1991; and Ladner, U.S. Patent 5,223,409).

*High Throughput Screening*

Test compounds can be screened for the ability to bind to P2Y1-like GPCR polypeptides or polynucleotides or to affect P2Y1-like GPCR activity or P2Y1-like GPCR gene expression using high throughput screening. Using high throughput screening, many discrete compounds can be tested in parallel so that large numbers of test compounds can be quickly screened. The most widely established techniques utilize 96-well microtiter plates. The wells of the microtiter plates typically require assay volumes that range from 50 to 500 μl. In addition to the plates, many instruments, materials, pipettors, robotics, plate washers, and plate readers are commercially available to fit the 96-well format.

Alternatively, "free format assays," or assays that have no physical barrier between samples, can be used. For example, an assay using pigment cells (melanocytes) in a simple homogeneous assay for combinatorial peptide libraries is described by Jayawickreme *et al., Proc. Natl. Acad. Sci. U.S.A. 19,* 1614-18 (1994). The cells are placed under agarose in petri dishes, then beads that carry combinatorial compounds are placed on the surface of the agarose. The combinatorial compounds are partially released the compounds from the beads. Active compounds can be visualized as dark pigment areas because, as the compounds diffuse locally into the gel matrix, the active compounds cause the cells to change colors.

Another example of a free format assay is described by Chelsky, "Strategies for Screening Combinatorial Libraries: Novel and Traditional Approaches," reported at the First Annual Conference of The Society for Biomolecular Screening in Philadelphia, Pa. (Nov. 7-10, 1995). Chelsky placed a simple homogenous enzyme assay for carbonic anhydrase inside an agarose gel such that the enzyme in the gel would cause a color change throughout the gel. Thereafter, beads carrying combinatorial compounds via a photolinker were placed inside the gel and the compounds were partially released by UV-light. Compounds that inhibited the enzyme were observed as local zones of inhibition having less color change.

Yet another example is described by Sahnon *et al., Molecular Diversity 2,* 57-63 (1996). In this example, combinatorial libraries were screened for compounds that had cytotoxic effects on cancer cells growing in agar.

Another high throughput screening method is described in Beutel *et al.*, U.S. Patent 5,976,813. In this method, test samples are placed in a porous matrix. One or more assay components are then placed within, on top of, or at the bottom of a matrix such as a gel, a plastic sheet, a filter, or other form of easily manipulated solid support. When samples are introduced to the porous matrix they diffuse sufficiently slowly, such that the assays can be performed without the test samples running together.

*Binding Assays*

For binding assays, the test compound is preferably a small molecule which binds to and occupies the active site of the P2Y1-like GPCR polypeptide, thereby making the ligand binding site inaccessible to substrate such that normal biological activity is prevented. Examples of such small molecules include, but are not limited to, small peptides or peptide-like molecules. Potential ligands which may bind to a polypeptide of the invention include, but are not limited to, the natural ligands of known GPCRs and analogues or derivatives thereof. Natural ligands of GPCRs include adrenomedullin, amylin, calcitonin gene related protein (CGRP), calcitonin, anandamide, serotonin, histamine, adrenalin, noradrenalin, platelet activating factor, thrombin, C5a, bradykinin, and chemokines.

In binding assays, either the test compound or the P2Y1-like GPCR polypeptide can comprise a detectable label, such as a fluorescent, radioisotopic, chemiluminescent, or enzymatic label, such as horseradish peroxidase, alkaline phosphatase, or luciferase. Detection of a test compound which is bound to the P2Y1-like GPCR polypeptide can then be accomplished, for example, by direct counting of radioemmission, by scintillation counting, or by determining conversion of an appropriate substrate to a detectable product.

Alternatively, binding of a test compound to a P2Y1-like GPCR polypeptide can be determined without labeling either of the interactants. For example, a microphysiometer can be used to detect binding of a test compound with a P2Y1-like GPCR polypeptide. A microphysiometer *(e.g.,* Cytosensor™) is an analytical instrument that measures the rate at which a cell acidifies its environment using a light-addressable potentiometric sensor (LAPS). Changes in this acidification rate can be used as an indicator of the interaction between a test compound and a P2Y1- like GPCR polypeptide (McConnell *et al., Science 257,* 1906-1912, 1992).

Determining the ability of a test compound to bind to a P2Y1-like GPCR polypeptide also can be accomplished using a technology such as real-time Bimolecular Interaction Analysis (BIA) (Sjolander & Urbaniczky, *Anal. Chem. 63,* 2338-2345, 1991, and *Szabo et al., Curr. Opin. Struct. Biol. 5*, 699-705, 1995). BIA is a technology for studying biospecific interactions in real time, without labeling any of the interactants (e.g., BIAcore™). Changes in the optical phenomenon surface plasmon resonance (SPR) can be used as an indication of real-time reactions between biological molecules.

In yet another aspect of the invention, a P2Y1-like GPCR polypeptide can be used as a "bait protein" in a two-hybrid assay or three-hybrid assay (see, *e.g.*, U.S. Patent 5,283,317; Zervos *et al., Cell 72,* 223-232, 1993; Madura *et al., J. Biol. Chem. 268,* 12046-12054, 1993; Bartel *et al., BioTechniques 14,* 920-924, 1993; Iwabuchi *et al.*, *Oncogene 8,* 1693-1696, 1993; and Brent W094/10300), to identify other proteins which bind to or interact with the P2Y1-like GPCR polypeptide and modulate its activity.

The two-hybrid system is based on the modular nature of most transcription factors, which consist of separable DNA-binding and activation domains. Briefly, the assay utilizes two different DNA constructs. For example, in one construct, polynucleotide encoding a P2Y1-like GPCR polypeptide can be fused to a polynucleotide encoding the DNA binding domain of a known transcription factor (e.g., GAL-4). In the other construct a DNA sequence that encodes an unidentified protein ("prey" or "sample") can be fused to a polynucleotide that codes for the activation domain of the known transcription factor. If the "bait" and the "prey" proteins are able to interact *in vivo* to form an protein-dependent complex, the DNA-binding and activation domains of the transcription factor are brought into close proximity. This proximity allows transcription of a reporter gene (e.g., LacZ), which is operably linked to a transcriptional regulatory site responsive to the transcription factor. Expression of the reporter gene can be detected, and cell colonies containing the functional transcription factor can be isolated and used to obtain the DNA sequence encoding the protein which interacts with the P2Y1-like GPCR polypeptide.

It may be desirable to immobilize either the P2Y1-like GPCR polypeptide (or polynucleotide) or the test compound to facilitate separation of bound from unbound forms of one or both of the interactants, as well as to accommodate automation of the assay. Thus, either the P2Y1-like GPCR polypeptide (or polynucleotide) or the test compound can be bound to a solid support. Suitable solid supports include, but are not limited to, glass or plastic slides, tissue culture plates, microtiter wells, tubes, silicon chips, or particles such as beads (including, but not limited to, latex, polystyrene, or glass beads). Any method known in the art can be used to attach the P2Y1-like GPCR polypeptide (or polynucleotide) or test compound to a solid support, including use of covalent and non-covalent linkages, passive absorption, or pairs of binding moieties attached respectively to the polypeptide (or polynucleotide) or test compound and the solid support. Test compounds are preferably bound to the solid support in an array, so that the location of individual test compounds can be tracked. Binding of a test compound to a P2Y1-like GPCR polypeptide (or polynucleotide) can be accomplished in any vessel suitable for containing the reactants. Examples of such vessels include microtiter plates, test tubes, and microcentrifuge tubes.

In one embodiment, the P2Y1-like GPCR polypeptide is a fusion protein comprising a domain that allows the P2Y1-like GPCR polypeptide to be bound to a solid support. For example, glutathione-S-transferase fusion proteins can be adsorbed onto glutathione sepharose beads (Sigma Chemical) or glutathione derivatized microtiter plates, which are then combined with the test compound or the test compound and the non-adsorbed P2Y1-like GPCR polypeptide; the mixture is then incubated under conditions conducive to complex formation (e.g., at physiological conditions for salt and pH). Following incubation, the beads or microtiter plate wells are washed to remove any unbound components. Binding of the interactants can be determined either directly or indirectly, as described above. Alternatively, the complexes can be dissociated from the solid support before binding is determined.

Other techniques for immobilizing proteins or polynucleotides on a solid support also can be used in the screening assays of the invention. For example, either a P2Y1-like GPCR polypeptide (or polynucleotide) or a test compound can be immobilized utilizing conjugation of biotin and streptavidin. Biotinylated P2Y1-like GPCR polypeptides (or polynucleotides) or test compounds can be prepared from biotin-NHS(N-hydroxysuccinimide) using techniques well known in the art (e.g., biotinylation kit, Pierce Chemicals, Rockford, Ill.) and immobilized in the wells of streptavidin-coated 96 well plates (Pierce Chemical). Alternatively, antibodies which specifically bind to a P2Y1-like GPCR polypeptide, polynucleotide, or a test compound, but which do not interfere with a desired binding site, such as the active site of the P2Y1-like GPCR polypeptide, can be derivatized to the wells of the plate. Unbound target or protein can be trapped in the wells by antibody conjugation.

Methods for detecting such complexes, in addition to those described above for the GST-immobilized complexes, include immunodetection of complexes using antibodies which specifically bind to the P2Y1-like GPCR polypeptide or test compound, enzyme-linked assays which rely on detecting an activity of the P2Y1- like GPCR polypeptide, and SDS gel electrophoresis under non-reducing conditions.

Screening for test compounds which bind to a P2Y1-like GPCR polypeptide or polynucleotide also can be carried out in an intact cell. Any cell which comprises a P2Y1-like GPCR polypeptide or polynucleotide can be used in a cell-based assay system. A P2Y1-like GPCR polynucleotide can be naturally occurring in the cell or can be introduced using techniques such as those described above. Binding of the test compound to a P2Y1-like GPCR polypeptide or polynucleotide is determined as described above.

*Functional Assays*

Test compounds can be tested for the ability to increase or decrease a biological effect of a P2Y1-like GPCR polypeptide. Such biological effects can be determined using the functional assays described in the specific examples, below. Functional assays can be carried out after contacting either a purified P2Y1-like GPCR polypeptide, a cell membrane preparation, or an intact cell with a test compound. A test compound which decreases a functional activity of a P2Y1-like GPCR by at least about 10, preferably about 50, more preferably about 75, 90, or 100% is identified as a potential agent for decreasing P2Y1-like GPCR activity. A test compound which increases P2Y1-like GPCR activity by at least about 10, preferably about 50, more preferably about 75, 90, or 100% is identified as a potential agent for increasing P2Y1-like GPCR activity.

One such screening procedure involves the use of melanophores which are transfected to express a P2Y1-like GPCR polypeptide. Such a screening technique is described in WO 92/01810 published Feb. 6, 1992. Thus, for example, such an assay may be employed for screening for a compound which inhibits activation of the receptor polypeptide by contacting the melanophore cells which comprise the receptor with both a receptor ligand and a test compound to be screened. Inhibition of the signal generated by the ligand indicates that a test compound is a potential antagonist for the receptor, i. e., inhibits activation of the receptor. The screen may be employed for identifying a test compound which activates the receptor by contacting such cells with compounds to be screened and determining whether each test compound generates a signal, *i.e.*, activates the receptor.

Other screening techniques include the use of cells which express a human P2Y1-like GPCR polypeptide (for example, transfected CHO cells) in a system which measures extracellular pH changes caused by receptor activation *(see, e.g., Science 246,* 181-296, 1989). For example, test compounds may be contacted with a cell which expresses a human P2Y1-like GPCR polypeptide and a second messenger response, *e.g.,* signal transduction or pH changes, can be measured to determine whether the test compound activates or inhibits the receptor.

Another such screening technique involves introducing RNA encoding a human P2Y1-like GPCR polypeptide into *Xenopus* oocytes to transiently express the receptor. The transfected oocytes can then be contacted with the receptor ligand and a test compound to be screened, followed by detection of inhibition or activation of a calcium signal in the case of screening for test compounds which are thought to inhibit activation of the receptor.

Another screening technique involves expressing a human P2Y1-like GPCR polypeptide in cells in which the receptor is linked to a phospholipase C or D. Such cells include endothelial cells, smooth muscle cells, embryonic kidney cells, etc. The screening may be accomplished as described above by quantifying the degree of activation of the receptor from changes in the phospholipase activity.

Details of functional assays such as those described above are provided in the specific examples, below.

*Gene Expression*

In another embodiment, test compounds which increase or decrease P2Y1-like GPCR gene expression are identified. A P2Y1-like GPCR polynucleotide is contacted with a test compound, and the expression of an RNA or polypeptide product of the P2Y1- like GPCR polynucleotide is determined. The level of expression of appropriate mRNA or polypeptide in the presence of the test compound is compared to the level of expression of mRNA or polypeptide in the absence of the test compound. The test compound can then be identified as a modulator of expression based on this comparison. For example, when expression of mRNA or polypeptide is greater in the presence of the test compound than in its absence, the test compound is identified as a stimulator or enhancer of the mRNA or polypeptide expression. Alternatively, when expression of the mRNA or polypeptide is less in the presence of the test compound than in its absence, the test compound is identified as an inhibitor of the mRNA or polypeptide expression.

The level of P2Y1-like GPCR mRNA or polypeptide expression in the cells can be determined by methods well known in the art for detecting mRNA or polypeptide. Either qualitative or quantitative methods can be used. The presence of polypeptide products of a P2Y1-like GPCR polynucleotide can be determined, for example, using a variety of techniques known in the art, including immunochemical methods such as radioimmunoassay, Western blotting, and immunohistochemistry. Alternatively, polypeptide synthesis can be determined *in vivo,* in a cell culture, or in an *in vitro* translation system by detecting incorporation of labeled amino acids into a P2Y1-like GPCR polypeptide.

Such screening can be carried out either in a cell-free assay system or in an intact cell. Any cell which expresses a P2Y1-like GPCR polynucleotide can be used in a cell-based assay system. The P2Y1-like GPCR polynucleotide can be naturally occurring in the cell or can be introduced using techniques such as those described above. Either a primary culture or an established cell line, such as CHO or human embryonic kidney 293 cells, can be used.

## *Pharmaceutical Compositions*

The invention also provides pharmaceutical compositions which can be administered to a patient to achieve a therapeutic effect. Pharmaceutical compositions of the invention can comprise, for example, a P2Y1-like GPCR polypeptide, P2Y1-like GPCR polynucleotide, antibodies which specifically bind to a P2Y1-like GPCR polypeptide, or mimetics, agonists, antagonists, or inhibitors of a P2Y1-like GPCR polypeptide activity. The compositions can be administered alone or in combination with at least one other agent, such as stabilizing compound, which can be administered in any sterile, biocompatible pharmaceutical carrier, including, but not limited to, saline, buffered saline, dextrose, and water. The compositions can be administered to a patient alone, or in combination with other agents, drugs or hormones.

In addition to the active ingredients, these pharmaceutical compositions can contain suitable pharmaceutically-acceptable carriers comprising excipients and auxiliaries which facilitate processing of the active compounds into preparations which can be used pharmaceutically. Pharmaceutical compositions of the invention can be administered by any number of routes including, but not limited to, oral, intravenous, intramuscular, intra-arterial, intramedullary, intrapulmonary, intrahepatic, intrathecal, intraventricular, transdermal, subcutaneous, intraperitoneal, intranasal, parenteral, topical, sublingual, or rectal means. Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

Pharmaceutical preparations for oral use can be obtained through combination of active compounds with solid excipient, optionally grinding a resulting mixture, and processing the mixture of granules, after adding suitable auxiliaries, if desired, to obtain tablets or dragee cores. Suitable excipients are carbohydrate or protein fillers, such as sugars, including lactose, sucrose, mannitol, or sorbitol; starch from corn, wheat, rice, potato, or other plants; cellulose, such as methyl cellulose, hydroxypropylmethyl-cellulose, or sodium carboxymethylcellulose; gums including arabic and tragacanth; and proteins such as gelatin and collagen. If desired, disintegrating or solubilizing agents can be added, such as the cross-linked polyvinyl pyrrolidone, agar, alginic acid, or a salt thereof, such as sodium alginate.

Dragee cores can be used in conjunction with suitable coatings, such as concentrated sugar solutions, which also can contain gum arabic, talc, polyvinylpyrrolidone, carbopol gel, polyethylene glycol, and/or titanium dioxide, lacquer solutions, and suitable organic solvents or solvent mixtures. Dyestuffs or pigments can be added to the tablets or dragee coatings for product identification or to characterize the quantity of active compound, *i.e.*, dosage.

Pharmaceutical preparations which can be used orally include push-fit capsules made of gelatin, as well as soft, sealed capsules made of gelatin and a coating, such as glycerol or sorbitol. Push-fit capsules can contain active ingredients mixed with a filler or binders, such as lactose or starches, lubricants, such as talc or magnesium stearate, and, optionally, stabilizers. In soft capsules, the active compounds can be dissolved or suspended in suitable liquids, such as fatty oils, liquid, or liquid polyethylene glycol with or without stabilizers.

Pharmaceutical formulations suitable for parenteral administration can be formulated in aqueous solutions, preferably in physiologically compatible buffers such as Hanks' solution, Ringer's solution, or physiologically buffered saline. Aqueous injection suspensions can contain substances which increase the viscosity of the suspension, such as sodium carboxymethyl cellulose, sorbitol, or dextran. Additionally, suspensions of the active compounds can be prepared as appropriate oily injection suspensions. Suitable lipophilic solvents or vehicles include fatty oils such as sesame oil, or synthetic fatty acid esters, such as ethyl oleate or triglycerides, or liposomes. Non-lipid polycationic amino polymers also can be used for delivery. Optionally, the suspension also can contain suitable stabilizers or agents which increase the solubility of the compounds to allow for the preparation of highly concentrated solutions. For topical or nasal administration, penetrants appropriate to the particular barrier to be permeated are used in the formulation. Such penetrants are generally known in the art.

The pharmaceutical compositions of the present invention can be manufactured in a manner that is known in the art, *e.g.*, by means of conventional mixing, dissolving, granulating, dragee-making, levigating, emulsifying, encapsulating, entrapping, or lyophilizing processes. The pharmaceutical composition can be provided as a salt and can be formed with many acids, including but not limited to, hydrochloric, sulfuric, acetic, lactic, tartaric, malic, succinic, etc. Salts tend to be more soluble in aqueous or other protonic solvents than are the corresponding free base forms. In other cases, the preferred preparation can be a lyophilized powder which can contain any or all of the following: 1-50 mM histidine, 0.1%-2% sucrose, and 2-7% mannitol, at a pH range of 4.5 to 5.5, that is combined with buffer prior to use.

Further details on techniques for formulation and administration can be found in the latest edition of REMINGTON'S PHARMACEUTICAL SCIENCES (Maack Publishing Co., Easton, Pa.). After pharmaceutical compositions have been prepared, they can be placed in an appropriate container and labeled for treatment of an indicated condition. Such labeling would include amount, frequency, and method of administration.

*Therapeutic Indications and Methods*

GPCRs are ubiquitous in the mammalian host and are responsible for many biological functions, including many pathologies. Accordingly, it is desirable to find compounds and drugs which stimulate a GPCR on the one hand and which can inhibit the function of a GPCR on the other hand. For example, compounds which activate a GPCR may be employed for therapeutic purposes, such as the treatment of asthma, inflammation, CNS disorders, including Parkinson's disease, acute heart failure, urinary retention, and osteoporosis. In particular, compounds which activate GPCRs are useful in treating various cardiovascular ailments such as caused by the lack of pulmonary blood flow or hypertension. In addition these compounds may also be used in treating various physiological disorders relating to abnormal control of fluid and electrolyte homeostasis and in diseases associated with abnormal angiotensin-induced aldosterone secretion. Regulation of human P2Y1-like GPCR may be particularly useful in conditions in which alterations in neuromodulation are desired.

In general, compounds which inhibit activation of a GPCR can be used for a variety of therapeutic purposes, for example, for the treatment of hypotension and/or hypertension, angina pectoris, myocardial infarction, inflammation, ulcers, asthma, allergies, benign prostatic hypertrophy, and psychotic and neurological disorders including schizophrenia, manic excitement, depression, delirium, dementia or severe mental retardation, dyskinesias, such as Huntington's disease or Tourett's syndrome, among others. Compounds which inhibit GPCRs also are useful in reversing endogenous anorexia and in the control of bulimia.

Treatment of diabetes with regulators of P2Y1-like GPCR activity is of particular interest. Diabetes mellitus is a common metabolic disorder characterized by an abnormal elevation in blood glucose, alterations in lipids and abnormalities (complications) in the cardiovascular system, eye, kidney and nervous system. Diabetes is divided into two separate diseases: type 1 diabetes (juvenile onset) that results from a loss of cells which make and secrete insulin, and type 2 diabetes (adult onset) which is caused by a defect in insulin secretion and a defect in insulin action.

Type 1 diabetes is initiated by an autoimmune reaction that attacks the insulin secreting cells (beta cells) in the pancreatic islets. Agents that prevent this reaction from occurring or that stop the reaction before destruction of the beta cells has been accomplished are potential therapies for this disease. Other agents that induce beta cell proliferation and regeneration are also potential therapies.

Type II diabetes is the most common of the two diabetic conditions (6% of the population). The defect in insulin secretion is an important cause of the diabetic condition and results from an inability of the beta cell to properly detect and respond to rises in blood glucose levels with insulin release. Therapies that increase the response by the beta cell to glucose would offer an important new treatment for this disease.

The defect in insulin action in Type II diabetic subjects is another target for therapeutic intervention. Agents that increase the activity of the insulin receptor in muscle, liver and fat will cause a decrease in blood glucose and a normalization of plasma lipids. The receptor activity can be increased by agents that directly stimulate the receptor or that increase the intracellular signals from the receptor. Other therapies can directly activate the cellular end process, i.e. glucose transport or various enzyme systems, to generate an insulin-like effect and therefore a produce beneficial outcome. Because overweight subjects have a greater susceptibility to Type II diabetes, any agent that reduces body weight is a possible therapy.

Both Type I and Type diabetes can be treated with agents that mimic insulin action or that treat diabetic complications by reducing blood glucose levels. Likewise agents that reduces new blood vessel growth can be used to treat the eye complications that develop in both diseases.

Human P2Y1-like GPCR also can be regulated to treat cancer. Cancer is a disease fundamentally caused by oncogenic cellular transformation. There are several hallmarks of transformed cells that distinguish them from their normal counterparts and underlie the pathophysiology of cancer. These include uncontrolled cellular proliferation, unresponsiveness to normal death-inducing signals (immortalization), increased cellular motility and invasiveness, increased ability to recruit blood supply through induction of new blood vessel formation (angiogenesis), genetic instability, and dysregulated gene expression. Various combinations of these aberrant physiologies, along with the acquisition of drug-resistance frequently lead to an intractable disease state in which organ failure and patient death ultimately ensue.

Most standard cancer therapies target cellular proliferation and rely on the differential proliferative capacities between transformed and normal cells for their efficacy. This approach is hindered by the facts that several important normal cell types are also highly proliferative and that cancer cells frequently become resistant to these agents. Thus, the therapeutic indices for traditional anti-cancer therapies rarely exceed 2.0.

The advent of genomics-driven molecular target identification has opened up the possibility of identifying new cancer-specific targets for therapeutic intervention that will provide safer, more effective treatments for cancer patients. Thus, newly discovered tumor-associated genes and their products can be tested for their role(s) in disease and used as tools to discover and develop innovative therapies. Genes playing important roles in any of the physiological processes outlined above can be characterized as cancer targets.

Genes or gene fragments identified through genomics can readily be expressed in one or more heterologous expression systems to produce functional recombinant proteins. These proteins are characterized *in vitro* for their biochemical properties and then used as tools in high-throughput molecular screening programs to identify chemical modulators of their biochemical activities. Agonists and/or antagonists of target protein activity can be identified in this manner and subsequently tested in cellular and *in vivo* disease models for anti-cancer activity. Optimization of lead compounds with iterative testing in biological models and detailed pharmacokinetic and toxicological analyses form the basis for drug development and subsequent testing in humans.

Human P2Y1-like GPCR can be regulated to treat osteoporosis. Osteoporosis is a disease characterized by low bone mass and microarchitectural deterioration of bone tissue, leading to enhanced bone fragility and a consequent increase in fracture risk. It is the most common human metabolic bone disorder. Established osteoporosis includes the presence of fractures. Bone turnover occurs by the action of two major effector cell types within bone: the osteoclast, which is responsible for bone resorption, and the osteoblast, which synthesizes and mineralizes bone matrix. The actions of osteoclasts and osteoblasts are highly coordinated. Osteoclast precursors are recruited to the site of turnover; they differentiate and fuse to form mature osteoclasts which then resorb bone. Attached to the bone surface, osteoclasts produce an acidic microenvironment in a tightly defined junction between the specialized osteoclast border membrane and the bone matrix, thus allowing the localized solubilization of bone matrix. This in turn facilitate the proteolysis of demineralized bone collagen. Matrix degradation is thought to release matrix-associated growth factor and cytokines, which recruit osteoblasts in a temporally and spatially controlled fashion. Osteoblasts synthesize and secrete new bone matrix proteins, and subsequently mineralize this new matrix. In the normal skeleton this is a physiological process which does not result in a net change in bone mass. In pathological states, such as osteoporosis, the balance between resorption and formation is altered such that bone loss occurs. See WO 99/45923.

The osteoclast itself is the direct or indirect target of all currently available osteoporosis agents with the possible exception of fluoride. Antiresorptive therapy prevents further bone loss in treated individuals. Osteoblasts are derived from multipotent stem cells which reside in bone marrow and also gives rise to adipocytes, chondrocytes, fibroblasts and muscle cells. Selective enhancement of osteoblast activity is a highly desirable goal for osteoporosis therapy since it would result in an increase in bone mass, rather than a prevention of further bone loss. An effective anabolic therapy would be expected to lead to a significantly greater reduction in fracture risk than currently available treatments.

The agonists or antagonists to the newly discovered polypeptides may act as antiresorptive by directly altering the osteoclast differentiation, osteoclast adhesion to the bone matrix or osteoclast function of degrading the bone matrix. The agonists or antagonists could indirectly alter the osteoclast function by interfering in the synthesis and/or modification of effector molecules of osteoclast differentiation or function such as cytokines, peptide or steroid hormones, proteases, etc.

The agonists or antagonists to the newly discovered polypeptides may act as anabolics by directly enhancing the osteoblast differentiation and /or its bone matrix forming function. The agonists or antagonists could also indirectly alter the osteoblast function by enhancing the synthesis of growth factors, peptide or steroid hormones or decreasing the synthesis of inhibitory molecules.

The agonists and antagonists may be used to mimic, augment or inhibit the action of the newly discovered polypeptides which may be useful to treat osteoporosis, Paget's disease, degradation of bone implants particularly dental implants.

Cardiovascular diseases, too, can be treated by regulating human P2Y1-like GPCR. Cardiovascular diseases include the following disorders of the heart and the vascular system: congestive heart failure, myocardial infarction, ischemic diseases of the heart, all kinds of atrial and ventricular arrhythmias, hypertensive vascular diseases and peripheral vascular diseases.

Heart failure is defined as a pathophysiologic state in which an abnormality of cardiac function is responsible for the failure of the heart to pump blood at a rate commensurate with the requirement of the metabolizing tissue. It includes all forms of pumping failure such as high-output and low-output, acute and chronic, right-sided or left-sided, systolic or diastolic, independent of the underlying cause.

Myocardial infarction (MI) is generally caused by an abrupt decrease in coronary blood flow that follows a thrombotic occlusion of a coronary artery previously narrowed by arteriosclerosis. MI prophylaxis (primary and secondary prevention) is included as well as the acute treatment of MI and the prevention of complications.

Ischemic diseases are conditions in which the coronary flow is restricted resulting in an perfusion which is inadequate to meet the myocardial requirement for oxygen. This group of diseases include stable angina, unstable angina and asymptomatic ischemia.

Arrhythmias include all forms of atrial and ventricular tachyarrhythmias (atrial tachycardia, atrial flutter, atrial fibrillation, atrio-ventricular reentrant tachycardia, preexcitation syndrome, ventricular tachycardia, ventricular flutter, ventricular fibrillation) as well as bradycardic forms of arrhythmias.

Hypertensive vascular diseases include primary as well as all kinds of secondary arterial hypertension (renal, endocrine, neurogenic, others). The genes may be used as drug targets for the treatment of hypertension as well as for the prevention of all complications. Peripheral vascular diseases are defined as vascular diseases in which arterial and/or venous flow is reduced resulting in an imbalance between blood supply and tissue oxygen demand. It includes chronic peripheral arterial occlusive disease (PAOD), acute arterial thrombosis and embolism, inflammatory vascular disorders, and Raynaud's disease.

Asthma and allergies, too, can be treated by regulating human P2Y1-like GPCR. Allergy is a complex process in which environmental antigens induce clinically adverse reactions. The inducing antigens, called allergens, typically elicit a specific IgE response and, although in most cases the allergens themselves have little or no intrinsic toxicity, they induce pathology when the IgE response in turn elicits an IgE-dependent or T cell-dependent hypersensitivity reaction. Hypersensitivity reactions can be local or systemic and typically occur within minutes of allergen exposure in individuals who have previously been sensitized to an allergen. The hypersensitivity reaction of allergy develops when the allergen is recognized by IgE antibodies bound to specific receptors on the surface of effector cells, such as mast cells, basophils, or eosinophils, which causes the activation of the effector cells and the release of mediators that produce the acute signs and symptoms of the reactions.

Allergic diseases include asthma, allergic rhinitis (hay fever), atopic dermatitis, and anaphylaxis.

Asthma is though to arise as a result of interactions between multiple genetic and environmental factors and is characterized by three major features: 1) intermittent and reversible airway obstruction caused by bronchoconstriction, increased mucus production, and thickening of the walls of the airways that leads to a narrowing of the airways, 2) airway hyperresponsiveness caused by a decreased control of airway caliber, and 3) airway inflammation. Certain cells are critical to the inflammatory reaction of asthma and they include T cells and antigen presenting cells, B cells that produce IgE, and mast cells, basophils, eosinophils, and other cells that bind IgE. These effector cells accumulate at the site of allergic reaction in the airways and release toxic products that contribute to the acute pathology and eventually to the tissue destruction related to the disorder. Other resident cells, such as smooth muscle cells, lung epithelial cells, mucus-producing cells, and nerve cells may also be abnormal in individuals with asthma and may contribute to the pathology. While the airway obstruction of asthma, presenting clinically as an intermittent wheeze and shortness of breath, is generally the most pressing symptom of the disease requiring immediate treatment, the inflammation and tissue destruction associated with the disease can lead to irreversible changes that eventually make asthma a chronic disabling disorder requiring long-term management.

Despite recent important advances in our understanding of the pathophysiology of asthma, the disease appears to be increasing in prevalence and severity (Gergen and Weiss, *Am. Rev. Respir. Dis. 146,* 823-24, 1992). It is estimated that 30-40% of the population suffer with atopic allergy, and 15% of children and 5% of adults in the population suffer from asthma (Gergen and Weiss, 1992). Thus, an enormous burden is placed on our health care resources. However, both diagnosis and treatment of asthma are difficult. The severity of lung tissue inflammation is not easy to measure and the symptoms of the disease are often indistinguishable from those of respiratory infections, chronic respiratory inflammatory disorders, allergic rhinitis, or other respiratory disorders. Often, the inciting allergen cannot be determined, making removal of the causative environmental agent difficult. Current pharmacological treatments suffer their own set of disadvantages. Commonly used therapeutic agents, such as beta agonists, can act as symptom relievers to transiently improve pulmonary function, but do not affect the underlying inflammation. Agents that can reduce the underlying inflammation, such as anti-inflammatory steroids, can have major drawbacks that range from immunosuppression to bone loss (Goodman and Gilman's THE PHARMACOLOGIC BASIS OF THERAPEUTICS, Seventh Edition, MacMillan Publishing Company, NY, USA, 1985). In addition, many of the present therapies, such as inhaled corticosteroids, are short-lasting, inconvenient to use, and must be used often on a regular basis, in some cases for life, making failure of patients to comply with the treatment a major problem and thereby reducing their effectiveness as a treatment.

Because of the problems associated with conventional therapies, alternative treatment strategies have been evaluated. Glycophorin A (Chu and Sharom, *Cell. Immunol. 145*, 223-39, 1992), cyclosporin (Alexander *et al., Lancet 339,* 324-28, 1992), and a nonapeptide fragment of IL-2 (Zav'yalov *et al., Immunol. Lett. 31, 285-88,* 1992) all inhibit interleukin-2 dependent T lymphocyte proliferation; however, they are known to have many other effects. For example, cyclosporin is used as a immuno- suppressant after organ transplantation. While these agents may represent alternatives to steroids in the treatment of asthmatics, they inhibit interleukin-2 dependent T lymphocyte proliferation and potentially critical immune functions associated with homeostasis. Other treatments that block the release or activity of mediators of bronchochonstriction, such as cromones or anti-leukotrienes, have recently been introduced for the treatment of mild asthma, but they are expensive and not effective in all patients and it is unclear whether they have any effect on the chronic changes associated with asthmatic inflammation. What is needed in the art is the identification of a treatment that can act in pathways critical to the development of asthma that both blocks the episodic attacks of the disorder and preferentially dampens the hyperactive allergic immune response without immunocompromising the patient.

**Potential relevance of P2Y-like GPCR to asthma.**

Extracellular nucleotides induce a wide variety of responses in many cell types, including muscle contraction and relaxation, vasodilation, neurotransmission, platelet aggregation, ion transport regulation, and cell growth. The effects are exerted through P2 receptors, which are classified into two main families: P2X receptors which are ligand-gated ion channels, and P2Y receptors which are G protein-coupled receptors. Twelve distinct P2Y family members have been cloned to date in various species, at least one of which is known to bind a non-nucleotide, namely $P2Y_7$ whose ligand is $LTB_4$. The nuclotide-binding P2Y receptors can be further subdivided into three groups according to ligand specificity: P2Ys activated by adenine nucleotides, P2Ys activated by uridine nucleotides, and P2Ys activated by both adenine and uridine nucleotides.

The human P2Y-like GPCR of the invention is a new P2Y-like seven-transmembrane-domain molecule that has highest homology to $P2Y_1$. It was originally found in a search for P2Y homologs in genomic sequence databases. Only one EST has been reported to date for this gene, from a cDNA library derived from normal human epithelium. Our own expression profiling of this gene shows that it is expressed highest in the trachea, salivary glands, and kidneys, and less so in fetal brain, colon, placenta, and lung.

Although human P2Y-like GPCR is closest in homology to P2Y1, which binds adenine nucleotides (ATP and ADP), it also has significant homology to $P2Y_2$ and $P2Y_4$, which bind both A and U nucleotides, to $P2Y_3$, which binds U nucleotides, and to leukotriene receptors, which bind $LTB_4$, $LTC_4$, and $LTD_4$. Therefore, although the likely range of ligands that human P2Y-like GPCR can bind is relatively limited, the true ligand of this receptor will have to be determined empirically.

In studies of airway epithelia, both ATP and UTP have been found to equipotently regulate epithelial electrolyte and water transport, trigger mucin secretion, and increase ciliary beat frequency. In the trachea, nucleotides can induce tracheal gland serous cells, which are responsible for the secretion of antibacterial and antiproteolytic proteins, to produce secretory leukocyte proteinase inhibitor and to increase chloride transport. Studies in a mouse knockout of the $P2Y_2$ receptor show that it is the dominant extracellular nucleotide receptor in airway epithelium, but that other nucleotide receptors exist that function similarly in the respiratory tract.

Our expression profiling studies of human P2Y-like GPCR show that it appears to be expressed highly in tissues of the upper respiratory tract. Its high expression in the salivary glands and trachea may indicate that it plays a role in exocrine secretion, which in the airways has mainly a protective role. In asthma, however, overproduction of mucin contributes to the viscid mucus plugs that occlude asthmatic airways. Submucosal glands in the large airways of asthmatics also frequently show evidence of hyperplasia, which may somehow be due to overstimulation by external mediators.

It is therefore unclear at this point what effect agonists or antagonists of the human P2Y-like GPCR receptor would have in asthmatics. Agonists may beneficially increase protective protein secretion, increase ciliary beat rate, and relax smooth muscle, while antagonists may slow mucus production and glandular hyperplasia.

**References.**

Nandanan E, Jang SY, Moro S, Kim HO, Siddiqui MA, Russ P, Marquez VE, Busson R, Herdewijn P, Harden TK, Boyer JL, Jacobson KA. Synthesis, biological activity, and molecular modeling of ribose-modified deoxyadenosine bisphosphate analogues as P2Y(1) receptor ligands. *J Med Chem.* 2000 Mar 9;43(5):829-42.

Major DT, Halbfinger E, Fischer B. Molecular recognition of modified adenine nucleotides by the P2Y(1)-receptor. 2. A computational approach. *J Med Chem. 1999 Dec 30;42(26):5338-47*.

Halbfinger E, Major DT, Ritzmann M, Ubl J, Reiser G, Boyer JL, Harden KT, Fischer B. Molecular recognition of modified adenine nucleotides by the P2Y(1)-receptor. 1. A synthetic, biochemical, and NMR approach. *J Med Chem.* 1999 Dec 30;42(26):5325-37.

Moro S, Hoffmann C, Jacobson KA. Role of the extracellular loops of G protein-coupled receptors in ligand recognition: a molecular modeling study of the human P2Y1 receptor. *Biochemistry*. 1999 Mar 23;38(12):3498-507.

Moro S, Guo D, Camaioni E, Boyer JL, Harden TK, Jacobson KA. Human P2Y1 receptor: molecular modeling and site-directed mutagenesis as tools to identify agonist and antagonist recognition sites. *J Med Chem.* 1998 Apr 23;41(9):1456-66.

Webb TE, Simon J, Barnard EA. Regional distribution of [35S]2'-deoxy 5'-O-(1-thio) ATP binding sites and the P2Y1 messenger RNA within the chick brain. *Neuroscience.* 1998 Jun;84(3):825-37.
Homolya L, Watt WC, Lazarowski ER, Koller BH, Boucher RC. Nucleotide- regulated calcium signaling in lung fibroblasts and epithelial cells from normal and P2Y(2) receptor (-/-) mice. *JBiol Chem.* 1999 Sep 10;274(37):26454-60.
Leon C, Hechler B, Vial C, Leray C, Cazenave JP, Gachet C. The P2Y1 receptor is an ADP receptor antagonized by ATP and expressed in platelets and megakaryoblastic cells. *FEBS Lett.* 1997 Feb 10;403(1):26-30.
Dehaye JP, Moran A, Marino A. Purines, a new class of agonists in salivary glands? *Arch Oral Biol.* 1999 May;44 Suppl 1:539-43.
King BF, Townsend-Nicholson A, Bumstock G. Metabotropic receptors for ATP and UTP: exploring the correspondence between native and recombinant nucleotide receptors. *Trends Pharmacol Sci.* 1998 Dec;19(12):506-14.
Saleh A, Figarella C, Kammouni W, Marchand-Pinatel S, Lazdunski A, Tubul A, Brun P, Merten MD. Pseudomonas aeruginosa quorum-sensing signal molecule N-(3-oxododecanoyl)-L-homoserine lactone inhibits expression of P2Y receptors in cystic fibrosis tracheal gland cells. *Infect Immun.* 1999 Oct;67(10):5076-82.
Cressman VL, Lazarowski E, Homolya L, Boucher RC, Koller BH, Grubb BR Effect of loss of P2Y(2) receptor gene expression on nucleotide regulation of murine epithelial Cl(-) transport. *J Biol Chem.* 1999 Sep 10;274(37):26461-8.

COPD. Chronic obstructive pulmonary (or airways) disease (COPD) is a condition defined physiologically as airflow obstruction that generally results from a mixture of emphysema and peripheral airway obstruction due to chronic bronchitis (Senior & Shapiro, *Pulmonary Diseases and Disorders,* 3d ed., New York, McGraw-Hill, 1998, pp. 659-681, 1998; Barnes, *Chest 117,* 10S-14S, 2000). Emphysema is characterized by destruction of alveolar walls leading to abnormal enlargement of the air spaces of the lung. Chronic bronchitis is defined clinically as the presence of chronic productive cough for three months in each of two successive years. In COPD, airflow obstruction is usually progressive and is only partially reversible. By far the most important risk factor for development of COPD is cigarette smoking, although the disease does occur in non-smokers.

Chronic inflammation of the airways is a key pathological feature of COPD (Senior & Shapiro, 1998). The inflammatory cell population comprises increased numbers of macrophages, neutrophils, and $CD8^+$ lymphocyes. Inhaled irritants, such as cigarette smoke, activate macrophages which are resident in the respiratory tract, as well as epithelial cells leading to release of chemokines (e.g., interleukin-8) and other chemotactic factors. These chemotactic factors act to increase the neutrophil/monocyte trafficking from the blood into the lung tissue and airways. Neutrophils and monocytes recruited into the airways can release a variety of potentially damaging mediators such as proteolytic enzymes and reactive oxygen species. Matrix degradation and emphysema, along with airway wall thickening, surfactant dysfunction, and mucus hypersecretion, all are potential sequelae of this inflammatory response that lead to impaired airflow and gas exchange.

Several GPCRs have been implicated in the pathology of COPD. For example, the chemokine IL-8 acts through CXCR1 and CXCR2, and antagonists for these receptors are under investigation as therapeutics for COPD. Members of the P2Y family of metabotropic receptors may play key roles in normal pulmonary function. In particular, the $P2Y_2$ receptor is believed to be involved in the regulation of mucociliary clearance mechanisms in the lung, and agonists of this receptor may stimulate airway mucus clearance in patients with chronic bronchitis (Yerxa Johnson, *Drugs of the Future* 24, 759-769, 1999). GPCRs, therefore, are therapeutic targets for COPD, and the identification of additional members of existing GPCR families or of novel GPCRs would yield further attractive targets.

This invention further pertains to the use of novel agents identified by the screening assays described above. Accordingly, it is within the scope of this invention to use a test compound identified as described herein in an appropriate animal model. For example, an agent identified as described herein (e.g., a modulating agent, an antisense nucleic acid molecule, a specific antibody, ribozyme, or a P2Y1-like GPCR polypeptide binding molecule) can be used in an animal model to determine the efficacy, toxicity, or side effects of treatment with such an agent. Alternatively, an agent identified as described herein can be used in an animal model to determine the mechanism of action of such an agent. Furthermore, this invention pertains to uses of novel agents identified by the above-described screening assays for treatments as described herein.

A reagent which affects P2Y1-like GPCR activity can be administered to a human cell, either *in vitro* or *in vivo,* to reduce P2Y1-like GPCR activity. The reagent preferably binds to an expression product of a human P2Y1-like GPCR gene. If the expression product is a protein, the reagent is preferably an antibody. For treatment of human cells *ex vivo*, an antibody can be added to a preparation of stem cells which have been removed from the body. The cells can then be replaced in the same or another human body, with or without clonal propagation, as is known in the art.

In one embodiment, the reagent is delivered using a liposome. Preferably, the liposome is stable in the animal into which it has been administered for at least about 30 minutes, more preferably for at least about 1 hour, and even more preferably for at least about 24 hours. A liposome comprises a lipid composition that is capable of targeting a reagent, particularly a polynucleotide, to a particular site in an animal, such as a human. Preferably, the lipid composition of the liposome is capable of targeting to a specific organ of an animal, such as the lung, liver, spleen, heart brain, lymph nodes, and skin.

A liposome useful in the present invention comprises a lipid composition that is capable of fusing with the plasma membrane of the targeted cell to deliver its contents to the cell. Preferably, the transfection efficiency of a liposome is about 0.5 μg of DNA per 16 nmole of liposome delivered to about $10^6$ cells, more preferably about 1.0 μg of DNA per 16 nmole of liposome delivered to about $10^6$ cells, and even more preferably about 2.0 μg of DNA per 16 nmol of liposome delivered to about $10^6$ cells. Preferably, a liposome is between about 100 and 500 nm, more preferably between about 150 and 450 nm, and even more preferably between about 200 and 400 nm in diameter.

Suitable liposomes for use in the present invention include those liposomes standardly used in, for example, gene delivery methods known to those of skill in the art. More preferred liposomes include liposomes having a polycationic lipid composition and/or liposomes having a cholesterol backbone conjugated to polyethylene glycol. Optionally, a liposome comprises a compound capable of targeting the liposome to a particular cell types, such as a cell-specific ligand exposed on the outer surface of the liposome.

Complexing a liposome with a reagent such as an antisense oligonucleotide or ribozyme can be achieved using methods which are standard in the art (see, for example, U.S. Patent 5,705,151). Preferably, from about 0.1 μg to about 10 μg of polynucleotide is combined with about 8 nmol of liposomes, more preferably from about 0.5 μg to about 5 μg of polynucleotides are combined with about 8 nmol liposomes, and even more preferably about 1.0 μg of polynucleotides is combined with about 8 nmol liposomes.

In another embodiment, antibodies can be delivered to specific tissues *in vivo* using receptor-mediated targeted delivery. Receptor-mediated DNA delivery techniques are taught in, for example, Findeis *et al. Trends in Biotechnol. 11,* 202-05 (1993); Chiou *et al.*, GENE THERAPEUTICS: METHODS AND APPLICATIONS OF DIRECT GENE TRANSFER (J.A. Wolff, ed.) (1994); Wu & Wu, *J. Biol. Chem. 263,* 621-24 (1988); Wu *et al., J. Biol. Chem. 269,* 542-46 (1994); Zenke *et al., Proc. Natl. Acad. Sci. U.S.A. 87*, 3655-59 (1990); *Wu et al., J. Biol. Chem. 266, 338-42* (1991).

*Determination of a Therapeutically Effective Dose*

The determination of a therapeutically effective dose is well within the capability of those skilled in the art: A therapeutically effective dose refers to that amount of active ingredient which increases or decreases P2Y1-like GPCR activity relative to the P2Y1-like GPCR activity which occurs in the absence of the therapeutically effective dose.

For any compound, the therapeutically effective dose can be estimated initially either in cell culture assays or in animal models, usually mice, rabbits, dogs, or pigs. The animal model also can be used to determine the appropriate concentration range and route of administration. Such information can then be used to determine useful doses and routes for administration in humans.

Therapeutic efficacy and toxicity, *e.g.*, $ED_{50}$ (the dose therapeutically effective in 50% of the population) and $LD_{50}$ (the dose lethal to 50% of the population), can be determined by standard pharmaceutical procedures in cell cultures or experimental animals. The dose ratio of toxic to therapeutic effects is the therapeutic index, and it can be expressed as the ratio, $LD_{50}/ED_{50}$.

Pharmaceutical compositions which exhibit large therapeutic indices are preferred. The data obtained from cell culture assays and animal studies is used in formulating a range of dosage for human use. The dosage contained in such compositions is preferably within a range of circulating concentrations that include the $ED_{50}$ with little or no toxicity. The dosage varies within this range depending upon the dosage form employed, sensitivity of the patient, and the route of administration.

The exact dosage will be determined by the practitioner, in light of factors related to the subject that requires treatment. Dosage and administration are adjusted to provide sufficient levels of the active ingredient or to maintain the desired effect. Factors which can be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions can be administered every 3 to 4 days, every week, or once every two weeks depending on the half-life and clearance rate of the particular formulation.

Normal dosage amounts can vary from 0.1 to 100,000 micrograms, up to a total dose of about 1 g, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc.

If the reagent is a single-chain antibody, polynucleotides encoding the antibody can be constructed and introduced into a cell either *ex vivo* or *in vivo* using well-established techniques including, but not limited to, transferrin-polycation-mediated DNA transfer, transfection with naked or encapsulated nucleic acids, liposome-mediated cellular fusion, intracellular transportation of DNA-coated latex beads, protoplast fusion, viral infection, electroporation, "gene gun," and DEAE- or calcium phosphate-mediated transfection.

Effective *in vivo* dosages of an antibody are in the range of about 5 μg to about 50 μg/kg, about 50 μg to about 5 mg/kg, about 100 μg to about 500 μg/kg of patient body weight, and about 200 to about 250 μg/kg of patient body weight. For administration of polynucleotides encoding single-chain antibodies, effective *in vivo* dosages are in the range of about 100 ng to about 200 ng, 500 ng to about 50 mg, about 1 μg to about 2 mg, about 5 μg to about 500 μg, and about 20 μg to about 100 μg of DNA.

If the expression product is mRNA, the reagent is preferably an antisense oligonucleotide or a ribozyme. Polynucleotides which express antisense oligonucleotides or ribozymes can be introduced into cells by a variety of methods, as described above.

Preferably, a reagent reduces expression of a P2Y1-like GPCR gene or the activity of a P2Y1-like GPCR polypeptide by at least about 10, preferably about 50, more preferably about 75, 90, or 100% relative to the absence of the reagent. The effectiveness of the mechanism chosen to decrease the level of expression of a P2Y1-like GPCR gene or the activity of a P2Y1-like GPCR polypeptide can be assessed using methods well known in the art, such as hybridization of nucleotide probes to P2Y1-like GPCR-specific mRNA, quantitative RT-PCR, immunologic detection of a P2Y1-like GPCR polypeptide, or measurement of P2Y1-like GPCR activity.

In any of the embodiments described above, any of the pharmaceutical compositions of the invention can be administered in combination with other appropriate therapeutic agents. Selection of the appropriate agents for use in combination therapy can be made by one of ordinary skill in the art, according to conventional pharmaceutical principles. The combination of therapeutic agents can act synergistically to effect the treatment or prevention of the various disorders described above. Using this approach, one may be able to achieve therapeutic efficacy with lower dosages of each agent, thus reducing the potential for adverse side effects.

Any of the therapeutic methods described above can be applied to any subject in need of such therapy, including, for example, mammals such as dogs, cats, cows, horses, rabbits, monkeys, and most preferably, humans.

## Diagnostic Methods

GPCRs also can be used in diagnostic assays for detecting diseases and abnormalities or susceptibility to diseases and abnormalities related to the presence of mutations in the nucleic acid sequences which encode a GPCR. Such diseases, by way of example, are related to cell transformation, such as tumors and cancers, and various cardiovascular disorders, including hypertension and hypotension, as well as diseases arising from abnormal blood flow, abnormal angiotensin-induced aldosterone secretion, and other abnormal control of fluid and electrolyte homeostasis.

According to the present invention, differences can be determined between the cDNA or genomic sequence encoding a P2Y1-like GPCR in individuals afflicted with a disease and in normal individuals. If a mutation is observed in some or all of the afflicted individuals but not in normal individuals, then the mutation is likely to be the causative agent of the disease.

Sequence differences between a reference gene and a gene having mutations can be revealed by the direct DNA sequencing method. In addition, cloned DNA segments can be employed as probes to detect specific DNA segments. The sensitivity of this method is greatly enhanced when combined with PCR. For example, a sequencing primer can be used with a double-stranded PCR product or a single-stranded template molecule generated by a modified PCR. The sequence determination is performed by conventional procedures using radiolabeled nucleotides or by automatic sequencing procedures using fluorescent tags.

Genetic testing based on DNA sequence differences can be carried out by detection of alteration in electrophoretic mobility of DNA fragments in gels with or without denaturing agents. Small sequence deletions and insertions can be visualized, for example, by high resolution gel electrophoresis. DNA fragments of different sequences can be distinguished on denaturing formamide gradient gels in which the mobilities of different DNA fragments are retarded in the gel at different positions according to their specific melting or partial melting temperatures (*see, e.g.,* Myers *et al., Science 230,* 1242, 1985). Sequence changes at specific locations can also be revealed by nuclease protection assays, such as RNase and S 1 protection or the chemical cleavage method *(e.g.,* Cotton *et al., Proc. Natl. Acad. Sci. USA 85,* 4397-4401, 1985). Thus, the detection of a specific DNA sequence can be performed by methods such as hybridization, RNase protection, chemical cleavage, direct DNA sequencing or the use of restriction enzymes and Southern blotting of genomic DNA. In addition to direct methods such as gel-electrophoresis and DNA sequencing, mutations can also be detected by *in situ* analysis.

Altered levels of a P2Y1-like GPCR also can be detected in various tissues. Assays used to detect levels of the receptor polypeptides in a body sample, such as blood or a tissue biopsy, derived from a host are well known to those of skill in the art and include radioimmunoassays, competitive binding assays, Western blot analysis, and ELISA assays.

All patents and patent applications cited in this disclosure are expressly incorporated herein by reference. The above disclosure generally describes the present invention. A more complete understanding can be obtained by reference to the following specific examples which are provided for purposes of illustration only and are not intended to limit the scope of the invention.

## EXAMPLE 1

*Detection of P2Y1-like GPCR activity*

The polynucleotide of SEQ ID NO: 1 is inserted into the expression vector pCEV4 and the expression vector pCEV4-P2Y1-like GPCR polypeptide obtained is transfected into human embryonic kidney 293 cells. These cells are scraped from a culture flask into 5 ml of Tris HCl, 5 mM EDTA, pH 7.5, and lysed by sonication. Cell lysates are centrifuged at 1000 rpm for 5 minutes at 4 °C. The supernatant is centrifuged at 30,000 x g for 20 minutes at 4 °C. The pellet is suspended in binding buffer containing 50 mM Tris HCl, 5 mM $MgSO_4$, 1 mM EDTA, 100 mM NaCl, pH 7.5, supplemented with 0.1 % BSA, 2 μg/ml aprotinin, 0.5 mg/ml leupeptin, and 10 μg/ml phosphoramidon. Optimal membrane suspension dilutions, defined as the protein concentration required to bind less than 10 % of the added radioligand, are added to 96-well polypropylene microtiter plates containing $^{125}$I-labeled ligand or test compound, non-labeled peptides, and binding buffer to a final volume of 250 μl.

In equilibrium saturation binding assays, membrane preparations are incubated in the presence of increasing concentrations (0.1 nM to 4 nM) of $^{125}$I-labeled ligand or test compound (specific activity 2200 Ci/mmol). The binding affinities of different test compounds are determined in equilibrium competition binding assays, using 0.1 nM $^{125}$I-peptide in the presence of twelve different concentrations of each test compound.

Binding reaction mixtures are incubated for one hour at 30 °C. The reaction is stopped by filtration through GF/B filters treated with 0.5% polyethyleneimine, using a cell harvester. Radioactivity is measured by scintillation counting, and data are analyzed by a computerized non-linear regression program.

Non-specific binding is defined as the amount of radioactivity remaining after incubation of membrane protein in the presence of 100 nM of unlabeled peptide. Protein concentration is measured by the Bradford method using Bio-Rad Reagent, with bovine serum albumin as a standard. It is shown that the polypeptide of SEQ ID NO: 2 has a P2Y1-like GPCR activity.

## EXAMPLE 2

*Expression of recombinant human P2Y1-like GPCR*

The *Pichia pastoris* expression vector pPICZB (Invitrogen, San Diego, CA) is used to produce large quantities of a human P2Y1-like GPCR polypeptides in yeast. The human P2Y1-like GPCR polypeptide-encoding DNA sequence is derived from the nucleotide sequence shown in SEQ ID NO:5. Before insertion into vector pPICZB the DNA sequence is modified by well known methods in such a way that it contains at its 5'-end an initiation codon and at its 3'-end an enterokinase cleavage site, a His6 reporter tag and a termination codon. Moreover, at both termini recognition sequences for restriction endonucleases are added and after digestion of the multiple cloning site of pPICZ B with the corresponding restriction enzymes the modified polypeptide encoding DNA sequence is ligated into pPICZB. This expression vector is designed for inducible expression in Pichia pastoris, expression is driven by a yeast promoter. The resulting pPICZ/md-His6 vector is used to transform the yeast.

The yeast are cultivated under usual conditions in 5 liter shake flasks and the recombinantly produced protein isolated from the culture by affinity chromatography (Ni-NTA-Resin) in the presence of 8 M urea. The bound polypeptide is eluted with buffer, pH 3.5, and neutralized. Separation of the P2Y1-like GPCR polypeptide from the His6 reporter tag is accomplished by site-specific proteolysis using enterokinase (Invitrogen, San Diego, CA) according to manufacturer's instructions. Purified human P2Y1-like GPCR polypeptide is obtained.

## EXAMPLE 3

*Radioligand binding assays*

Human embryonic kidney 293 cells transfected with a polynucleotide which expresses human P2Y1-like GPCR are scraped from a culture flask into 5 ml of Tris HCl, 5 mM EDTA, pH 7.5, and lysed by sonication. Cell lysates are centrifuged at 1000 rpm for 5 minutes at 4 °C. The supernatant is centrifuged at 30,000 x g for 20 minutes at 4 °C. The pellet is suspended in binding buffer containing 50 mM Tris HCl, 5 mM $MgSO_4$, 1 mM EDTA, 100 mM NaCl, pH 7.5, supplemented with 0.1 % BSA, 2 μg/ml aprotinin, 0.5 mg/ml leupeptin, and 10 μg/ml phosphoramidon. Optimal membrane suspension dilutions, defined as the protein concentration required to bind less than 10 % of the added radioligand, are added to 96-well polypropylene microtiter plates containing $^{125}$I-labeled ligand or test compound, non-labeled peptides, and binding buffer to a final volume of 250 μl.

In equilibrium saturation binding assays, membrane preparations are incubated in the presence of increasing concentrations (0.1 nM to 4 nM) of $^{125}$I-labeled ligand or test compound (specific activity 2200 Ci/mmol). The binding affinities of different test compounds are determined in equilibrium competition binding assays, using 0.1 nM $^{125}$I-peptide in the presence of twelve different concentrations of each test compound.

Binding reaction mixtures are incubated for one hour at 30 °C. The reaction is stopped by filtration through GF/B filters treated with 0.5% polyethyleneimine, using a cell harvester. Radioactivity is measured by scintillation counting, and data are analyzed by a computerized non-linear regression program.

Non-specific binding is defined as the amount of radioactivity remaining after incubation of membrane protein in the presence of 100 nM of unlabeled peptide. Protein concentration is measured by the Bradford method using Bio-Rad Reagent, with bovine serum albumin as a standard. A test compound which increases the radioactivity of membrane protein by at least 15% relative to radioactivity of membrane protein which was not incubated with a test compound is identified as a compound which binds to a human P2Y1-like GPCR polypeptide.

## EXAMPLE 4

*Effect of a test compound on human P2Y1-like GPCR-mediated cyclic AMP formation*

Receptor-mediated inhibition of cAMP formation can be assayed in host cells which express human P2Y1-like GPCR. Cells are plated in 96-well plates and incubated in Dulbecco's phosphate buffered saline (PBS) supplemented with 10 mM HEPES, 5 mM theophylline, 2 μg/ml aprotinin, 0.5 mg/ml leupeptin, and 10 μg/ml phosphoramidon for 20 minutes at 37 °C in 5% $CO_2$. A test compound is added and incubated for an additional 10 minutes at 37 °C. The medium is aspirated, and the reaction is stopped by the addition of 100 mM HCl. The plates are stored at 4 °C for 15 minutes. cAMP content in the stopping solution is measured by radioimmunoassay.

Radioactivity is quantified using a gamma counter equipped with data reduction software. A test compound which decreases radioactivity of the contents of a well relative to radioactivity of the contents of a well in the absence of the test compound is identified as a potential inhibitor of cAMP formation. A test compound which increases radioactivity of the contents of a well relative to radioactivity of the contents of a well in the absence of the test compound is identified as a potential enhancer of cAMP formation.

## EXAMPLE 5

*Effect of a test compound on the mobilization of intracellular calcium*

Intracellular free calcium concentration can be measured by microspectrofluorometry using the fluorescent indicator dye Fura-2/AM (Bush *et al., J Neurochem. 57,* 562-74, 1991). Stably transfected cells are seeded onto a 35 mm culture dish containing a glass coverslip insert. Cells are washed with HBS , incubated with a test compound, and loaded with 100 μl of Fura-2/AM (10 μM) for 20-40 minutes. After washing with HBS to remove the Fura-2/AM solution, cells are equilibrated in HBS for 10-20 minutes. Cells are then visualized under the 40X objective of a Leitz Fluovert FS microscope.

Fluorescence emission is determined at 510 nM, with excitation wavelengths alternating between 340 nM and 380 nM. Raw fluorescence data are converted to calcium concentrations using standard calcium concentration curves and software analysis techniques. A test compound which increases the fluorescence by at least 15% relative to fluorescence in the absence of a test compound is identified as a compound which mobilizes intracellular calcium.

## EXAMPLE 6

*Effect of a test compound on phosphoinositide metabolism*

Cells which stably express human P2Y1-like GPCR cDNA are plated in 96-well plates and grown to confluence. The day before the assay, the growth medium is changed to 100 μl of medium containing 1% serum and 0.5 μCi $^3$H-myinositol. The plates are incubated overnight in a $CO_2$ incubator (5% $CO_2$ at 37 °C). Immediately before the assay, the medium is removed and replaced by 200 μl of PBS containing 10 mM LiCl, and the cells are equilibrated with the new medium for 20 minutes. During this interval, cells also are equilibrated with antagonist, added as a 10 μl aliquot of a 20-fold concentrated solution in PBS.

The $^3$H-inositol phosphate accumulation from inositol phospholipid metabolism is started by adding 10 μl of a solution containing a test compound. To the first well 10 μl are added to measure basal accumulation. Eleven different concentrations of test compound are assayed in the following 11 wells of each plate row. All assays are performed in duplicate by repeating the same additions in two consecutive plate rows.

The plates are incubated in a $CO_2$ incubator for one hour. The reaction is terminated by adding 15 μl of 50% v/v trichloroacetic acid (TCA), followed by a 40 minute incubation at 4 °C. After neutralizing TCA with 40 μl of 1 M Tris, the content of the wells is transferred to a Multiscreen HV filter plate (Millipore) containing Dowex AG1-X8 (200-400 mesh, formate form). The filter plates are prepared by adding 200 μl of Dowex AG1-X8 suspension (50% v/v, water: resin) to each well. The filter plates are placed on a vacuum manifold to wash or elute the resin bed. Each well is washed 2 times with 200 μl of water, followed by 2 x 200 μl of 5 mM sodium tetraborate/60 mM ammonium formate.

The $^3$H-IPs are eluted into empty 96-well plates with 200 μl of 1.2 M ammonium formate/0.1 formic acid. The content of the wells is added to 3 ml of scintillation cocktail, and radioactivity is determined by liquid scintillation counting.

## EXAMPLE 7

*Receptor Binding Methods*

Standard Binding Assays. Binding assays are carried out in a binding buffer containing 50 mM HEPES, pH 7.4, 0.5% BSA, and 5 mM $MgCl_2$. The standard assay for radioligand (e.g., $^{125}$I- test compound) binding to membrane fragments comprising P2Y1-like GPCR polypeptides is carried out as follows in 96 well microtiter plates (e.g., Dynatech Immulon II Removawell plates). Radioligand is diluted in binding buffer+ PMSF/Baci to the desired cpm per 50 μl, then 50 μl aliquots are added to the wells. For non-specific binding samples, 5 μl of 40 μM cold ligand also is added per well. Binding is initiated by adding 150 μl per well of membrane diluted to the desired concentration (10-30 μg membrane protein/well) in binding buffer+ PMSF/Baci. Plates are then covered with Linbro mylar plate sealers (Flow Labs) and placed on a Dynatech Microshaker II. Binding is allowed to proceed at room temperature for 1-2 hours and is stopped by centrifuging the plate for 15 minutes at 2,000 x g. The supernatants are decanted, and the membrane pellets are washed once by addition of 200 μl of ice cold binding buffer, brief shaking, and recentrifugation. The individual wells are placed in 12 x 75 mm tubes and counted in an LKB Gammamaster counter (78% efficiency). Specific binding by this method is identical to that measured when free ligand is removed by rapid (3-5 seconds) filtration and washing on polyethyleneimine-coated glass fiber filters.

Three variations of the standard binding assay are also used.

1. Competitive radioligand binding assays with a concentration range of cold ligand vs. $^{125}$ I-labeled ligand are carried out as described above with one modification. All dilutions of ligands being assayed are made in 40X PMSF/ Baci to a concentration 40X the final concentration in the assay. Samples of peptide (5 μl each) are then added per microtiter well. Membranes and radioligand are diluted in binding buffer without protease inhibitors. Radioligand is added and mixed with cold ligand, and then binding is initiated by addition of membranes.

2. Chemical cross-linking of radioligand with receptor is done after a binding step identical to the standard assay. However, the wash step is done with binding buffer minus BSA to reduce the possibility of non-specific cross-linking of radioligand with BSA. The cross-linking step is carried out as described below.

3. Larger scale binding assays to obtain membrane pellets for studies on solubilization of receptor:ligand complex and for receptor purification are also carried out. These are identical to the standard assays except that (a) binding is carried out in polypropylene tubes in volumes from 1-250 ml, (b) concentration of membrane protein is always 0.5 mg/ml, and (c) for receptor purification, BSA concentration in the binding buffer is reduced to 0.25%, and the wash step is done with binding buffer without BSA, which reduces BSA contamination of the purified receptor.

## EXAMPLE 8

*Chemical Cross-Linking of Radioligand to Receptor*

After a radioligand binding step as described above, membrane pellets are resuspended in 200 μl per microtiter plate well of ice-cold binding buffer without BSA. Then 5 μl per well of 4 mM N-5-azido-2-nitrobenzoyloxysuccinimide (ANB-NOS, Pierce) in DMSO is added and mixed. The samples are held on ice and UV-irradiated for 10 minutes with a Mineralight R-52G lamp (UVP Inc., San Gabriel, Calif.) at a distance of 5-10 cm. Then the samples are transferred to Eppendorf microfuge tubes, the membranes pelleted by centrifugation, supernatants removed, and membranes solubilized in Laemmli SDS sample buffer for polyacrylamide gel electrophoresis (PAGE). PAGE is carried out as described below. Radiolabeled proteins are visualized by autoradiography of the dried gels with Kodak XAR film and DuPont image intensifier screens.

## EXAMPLE 9

*Membrane Solubilization*

Membrane solubilization is carried out in buffer containing 25 mM Tris , pH 8, 10% glycerol (w/v) and 0.2 mM $CaCl_2$ (solubilization buffer). The highly soluble detergents including Triton X-100, deoxycholate, deoxycholate:lysolecithin, CHAPS, and zwittergent are made up in solubilization buffer at 10% concentrations and stored as frozen aliquots. Lysolecithin is made up fresh because of insolubility upon freeze-thawing and digitonin is made fresh at lower concentrations due to its more limited solubility.

To solubilize membranes, washed pellets after the binding step are resuspended free of visible particles by pipetting and vortexing in solubilization buffer at 100,000 x g for 30 minutes. The supernatants are removed and held on ice and the pellets are discarded.

## EXAMPLE 10

*Assay of Solubilized Receptors*

After binding of $^{125}$I ligands and solubilization of the membranes with detergent, the intact R:L complex can be assayed by four different methods. All are carried out on ice or in a cold room at 4-10 °C).

1. Column chromatography (Knuhtsen *et al., Biochem. J. 254*, 641-647, 1988). Sephadex G-50 columns (8 x 250 mm) are equilibrated with solubilization buffer containing detergent at the concentration used to solubilize membranes and 1 mg/ml bovine serum albumin. Samples of solubilized membranes (0.2-0.5 ml) are applied to the columns and eluted at a flow rate of about 0.7 ml/minute. Samples (0.18 ml) are collected. Radioactivity is determined in a gamma counter. Void volumes of the columns are determined by the elution volume of blue dextran. Radioactivity eluting in the void volume is considered bound to protein. Radioactivity eluting later, at the same volume as free $^{125}$I ligands, is considered non-bound.

2. Polyethyleneglycol precipitation (Cuatrecasas, *Proc. Natl. Acad. Sci. USA 69,* 318-322, 1972). For a 100 μl sample of solubilized membranes in a 12 x 75 mm polypropylene tube, 0.5 ml of 1% (w/v) bovine gamma globulin (Sigma) in 0.1 M sodium phosphate buffer is added, followed by 0.5 ml of 25% (w/v) polyethyleneglycol (Sigma) and mixing. The mixture is held on ice for 15 minutes. Then 3 ml of 0.1 M sodium phosphate, pH 7.4, is added per sample. The samples are rapidly (1-3 seconds) filtered over Whatman GF/B glass fiber filters and washed with 4 ml of the phosphate buffer. PEG-precipitated receptor : $^{125}$ I-ligand complex is determined by gamma counting of the filters.

3. GFB/PEI filter binding (Bruns *et al., Analytical Biochem. 132,* 74-81, 1983). Whatman GF/B glass fiber filters are soaked in 0.3% polyethyleneimine (PEI, Sigma) for 3 hours. Samples of solubilized membranes (25-100 μl) are replaced in 12 x 75 mm polypropylene tubes. Then 4 ml of solubilization buffer without detergent is added per sample and the samples are immediately filtered through the GFB/PEI filters (1-3 seconds) and washed with 4 ml of solubilization buffer. CPM of receptor : $^{125}$ I-ligand complex adsorbed to filters are determined by gamma counting.

4. Charcoal/Dextran (Paul and Said, *Peptides 7[Suppl. 1]*,147-149, 1986). Dextran T70 (0.5 g, Pharmacia) is dissolved in 1 liter of water, then 5 g of activated charcoal (Norit A, alkaline; Fisher Scientific) is added. The suspension is stirred for 10 minutes at room temperature and then stored at 4 °C. until use. To measure R:L complex, 4 parts by volume of charcoal/dextran suspension are added to 1 part by volume of solubilized membrane. The samples are mixed and held on ice for 2 minutes and then centrifuged for 2 minutes at 11,000 x g in a Beckman microfuge. Free radioligand is adsorbed charcoal/dextran and is discarded with the pellet. Receptor : $^{125}$ I-ligand complexes remain in the supernatant and are determined by gamma counting.

## EXAMPLE 11

*Receptor Purification*

Binding of biotinyl-receptor to $GH_4$ C1 membranes is carried out as described above. Incubations are for 1 hour at room temperature. In the standard purification protocol, the binding incubations contain 10 nM Bio-S29. $^{125}$I ligand is added as a tracer at levels of 5,000-100,000 cpm per mg of membrane protein. Control incubations contain 10 μM cold ligand to saturate the receptor with non-biotinylated ligand.

Solubilization of receptor:ligand complex also is carried out as described above, with 0.15% deoxycholate: lysolecithin in solubilization buffer containing 0.2 mM $MgCl_2$, to obtain 100,000 x g supernatants containing solubilized R:L complex.

Immobilized streptavidin (streptavidin cross-linked to 6% beaded agarose, Pierce Chemical Co.; "SA-agarose") is washed in solubilization buffer and added to the solubilized membranes as 1/30 of the final volume. This mixture is incubated with constant stirring by end-over-end rotation for 4-5 hours at 4-10 °C. Then the mixture is applied to a column and the non-bound material is washed through. Binding of radioligand to SA-agarose is determined by comparing cpm in the 100,000 x g supernatant with that in the column effluent after adsorption to SA-agarose. Finally, the column is washed with 12-15 column volumes of solubilization buffer+0.15% deoxycholate:lysolecithin +1/500 (vol/vol) 100 x 4pase.

The streptavidin column is eluted with solubilization buffer+0.1 mM EDTA+0.1 mM BGTA+0.1 mM GTP-gamma-S (Sigma)+0.15% (wt/vol) deoxycholate:lysolecithin +1/1000 (vol/vol) 100.times.4pase. First, one column volume of elution buffer is passed through the column and flow is stopped for 20-30 minutes. Then 3-4 more column volumes of elution buffer are passed through. All the eluates are pooled.

Eluates from the streptavidin column are incubated overnight (12-15 hours) with immobilized wheat germ agglutinin (WGA agarose, Vector Labs) to adsorb the receptor via interaction of covalently bound carbohydrate with the WGA lectin. The ratio (vol/vol) of WGA-agarose to streptavidin column eluate is generally 1:400. A range from 1:1000 to 1:200 also can be used. After the binding step, the resin is pelleted by centrifugation, the supernatant is removed and saved, and the resin is washed 3 times (about 2 minutes each) in buffer containing 50 mM HEPES, pH 8, 5 mM $MgCl_2$, and 0.15% deoxycholate:lysolecithin. To elute the WGA-bound receptor, the resin is extracted three times by repeated mixing (vortex mixer on low speed) over a 15-30 minute period on ice, with 3 resin columns each time, of 10 mM N-N'-N"-triacetylchitotriose in the same HEPES buffer used to wash the resin. After each elution step, the resin is centrifuged down and the supernatant is carefully removed, free of WGA-agarose pellets. The three, pooled eluates contain the final, purified receptor. The material non-bound to WGA contain G protein subunits specifically eluted from the streptavidin column, as well as non-specific contaminants. All these fractions are stored frozen at -90 °C.

## EXAMPLE 12

*Identification of test compounds that bind to P2Y1-like GPCR polypeptides*

Purified P2Y1-like GPCR polypeptides comprising a glutathione-S-transferase protein and absorbed onto glutathione-derivatized wells of 96-well microtiter plates are contacted with test compounds from a small molecule library at pH 7.0 in a physiological buffer solution. P2Y1-like GPCR polypeptides comprise an amino acid sequence shown in SEQ ID NO:2. The test compounds comprise a fluorescent tag. The samples are incubated for 5 minutes to one hour. Control samples are incubated in the absence of a test compound.

The buffer solution containing the test compounds is washed from the wells. Binding of a test compound to a P2Y1-like GPCR polypeptide is detected by fluorescence measurements of the contents of the wells. A test compound which increases the fluorescence in a well by at least 15% relative to fluorescence of a well in which a test compound was not incubated is identified as a compound which binds to a P2Y1-like GPCR polypeptide.

## EXAMPLE 13

*Identification of a test compound which decreases P2Y1-like GPCR gene expression*

A test compound is administered to a culture of human gastric cells and incubated at 37 °C for 10 to 45 minutes. A culture of the same type of cells incubated for the same time without the test compound provides a negative control.

RNA is isolated from the two cultures as described in Chirgwin *et al., Biochem. 18,* 5294-99, 1979). Northern blots are prepared using 20 to 30 $\mu$g total RNA and hybridized with a $^{32}$P-labeled P2Y1-like GPCR-specific probe at 65 °C in Expresshyb (CLONTECH). The probe comprises at least 11 contiguous nucleotides selected from the complement of SEQ ID NO:5. A test compound which decreases the P2Y1like GPCR-specific signal relative to the signal obtained in the absence of the test compound is identified as an inhibitor of P2Y1-like GPCR gene expression.

## EXAMPLE 14

*Treatment of a disease in which human P2Y1-like GPCR is overexpressed with a reagent which specifically binds to a P2Y1-like GPCR gene product*

Synthesis of antisense P2Y1-like GPCR oligonucleotides comprising at least 11 contiguous nucleotides selected from the complement of SEQ ID NO:5 is performed on a Pharmacia Gene Assembler series synthesizer using the phosphoramidite procedure (Uhlmann *et al., Chem. Rev. 90,* 534-83, 1990). Following assembly and deprotection, oligonucleotides are ethanol-precipitated twice, dried, and suspended in phosphate-buffered saline (PBS) at the desired concentration. Purity of these oligonucleotides is tested by capillary gel electrophoreses and ion exchange HPLC. Endotoxin levels in the oligonucleotide preparation are determined using the Luminous Amebocyte Assay (Bang, *Biol. Bull. (Woods Hole, Mass.) 105,* 361-362, 1953).

The antisense oligonucleotides are administered to a patient. The severity of the patient's disease is decreased.

## EXAMPLE 15

*Tissue-specific expression of P2Y-Like GPCR*

As a first step to establishing a role for P2Y-like GPCR in the pathogenesis of COPD, expression profiling of the gene was done using real-time quantitative PCR with RNA samples from human respiratory tissues and inflammatory cells relevant to COPD. The panel consisted of total RNA samples lung (adult and fetal), trachea, freshly isolated alveolar type II cells, cultured human bronchial epithelial cells, cultured small airway epithelial cells, cultured bronchial sooth muscle cells, cultured H441 cells (Clara-like), freshly isolated neutrophils and monocytes, and cultured monocytes (macrophage-like). Expression of P2Y-like GPCR also was evaluated in a range of human tissues using total RNA panels obtained from Clontech Laboratories, UK, Ltd.. The tissues were adrenal gland, bone marrow, brain, colon, heart, kidney, liver, lung, mammary gland, pancreas, prostate, salivary gland, skeletal muscle, small intesting, spleen, stomach, testis, thymus, trachea, thyroid, and uterus.

*Real-time quantitative PCR.* Expression profiling of the target gene was performed using real-time quantitative PCR, a development of the kinetic analysis of PCR first described in Higuchi *et al., BioTechnology 10,* 413-17, 1992, and Higuchi *et al.*, *BioTechnology 11,* 1026-30, 1993. The principle is that at any given cycle within the exponential phase of PCR, the amount of product is proportional to the initial number of template copies.

PCR amplification is performed in the presence of an oligonucleotide probe (TaqMan probe) that is complementary to the target sequence and labeled with a fluorescent reporter dye and a quencher dye. During the extension phase of PCR, the probe is cleaved by the 5'-3' endonuclease activity of Taq DNA polymerase, releasing the fluorophore from the effect of the quenching dye (Holland *et al., Proc. Natl. Acad Sci. U.S.A. 88,* 7276-80, 1991). Because the fluorescence emission increases in direct proportion to the amount of the specific amplified product, the exponential growth phase of PCR product can be detected and used to determine the initial template concentration (Heid *et al., Genome Res.* 6, 986-94, 1996, and Gibson *et al.*, *Genome Res. 6*, 995-1001, 1996).

Real-time quantitative PCR was done using an ABI Prism 7700 Sequence Detector. The $C_T$ value generated for each reaciton was used to determine the initial template concentration (copy number) by interpolation from a universal standard curve. The level of expression of the target gene in each sample was calculated relative to the sample with the lowest expression of the gene.

*RNA extraction and cDNA preparation.* Total RNA from each of the respiratory tissues and inflammatory cell types listed above were isolated using Qiagen's RNeasy system according to the manufacturer's protocol (Crawley, West Sussex, UK). The concentration of purified RNA was determined using a RiboGreen RNA quantitation kit (Molecular Probes Europe, The Netherlands). For the preparation of cDNA, 1 $\mu$g of total RNA was reverse transcribed in a final volume of 20 $\mu$l, using 200 U of SUPERSCRIPT™ RNase $H^-$ Reverse Transcriptase (Life Technologies, Paisley, UK), 10 mM dithiothreitol, 0.5 mM of each dNTP and 5 $\mu$M random hexamers (Applied Biosystems, Warrington, Cheshire, UK) according to the manufacturer's protocol.

*TaqMan quantitative analysis.* Specific primers and probe were designed according to the recommendations of PE Applied Biosystems. The probe was labeled at the 5' end with FAM (6-carboxyfluorescein). Quantification PCR was performed with 5 ng of reverse transcribed RNA from each sample. Each determination is done in duplicate.

The assay reaction mix was as follows: 1X final TaqMan Universal PCR Master Mix (from 2X stock) (PE Applied Biosystems, CA); 900 nM forward primer; 900 nM reverse primer; 200 nM probe; 5 ng cDNA; and water to 25 $\mu$l.

Each of the following steps were carried out once: pre PCR, 2 minutes at 50° C, and 10 minutes at 95°C. The following steps are carried out 40 times: denaturation, 15 seconds at 95°C, annealing/extension, 1 minute at 60°C.

All experiments were performed using an ABI Prism 7700 Sequence Detector (PE Applied Biosystems, CA). At the end of the run, fluorescence data acquired during PCR were processed as described in the ABI Prism 7700 user's manual to achieve better background subtraction as well as signal linearity with the starting target quantity.

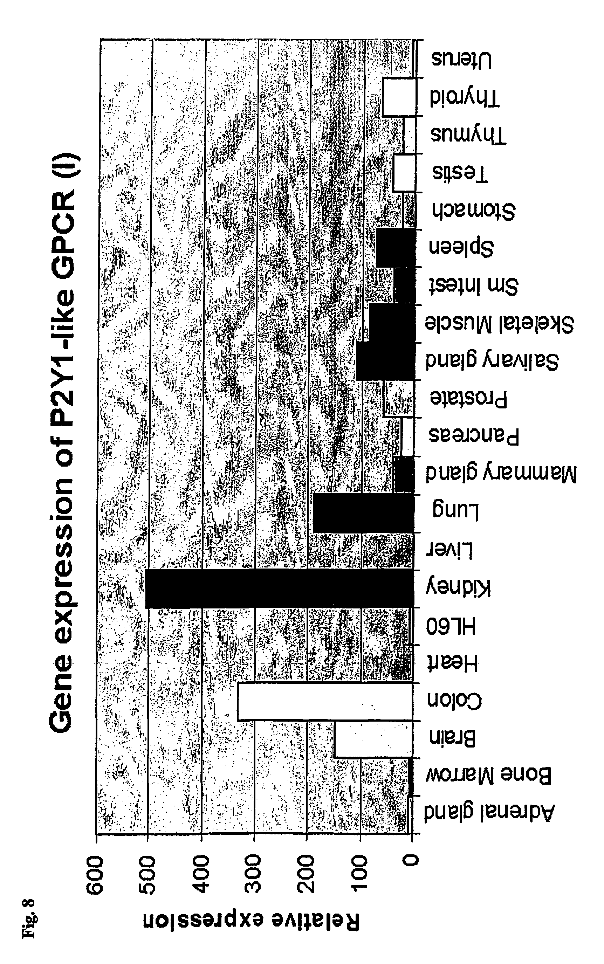

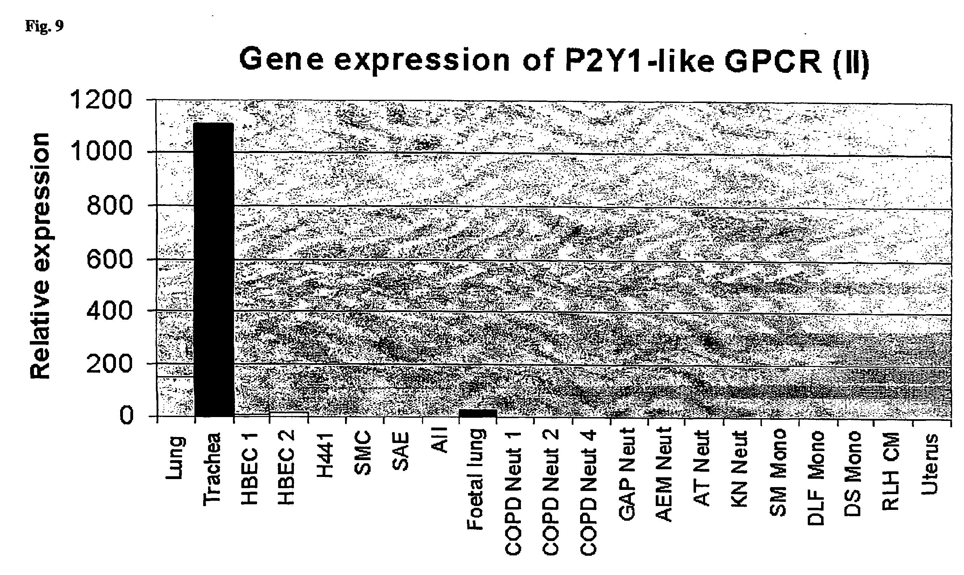

Tables 1 and 2 show the results of expression profiling for P2Y-like GPCR using the indicated cell and tissue samples. For Table 1, the cells are defined as follows: HBEC, cultured human bronchial epithelial cells; H441, a Clara-like cell line; SAE, cultured small airway epithelial cells; SMC, cultured airway smooth muscle cells; AII, freshly isolated human alveolar type II cells; Neut, freshly isolated circulating neutrophils; Mono, freshly isolated monocytes; and CM, cultured monocytes. Other letters identify the donor. The results are shown graphically in FIGS. 8 and 9.

Table 1.

| Tissue | Relative expression |
|---|---|
| Lung | 151.5354699 |
| Trachea | 1107.743218 |
| HBEC 1 | 4.685060463 |
| HBEC 2 | 12.9520377 |
| H441 | 1.202387631 |
| SMC | 1.41842932 |
| SAE | 1.749411592 |
| AII | 1 |
| Foetal lung | 26.72970095 |
| COPD Neut 1 | 0.897587922 |
| COPD Neut 2 | 0.76087585 |
| COPD Neut 4 | 0 |
| GAP Neut | 1.694694812 |
| AEM Neut | 1.331087791 |
| AT Neut | 0 |
| KN Neut | 0.987369255 |
| SM Mono | 0.891902539 |
| DLF Mono | 0.926568646 |
| DS Mono | 1.257087188 |
| RLH CM | 0 |
| Uterus | 0 |

Table 2.

| Tissue | Relative expression |
|---|---|
| Adrenal gland | 9.189455584 |
| Bone Marrow | 5.852208398 |
| Brain | 149.621596 |
| Colon | 333.2508632 |
| Heart | 1.093054592 |
| HL60 | 4.424597636 |
| Kidney | 506.9221673 |
| Liver | 1 |
| Lung | 190.4931147 |
| Mammary gland | 40.91893135 |
| Pancreas | 22.80291052 |
| Prostate | 58.4105483 |
| Salivary gland | 109.5837815 |
| Skeletal Muscle | 84.98460946 |
| Sm Intest | 40.14618513 |
| Spleen | 71.58421767 |
| Stomach | 23.68924846 |
| Testis | 41.70655162 |
| Thymus | 22.94834038 |
| Thyroid | 63.44162273 |
| Uterus | 4.125836209 |

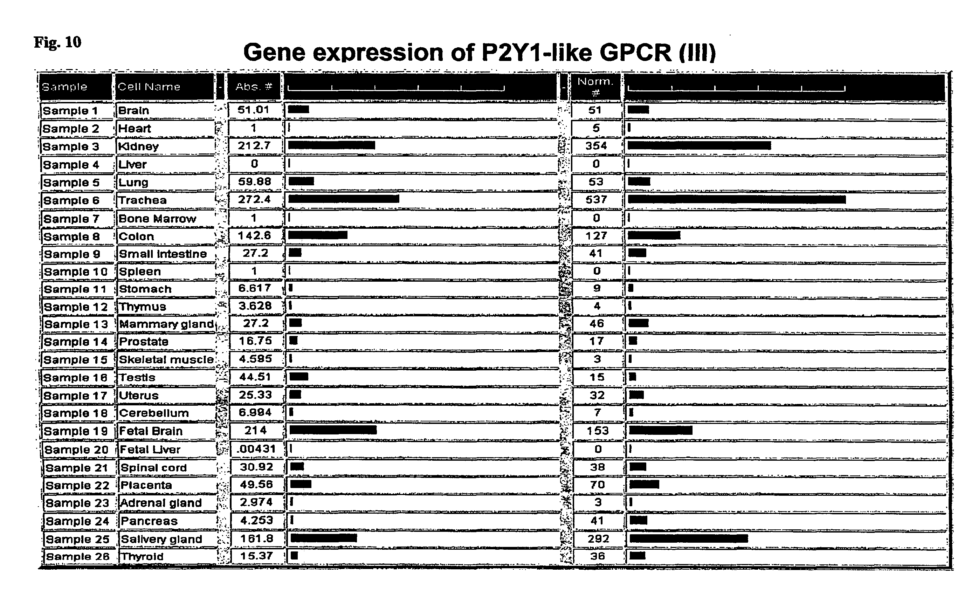

Furthermore, a specific gene expression of P2Y1-like GPCR (III) is shown in Fig. 10. In this experiment a polymerase chain reaction was carried out using oligonucleotide primers LBRI119cds-L2 (ttcggatcgaatctcgcctgct) and LBRI119cds-R2 (tgcttgctcaaggttcccgctta) and measurements of the intensity of emitted light were taken following each cycle of the reaction when the reaction had reached a temperature of 82 degrees C.

**Potential relevance of P2Y1-like GPCR to asthma.**

Extracellular nucleotides induce a wide variety of responses in many cell types, including muscle contraction and relaxation, vasodilation, neurotransmission, platelet aggregation, ion transport regulation, and cell growth. The effects are exerted through P2 receptors, which are classified into two main families: P2X receptors which are ligand-gated ion channels, and P2Y receptors which are G protein-coupled receptors. Twelve distinct P2Y family members have been cloned to date in various species, at least one of which is known to bind a non-nucleotide, namely $P2Y_7$ whose ligand is $LTB_4$. The nuclotide-binding P2Y receptors can be further subdivided into three groups according to ligand specificity: P2Ys activated by adenine nucleotides, P2Ys activated by uridine nucleotides, and P2Ys activated by both adenine and uridine nucleotides.

P2Y1-like GPCR is a new P2Y-like seven-transmembrane-domain molecule that has highest homology to $P2Y_1$. It was originally found in a search for P2Y homologs in genomic sequence databases. Only one EST has been reported to date for this gene, from a cDNA library derived from normal human epithelium. Our own expression profiling of this gene shows that it is expressed highest in the trachea, salivary glands, and kidneys, and less so in fetal brain, colon, placenta, and lung.

Although P2Y1-like GPCR is closest in homology to P2Y1, which binds adenine nucleotides (ATP and ADP), it also has significant homology to $P2Y_2$ and $P2Y_4$, which bind both A and U nucleotides, to $P2Y_3$, which binds U nucleotides, and to leukotriene receptors, which bind $LTB_4$, $LTC_4$, and $LTD_4$. Therefore, although the likely range of ligands that P2Y1-like GPCR can bind is relatively limited, the true ligand of this receptor will have to be determined empirically.

In studies of airway epithelia, both ATP and UTP have been found to equipotently regulate epithelial electrolyte and water transport, trigger mucin secretion, and increase ciliary beat frequency. In the trachea, nucleotides can induce tracheal gland serous cells, which are responsible for the secretion of antibacterial and antiproteolytic proteins, to produce secretory leukocyte proteinase inhibitor and to increase chloride transport. Studies in a mouse knockout of the $P2Y_2$ receptor show that it is the dominant extracellular nucleotide receptor in airway epithelium, but that other nucleotide receptors exist that function similarly in the respiratory tract.

Our expression profiling studies of P2Y1-like GPCR show that it appears to be expressed highly in tissues of the upper respiratory tract. Its high expression in the salivary glands and trachea may indicate that it plays a role in exocrine secretion, which in the airways has mainly a protective role. In asthma, however, overproduction of mucin contributes to the viscid mucus plugs that occlude asthmatic airways. Submucosal glands in the large airways of asthmatics also frequently show evidence of hyperplasia, which may somehow be due to overstimulation by external mediators.

It is therefore unclear at this point what effect agonists or antagonists of the P2Y1- like GPCR P2Y receptor would have in asthmatics. Agonists may beneficially increase protective protein secretion, increase ciliary beat rate, and relax smooth muscle, while antagonists may slow mucus production and glandular hyperplasia.

**References.**

Nandanan E, Jang SY, Moro S, Kim HO, Siddiqui MA, Russ P, Marquez VE, Busson R, Herdewijn P, Harden TK, Boyer JL, Jacobson KA. Synthesis, biological activity, and molecular modeling of ribose-modified deoxyadenosine bisphosphate analogues as P2Y(1) receptor ligands. *J Med Chem.* 2000 Mar 9;43(5):829-42.

Major DT, Halbfinger E, Fischer B. Molecular recognition of modified adenine nucleotides by the P2Y(1)-receptor. 2. A computational approach. *J Med Chem. 1999 Dec 30;42(26):5338-47*.

Halbfinger E, Major DT, Ritzmann M, Ub1 J, Reiser G, Boyer JL, Harden KT, Fischer B. Molecular recognition of modified adenine nucleotides by the P2Y(1)-receptor. 1. A synthetic, biochemical, and NMR approach. *J Med Chem.* 1999 Dec 30;42(26):5325-37.

Moro S, Hoffmann C, Jacobson KA. Role of the extracellular loops of G protein-coupled receptors in ligand recognition: a molecular modeling study of the human P2Y1 receptor. *Biochemistry.* 1999 Mar 23;38(12):3498-507.

Moro S, Guo D, Camaioni E, Boyer JL, Harden TK, Jacobson KA. Human P2Y1 receptor: molecular modeling and site-directed mutagenesis as tools to identify agonist and antagonist recognition sites. *J Med Chem.* 1998 Apr 23;41(9): 1456-66.

Webb TE, Simon J, Barnard EA. Regional distribution of [35S]2'-deoxy 5'-O-(1-thio) ATP binding sites and the P2Y1 messenger RNA within the chick brain. *Neuroscience.* 1998 Jun;84(3):825-37.

Homolya L, Watt WC, Lazarowski ER, Koller BH, Boucher RC. Nucleotide- regulated calcium signaling in lung fibroblasts and epithelial cells from normal and P2Y(2) receptor (-/-) mice. *J Biol Chem.* 1999 Sep 10;274(37):26454-60.

Leon C, Hechler B, Vial C, Leray C, Cazenave JP, Gachet C. The P2Y1 receptor is an ADP receptor antagonized by ATP and expressed in platelets and megakaryoblastic cells. *FEBS Lett.* 1997 Feb 10;403(1):26-30.
Dehaye JP, Moran A, Marino A. Purines, a new class of agonists in salivary glands? *Arch Oral Biol.* 1999 May;44 Suppl 1:S39-43.
King BF, Townsend-Nicholson A, Burnstock G. Metabotropic receptors for ATP and UTP: exploring the correspondence between native and recombinant nucleotide receptors. *Trends Pharmacol Sci.* 1998 Dec;19(12):506-14.
Saleh A, Figarella C, Kammouni W, Marchand-Pinatel S, Lazdunski A, Tubul A, Brun P, Merten MD. Pseudomonas aeruginosa quorum-sensing signal molecule N-(3-oxododecanoyl)-L-homoserine lactone inhibits expression of P2Y receptors in cystic fibrosis tracheal gland cells. *Infect Immun.* 1999 Oct;67(10):5076-82.
Cressman VL, Lazarowski E, Homolya L, Boucher RC, Koller BH, Grubb BR. Effect of loss of P2Y(2) receptor gene expression on nucleotide regulation of murine epithelial Cl(-) transport. *J Biol Chem.* 1999 Sep 10;274(37):26461-8.

Further embodiments of the invention are determined below:

1. An isolated polynucleotide encoding a P2Y1-like GPCR polypeptide and being selected from the group consisting of:

a) a polynucleotide encoding aP2Y1-like GPCR polypeptide comprising an amino acid sequence selected form the group consisting of:

amino acid sequences which are at least about 50% identical to the amino acid sequence shown in SEQ ID NO: 2; and

the amino acid sequence shown in SEQ ID NO: 2.

b) a polynucleotide comprising the sequence of SEQ ID NO: 1, 3, 4 or 5;

c) a polynucleotide which hybridizes under stringent conditions to a polynucleotide specified in (a) and (b);

d) a polynucleotide the sequence of which deviates from the polynucleotide sequences specified in (a) to (c) due to the degeneration of the genetic code; and

e) a polynucleotide which represents a fragment, derivative or allelic variation of a polynucleotide sequence specified in (a to (d).

2. An expression vector containing any polynucleotide as determined in 1. above.

3. A host cell containing the expression vector as determined in 2. above.

4. A substantially purified P2Y1-like GPCR polypeptide encoded by a polynucleotide as determined in 1. above.

5. A method for producing a P2Y1-like GPCR polypeptide, wherein the method comprises the following steps:

a) culturing the host cell as determined in 3. above under conditions suitable for the expression of the P2Y1-like GPCR polypeptide; and

b) recovering the P2Y1-like GPCR polypeptide from the host cell culture.

6. A method for detection of a polynucleotide encoding a P2Yl-like GPCR polypeptide in a biological sample comprising the following steps:

a) hybridizing any polynucleotide as determined in 1, above to a nucleic acid material of a biological sample, thereby forming a hybridization complex; and

b) detecting said hybridization complex.

7. The method as determined in 6. above, wherein before hybridization, the nucleic acid material of the biological sample is amplified.

8. A method for the detection of a polynucleotide as determined in 1. above or a P2Yl-like GPCR polypeptide as determined in 4. above comprising the steps of:

contacting a biological sample with a reagent which specifically interacts with the polynucleotide or the P2Y1-like GPCR polypeptide.

9. A diagnostic kit for conducting the method as determined in 6. to 8. above.

10. A method of screening for agents which decrease the activity of a P2Y1-like GPCR, comprising the steps of:

contacting a test compound with any P2Y1-like GPCR polypeptide encoded by any polynucleotide as determined in 1. above;

detecting binding of the test compound to the P2Y1-like GPCR polypeptide, wherein a test compound which binds to the polypeptide is identified as a potential therapeutic agent for decreasing the activity of a P2Y1-like GPCR.

11. A method of screening for agents which regulate the activity of a P2Yl-like-GPCR, comprising the steps of:

contacting a test compound with a P2Y1-like GPCR polypeptide encoded by any polynucleotide as determined in 1. above; and

detecting a P2Y1-like GPCR activity of the polypeptide, wherein a test compound which increases the P2Y1-like GPCR activity is identified as a potential therapeutic agent for increasing the activity of the P2Y1-like GPCR, and wherein a test compound which decreases the P2Y1-like GPCR activity of the polypeptide is identified as a potential therapeutic agent for decreasing the activity of the P2Y1-like GPCR.

12. A method of screening for agents which decrease the activity of a P2Y1-like GPCR, comprising the steps of:

contacting a test compound with any polynucleotide as determined in 1. above and detecting binding of the test compound to the polynucleotide, wherein a test compound which binds to the polynucleotide is identified as a potential therapeutic agent for decreasing the activity of P2Y1-like GPCR.

13. A method of reducing the activity of P2Y1-like GPCR, comprising the steps of:

contacting a cell with a reagent which specifically binds to any polynucleotide as determined in 1. above or any P2Y1-like GPCR polypeptide as determined in 4. above, whereby the activity of P2Y1-like GPCR is reduced.

14. A reagent that modulates the activity of a P2Y1-like GPCR polypeptide or a polynucleotide wherein said reagent is identified by the method as determined in 10. to 12. above.

15. A pharmaceutical composition, comprising:

the expression vector as determined in 2. above or the reagent as determined in 14. above and a pharmaceutically acceptable carrier.

16. Use of the pharmaceutical composition as determined in 15. above for modulating the activity of a P2Y1-like GPCR in a disease.

17. Use as determined in 16. above wherein the disease is a bacterial, fungal, protozoan, and viral infection, pain, cancer, anorexia, bulimia, asthma, CNS disease, acute heart failure, hypotension, hypertension, urinary retention, osteoporosis, diabetes, angina pectoris, myocardial infarction, ulcer, inflammation, allergy, multiple sclerosis, benign prostatic hypertrophy, a psychotic, a neurological disorder or dyskinesia.

18. A cDNA encoding a polypeptide comprising the amino acid sequence shown in SEQ ID NO: 2.

19. The cDNA as determined in 18. above which comprises SEQ ID NO: 1, 3, 4 or 5.

20. The cDNA as determined in 18. above which consists of SEQ ID NO: 1, 3, 4 or 5.

21. An expression vector comprising a polynucleotide which encodes a polypeptide comprising the amino acid sequence shown in SEQ ID NO: 2.

22. The expression vector as determined in 21. above wherein the polynucleotide consists of SEQ ID NO: 1, 3, 4 or 5.

23. A host cell comprising an expression vector which encodes a polypeptide comprising the amino acid sequence shown in SEQ ID NO: 2.

24. The host cell as determined in 23. above wherein the polynucleotide consists of SEQ ID NO: 1, 3, 4 or 5.

25. A purified polypeptide comprising the amino acid sequence shown in SEQ ID NO: 2.

26. The purified polypeptide as determined in 25. above which consists of the amino acid sequence shown in SEQ ID NO: 2.

27. A fusion protein comprising a polypeptide having the amino acid sequence shown in SEQ ID NO: 2.

28. A method of producing a polypeptide comprising the amino acid sequence shown in SEQ ID NO: 2, comprising the steps of:

culturing a host cell comprising an expression vector which encodes the polypeptide under conditions whereby the polypeptide is expressed; and isolating the polypeptide.

29. The method as determined in 28. above wherein the expression vector comprises SEQ ID NO: 1, 3, 4 or 5.

30. A method of detecting a coding sequence for a polypeptide comprising the amino acid sequence shown in SEQ ID NO: 2, comprising the steps of:

hybridizing a polynucleotide comprising 11 contiguous nucleotides of SEQ ID NO: 1, 3, 4 or 5 to nucleic acid material of a biological sample, thereby forming a hybridization complex; and

detecting the hybridization complex.

31. The method as determined in 30. above further comprising the step of amplifying the nucleic acid material before the step of hybridizing.

32. A kit for detecting a coding sequence for a polypeptide comprising the amino acid sequence shown in SEQ ID NO: 2, comprising:

a polynucleotide comprising 11 contiguous nucleotides of SEQ ID NO: 1, 3, 4 or 5; and
instructions for the method as determined in 30. above.

33. A method of detecting a polypeptide comprising the amino acid sequence shown in SEQ ID NO: 2, comprising the steps of:

contacting a biological sample with a reagent that specifically binds to the polypeptide to form a reagent-polypeptide complex; and detecting the reagent-polypeptide complex.

34. The method as determined in 33. above wherein the reagent is an antibody.

35. A kit for detecting a polypeptide comprising the amino acid sequence shown in SEQ ID NO: 2, comprising:

an antibody which specifically binds to the polypeptide; and instructions for the method as determined in 33. above.

36. A method of screening for agents which can modulate the activity of a human P2Y1-like GPCR, comprising the steps of:

contacting a test compound with a polypeptide comprising an amino acid sequence selected from the group consisting of: (1) amino acid sequences which are at least about 50% identical to the amino acid sequence shown in SEQ ID NO: 2 and (2) the amino acid sequence shown in SEQ ID NO: 2; and

detecting binding of the test compound to the polypeptide, wherein a test compound which binds to the polypeptide is identified as a potential agent for regulating activity of the human P2Y1-like GPCR.

37. The method as determined in 36. above wherein the step of contacting is in a cell.

38. The method as determined in 36. above wherein the cell is in vitro.

39. The method as determined in 36. above wherein the step of contacting is in a cell-free system.

40. The method as determined in 36. above wherein the polypeptide comprises a detectable label.

41. The method as determined in 36. above wherein the test compound comprises a detectable label.

42. The method as determined in 36. above wherein the test compound displaces a labeled ligand which is bound to the polypeptide.

43. The method as determined in 36. above wherein the polypeptide is bound to a solid support.

44. The method as determined in 36. above wherein the test compound is bound to a solid support.

45. A method of screening for agents which modulate an activity of a human P2Y1-like GPCR, comprising the steps of:

contacting a test compound with a polypeptide comprising an amino acid sequence selected from the group consisting of: (1) amino acid sequences which are at least about 50% identical to the amino acid sequence shown in SEQ ID NO: 2 and (2) the amino acid sequence shown in SEQ ID NO: 2; and

detecting an activity of the polypeptide, wherein a test compound which increases the activity of the polypeptide is identified as a potential agent for increasing the activity of the human P2Y1-like GPCR, and wherein a test compound which decreases the activity of the polypeptide is identified as a potential agent for decreasing the activity of the human P2Y1-like GPCR.

46. The method as determined in 45. above wherein the step of contacting is in a cell.

47. The method as determined in 45. above wherein the cell is in vitro.

48. The method as determined in 45. above wherein the step of contacting is in a cell-free system.

49. A method of screening for agents which modulate an activity of a human P2Y1-like GPCR, comprising the steps of:

contacting a test compound with a product encoded by a polynucleotide which comprises the nucleotide sequence shown in SEQ ID NO: 1, 3, 4 or 5; and

detecting binding of the test compound to the product, wherein a test compound which binds to the product is identified as a potential agent for regulating the activity of the human P2Y1-like GPCR.

50. The method as determined in 49. above wherein the product is a polypeptide.

51. The method as determined in 49. above wherein the product is RNA.

52. A method of reducing activity of a human P2Y1-like GPCR, comprising the step of:

contacting a cell with a reagent which specifically binds to a product encoded by a polynucleotide comprising the nucleotide sequence shown in SEQ ID NO: 1,3, 4 or 5, whereby the activity of a human P2Y1-like GPCR is reduced.

53. The method as determined in 52. above wherein the product is a polypeptide.

54. The method as determined in 53. above wherein the reagent is an antibody.

55. The method as determined in 52. above wherein the product is RNA.

56. The method as determined in 55. above wherein the reagent is an antisense oligonucleotide.

57. The method as determined in 56. above wherein the reagent is a ribozyme.

58. The method as determined in 52. above wherein the cell is *in vitro.*

59. The method as determined in 52. above wherein the cell is *in vivo.*

60. A pharmaceutical composition, comprising:

a reagent which specifically binds to a polypeptide comprising the amino acid sequence shown in SEQ ID NO: 2; and a pharmaceutically acceptable carrier.

61. The pharmaceutical composition as determined in 60. above wherein the reagent is an antibody.

62. A pharmaceutical composition, comprising:

a reagent which specifically binds to a product of a polynucleotide comprising the nucleotide sequence shown in SEQ ID NO: 1, 3, 4 or 5; and

a pharmaceutically acceptable carrier.

63. The pharmaceutical composition as determined in 62. above wherein the reagent is a ribozyme.

64. The pharmaceutical composition as determined in 62. above wherein the reagent is an antisense oligonucleotide.

65. The pharmaceutical composition as determined in 62. above wherein the reagent is an antibody.

66. A pharmaceutical composition, comprising:

an expression vector encoding a polypeptide comprising the amino acid sequence shown in SEQ ID NO: 2; and

a pharmaceutically acceptable carrier.

67. The pharmaceutical composition as determined in 66. above wherein the expression vector comprises SEQ ID NO: 1, 3, 4 or 5.

68. A method of treating a P2Y1-like GPCR dysfunction related disease, wherein the disease is selected from a bacterial, fungal, protozoan, and viral infection, pain, cancer, anorexia, bulimia, asthma, CNS disease, acute heart failure, hypotension, hypertension, urinary retention, osteoporosis, diabetes, angina pectoris, myocardial infarction, ulcer, inflammation, allergy, multiple sclerosis, benign prostatic hypertrophy, a psychotic, a neurological disorder and dyskinesia, comprising the step of:

administering to a patient in need thereof a therapeutically effective dose of a reagent that modulates a function of a human P2Y1-like GPCR, whereby symptoms of the P2Y1-like GPCR dysfunction related disease are ameliorated.

69. The method as determined in 68. above wherein the reagent is identified by the method as determined in 36. above.

70. The method as determined in 68. above wherein the reagent is identified by the method as determined in 45. above.

71. The method as determined in 68. above wherein the reagent is identified by the method as determined in 49. above.

## Claims

1. Use of a screening method, said method comprising the steps of

   (a) contacting a test compound with a polypeptide comprising an amino acid sequence which is at least 90% identical to the amino acid sequence of SEQ ID NO:2, and

   detecting binding of said test compound to the polypeptide of (a);
   for the identification of compounds useful in the treatment of chronic obstructive pulmonary or airway disease ("COPD").

2. Use of a screening method, said method comprising the steps of

   (a) contacting a test compound with a polypeptide comprising an amino acid sequence which is at least 90% identical to the amino acid sequence of SEQ ID NO:2, and
   (b) detecting an activity of a polypeptide of (a);
   for the identification of compounds useful in the treatment of chronic obstructive pulmonary or airway disease ("COPD").

3. Use of Claim 1 or 2, wherein the contacting step is in a cell.

4. An isolated polynucleotide encoding a P2Y1-like GPCR polypeptide and being selected from the group consisting of:

   a) a polynucleotide encoding a P2Y1-like GPCR polypeptide comprising an amino acid sequence selected form the group consisting of:

      amino acid sequences which are at least about 50% identical to the amino acid sequence shown in SEQ ID NO: 2; and
      the amino acid sequence shown in SEQ ID NO: 2.

   b) a polynucleotide comprising the sequence of SEQ ID NO: 1, 3, 4 or 5;
   c) a polynucleotide which hybridizes under stringent conditions to a polynucleotide specified in (a) and (b);
   d) a polynucleotide the sequence of which deviates from the polynucleotide sequences specified in (a) to (c) due to the degeneration of the genetic code; and
   e) a polynucleotide which represents a fragment, derivative or allelic variation of a polynucleotide sequence specified in (a) to (d) or
   an expression vector containing any polynucleotide as determined above, or
   a host cell containing the expression vector as determined above, or
   a substantially purified P2Y1-like GPCR polypeptide encoded by a polynucleotide as determined above.

5. A method for producing a P2Y1-like GPCR polypeptide, wherein the method comprises the following steps:

   a) culturing the host cell as determined in 4. above under conditions suitable for the expression of the P2Y1-like GPCR polypeptide; and
   b) recovering the P2Y1-like GPCR polypeptide from the host cell culture, or
   a method for detection of a polynucleotide encoding a P2Y1-like GPCR polypeptide in a biological sample comprising the following steps:
   a)' hybridizing any polynucleotide as determined in 4. above to a nucleic acid material of a biological sample, thereby forming a hybridization complex; and
   b)' detecting said hybridization complex, wherein, optionally, before hybridization, the nucleic acid material of the biological sample is amplified, or
   a method for the detection of a polynucleotide as determined in 4. above or a P2Y1-like GPCR polypeptide as determined in 4. above comprising the steps of:

contacting a biological sample with a reagent which specifically interacts with the polynucleotide or the P2Y1-like GPCR polypeptide.

**6.** A method of screening for agents which decrease the activity of a P2Y1-like GPCR, comprising the steps of:

contacting a test compound with any P2Y1-like GPCR polypeptide encoded by any polynucleotide as determined in 4. above;
detecting binding of the test compound to the P2Y1-like GPCR polypeptide, wherein a test compound which binds to the polypeptide is identified as a potential therapeutic agent for decreasing the activity of a P2Y1-like GPCR, or

a method of screening for agents which regulate the activity of a P2YI-like-GPCR, comprising the steps of:

contacting a test compound with a P2Y1-like GPCR polypeptide encoded by any polynucleotide as determined in 4. above; and
detecting a P2Y1-like GPCR activity of the polypeptide, wherein a test compound which increases the P2Y1-like GPCR activity is identified as a potential therapeutic agent for increasing the activity of the P2Y1-like GPCR, and wherein a test compound which decreases the P2Y1-like GPCR activity of the polypeptide is identified as a potential therapeutic agent for decreasing the activity of the P2Y1-like GPCR, or

a method of screening for agents which decrease the activity of a P2Y1-like GPCR, comprising the steps of:

contacting a test compound with any polynucleotide as determined in 4. above and detecting binding of the test compound to the polynucleotide, wherein a test compound which binds to the polynucleotide is identified as a potential therapeutic agent for decreasing the activity of P2Y1-like GPCR.

**7.** A method of reducing the activity of P2Y1-like GPCR, comprising the steps of:

contacting a cell with a reagent which specifically binds to any polynucleotide as determined in 4. above or any P2Y1-like GPCR polypeptide as determined in 4. above, whereby the activity of P2Y1-like GPCR is reduced.

**8.** A reagent that modulates the activity of a P2Y1-like GPCR polypeptide or a polynucleotide wherein said reagent is identified by the method as determined in 6. above.

**9.** A pharmaceutical composition, comprising:

the expression vector as determined in 4. above or the reagent as determined in 8. above and a pharmaceutically acceptable carrier.

**10.** Use of the pharmaceutical composition as determined in 9. above for modulating the activity of a P2Y1-like GPCR in a disease, such as a bacterial, fungal, or protozoan disease, and viral infection, pain, cancer, anorexia, bulimia, asthma, CNS disease, acute heart failure, hypotension, hypertension, urinary retention, osteoporosis, diabetes, angina pectoris, myocardial infarction, ulcer, inflammation, allergy, multiple sclerosis, benign prostatic hypertrophy, a psychotic, a neurological disorder or dyskinesia.

**11.** A cDNA encoding a polypeptide comprising the amino acid sequence shown in SEQ ID NO: 2, or an expression vector comprising a polynucleotide which encodes a polypeptide comprising the amino acid sequence shown in SEQ ID NO: 2, or a host cell comprising an expression vector which encodes a polypeptide comprising the amino acid sequence shown in SEQ ID NO: 2, or a purified polypeptide comprising the amino acid sequence shown in SEQ ID NO: 2, or a fusion protein comprising a polypeptide having the amino acid sequence shown in SEQ ID NO: 2.

**12.** The cDNA as determined in 11. above which comprises SEQ ID NO: 1, 3, 4 or 5, or the expression vector as determined in 11. above wherein the polynucleotide consists of SEQ ID NO: 1, 3, 4 or 5, or the host cell as determined in 11. above wherein the polynucleotide consists of SEQ ID NO: 1, 3, 4 or 5.

**13.** A method of producing a polypeptide comprising the amino acid sequence shown in SEQ ID NO: 2, comprising the steps of:

culturing a host cell comprising an expression vector which encodes the polypeptide under conditions whereby the polypeptide is expressed; and
isolating the polypeptide, wherein, optionally, the expression vector comprises SEQ ID NO: 1, 3, 4 or 5.

**14.** A method of detecting a coding sequence for a polypeptide comprising the amino acid sequence shown in SEQ ID NO: 2, comprising the steps of:

hybridizing a polynucleotide comprising 11 contiguous nucleotides of SEQ ID NO: 1, 3, 4 or 5 to nucleic acid material of a biological sample, thereby forming a hybridization complex; and
detecting the hybridization complex, or
a method of detecting a polypeptide comprising the amino acid sequence shown in SEQ ID NO: 2, comprising the steps of:

contacting a biological sample with a reagent that specifically binds to the polypeptide to form a reagent-polypeptide complex; and detecting the reagent-polypeptide complex, wherein, optionally, the reagent is an antibody.

**15.** A method of screening for agents which can modulate the activity of a human P2Y1-like GPCR, comprising the steps of:

contacting a test compound with a polypeptide comprising an amino acid sequence selected from the group consisting of: (1) amino acid sequences which are at least about 50% identical to the amino acid sequence shown in SEQ ID NO: 2 and (2) the amino acid sequence shown in SEQ ID NO: 2; and
detecting binding of the test compound to the polypeptide, wherein a test compound which binds to the polypeptide is identified as a potential agent for regulating activity of the human P2Y1-like GPCR.

**16.** A method of screening for agents which modulate an activity of a human P2Y1-like GPCR, comprising the steps of:

contacting a test compound with a polypeptide comprising an amino acid sequence selected from the group consisting of: (1) amino acid sequences which are at least about 50% identical to the amino acid sequence shown in SEQ ID NO: 2 and (2) the amino acid sequence shown in SEQ ID NO: 2; and
detecting an activity of the polypeptide, wherein a test compound which increases the activity of the polypeptide is identified as a potential agent for increasing the activity of the human P2Y1-like GPCR, and wherein a test compound which decreases the activity of the polypeptide is identified as a potential agent for decreasing the activity of the human P2Y1-like GPCR.

**17.** A method of screening for agents which modulate an activity of a human P2Y1-like GPCR, comprising the steps of:

contacting a test compound with a product encoded by a polynucleotide which comprises the nucleotide sequence shown in SEQ ID NO: 1, 3, 4 or 5; and
detecting binding of the test compound to the product, wherein a test compound which binds to the product is identified as a potential agent for regulating the activity of the human P2Y1-like GPCR.

**18.** A method of reducing activity of a human P2Y1-like GPCR, comprising the step of:

contacting a cell with a reagent which specifically binds to a product encoded by a polynucleotide comprising the nucleotide sequence shown in SEQ ID NO: 1,3, 4 or 5, whereby the activity of a human P2Y1-like GPCR is reduced.

**19.** A method of treating a P2Y1-like GPCR dysfunction related disease, wherein the disease is selected from a bacterial, fungal, protozoan, and viral infection, pain, cancer, anorexia, bulimia, asthma, CNS disease, acute heart failure, hypotension, hypertension, urinary retention, osteoporosis, diabetes, angina pectoris, myocardial infarction, ulcer, inflammation, allergy, multiple sclerosis, benign prostatic hypertrophy, a psychotic, a neurological disorder and dyskinesia, comprising the step of:

administering to a patient in need thereof a therapeutically effective dose of a reagent that modulates a function of a human P2Y1-like GPCR, whereby symptoms of the P2Y1-like GPCR dysfunction related disease are ameliorated.

**20.** A pharmaceutical composition, comprising: a reagent which specifically binds to a polypeptide comprising the amino acid sequence shown in SEQ ID NO : 2; and a pharmaceutically acceptable carrier.

**21.** A pharmaceutical composition, comprising: a reagent which specifically binds to a product of a polynucleotide comprising the nucleotide sequence shown in SEQ ID NO: 1,3,4 or 5; and a pharmaceutically acceptable carrier.

**22.** A pharmaceutical composition, comprising: an expression vector encoding a polypeptide comprising the amino acid sequence shown in SEQ ID NO : 2; and a pharmaceutically acceptable carrier.

**Fig. 1**

5′ end

TGGCTTTTGAGTCAGAAAAATGCGAGTGTGGATCCCAGCTAAGCCCCT
AGATGTGTTACCAGAGACGAGCCGCAAAACATTTCTCAGTCTTAGTCT
CTTGTAAAATAGAGGTAATAAGAAACACTTTTCAGTATTTTGTGACAT
GTAGAAGTAAGTGATGGTGGCATGCATCACACTTGGTTAATAGTAGGT
CCTGTTGTTAAGTCTCTAATGGCGATACCCTATGGCTTCTCCAAATGG
TGACCTTGCCAAATTGTTTTCCAAAGCGACATGTGGCTTTTTTCTCCC
AATCCCTCATTTTAACTCTCATGGTAATTTAACTTTTATATTTTTATT
AGATGCATTTAGTAACTTGCCTCATAGTCATTTTCTTGGAAATTCAAT
TTCTTCTCCACAGGGTCTCTTTTGAGATTAAAGAGAGAGAAGTGGCAA
ATTTAGGATGTTAGAATAATTTTCATTTAAAAGTAGATCCTTGTTTTT
ATTACCCTATCATTAATGTTTTCTGTTTTCCTTTATCAGCGAGTTACT
GCTCATTTGATTCATATTGCCAAACTGAACTCTCTTGTTTTCTTGCAA
GATGAAAGGAGACAACC

**Fig. 2**

MNEPLDYLANASDFPDYAAAFGNCTDENIPLKMHYLPVIYGIIFLVGF
PGNAVVISTYIFKMRPWKSSTIIMLNLACTDLLYLTSLPFLIHYYASG
ENWIFGDFMCKFIRFSFHFNLYSSILFLTCFSIFRYCVIIHPMSCFSI
HKTRCAVVACAVVWIISLVAVIPMTFLITSTNRTNRSACLDLTSSDEL
NTIKWYNLILTATTFCLPLVIVTLCYTTIIHTLTHGLQTDSCLKQKAR
RLTILLLLAFYVCFLPFHILRVIRIESRLLSISCSIENQIHEAYIVSR
PLAALNTFGNLLLYVVVSDNFQQAVCSTVRCKVSGNLEQAKKISYSNN
P

**Fig. 3**

3'END

AATATTTCATTTACTTAACCAAAAACAAATACTTGCTGATACTTTACC
TAGCATCCTAAGATGTTCAGGATGTCTCCCTCAATGGAACTCCTGGTA
AATACTGTGTATTCAAGTAATCATGTGCCAAAGCCAGGGCAGAGCTTC
TAGTTCTTTGCAATCCCTTTATTGAGCTCCTCCACTGGGGAGATATAA
GAATGGGATGCATGTATATCAGCAAAGTATTCAGACATAGTATTACAA
GCTATTGGAACTCAGAGGCATCTTAGAGAACATCTGTTCCCACCAACT
TACTATATATACACGGAAACCAATTTCTTACCCTTGCCCTAGATTGCT
CAGTAAATTTGTGCCAAGATAGGAGAAAACCAATCTTTTCACTCATCA
TTTCATGCTTCTCTGCACTCTGGGCCTATTTGTATTGAACCATTAGAC
AATTCAAACCACTACTTGTATCTTTCTTAATATTTATTTTTTACATCT
CAGAGCTCTACAATTTGTTTCCTTCAAGCTTAACTTTGAGATTATAAA
ACTGGGTTTAGCCAGTTCTGTATATTACTTCAAGCCAGTAAGATACCC
TTGAAATAATCCAAGGACGTCCATGCAAATAGTTGAAATTAGTACCTG
CAATATATTTGGAGTATTATGTCTTTATTGTTGTTAAAAAGTTTTTAT
T

Fig. 4

>promoter region with start ATG

aacaagactgccacagtcgtatcctggctcacccctaactagcggttt
gactttgagcacatcactgcctcctccgtgcctcagtttcctcttctg
ttgaatggggatgatcacagcactacctcaccatttgttgtgaggatt
aaatagattcaaatatgtaagacacttacagtaatgctttgtacacag
aaggcactattatttttattaatccctatttttccttcccaacttca
tctcccaatatcccataaccatgctgatttccttacaactcccccaga
cctcctggactaagtgagatttggaagagtatactcagggcagtcaag
aaggactgattctgctaatttaggatttgtcaagttgggttagtttga
aatatacctctgtactcctctcaggaaatgctggatagtgtagattta
gtatcagataatagtaataatggtggcagactctgatgtttcctcctt
cccttgtgatctcagtagtcttgtaccattcatacatcaaatgactta
gcaactaacaatcacgtaatatttttaagcttttcttatgtgcccag
tagtgagccaagagtttttttcctatttttaacatactattgacagc
tccatgaggtgggtttatattttgttaaaataatttgccatgtccact
tccccagtagactgtgacagtggcatatacatctgtgtgtgttattca
gtttgtatctctaggacttgacagaggacccaatacagagtgggtgct
ccagaaatgtggacttggatgcatttcaccaacagctatttacatcca
aataataagctaattataactcttcttctagatgtacttttctcattg
caaaaatcaccgtcaaaatttttaaaaacactaataagcaagaagaag
gaagttaaaattgcactccttccagccctagagagcttaatcaacttg
cttaggaacacgctactaaatggctaatctgagcaccaaaatctggcc
atctggcttcagggctcagcctcctaatggccgcattataccattgat
cccgccccatccaccccaacacacacactcccctgcacaaacaaatta
tgttagctattaaaaggaattggaagagtggaaatgtgcaattaaatc
taccacaaatacaatgttgcaatacgtgcaatctatatttttaaaatt
tacagtattcagcattttcctgtcttcacacttgttccctccgttatt
tacggcaatcatgacgaaaatcaacaaaatacagattgagaggcagca
ctgagaggtaatgtagaaagggaatggcttttgagtcagaaaaatgcg
agtgtggatcccagctaagcccctagatgtgttaccagagacgagccg
caaaacatttctcagtcttagtctcttgtaaaatagaggtaataagaa
acacttttcagtattttgtgacatgtagaagtaagtgatggtggcatg
catcacacttggttaatagtaggtcctgttgttaagtctctaatggcg
ataccctatggcttctccaaatggtgaccttgccaaattgttttccaa
agcgacatgtggctttttttctcccaatccctcattttaactctcatgg
taatttaacttttatatttttattagatgcatttagtaacttgcctca
tagtcatttttcttggaaattcaatttcttctccacagggtctcttttg
agattaaagagagagaagtggcaaatttaggatgttagaataatttc
atttaaaagtagatccttgtttttattaccctatcattaatgttttct
gttttcctttatcagcgagttactgctcatttgattcatattgccaaa
ctgaactctcttgtttttcttgcaagatgaaaggagacaaccATGaatg
agccactagactatttagcaaatgcttctgatttccccgattatgcag
ctgcttttggaaattgcactgatgaaaacatcccactcaagatgcact

acctccctgttatttatggcattatcttcctcgtgggatttccaggca
atgcagtagtgatatccacttacattttcaaaatgagaccttggaaga
gcagcaccatcattatgctgaacctggcctgcacagatctgctgtatc
tgaccagcctccccttcctgattcactactatgccagtggcgaaaact
ggatctttggagatttcatgtgtaagtttatccgctt

TATA box found by TSS program

**Fig. 5**

atgaatgagccactagactatttagcaaatgcttctgatttccccgat
tatgcagctgcttttggaaattgcactgatgaaaacatcccactcaag
atgcactacctccctgttatttatggcattatcttcctcgtgggattt
ccaggcaatgcagtagtgatatccacttacattttcaaaatgagacct
tggaagagcagcaccatcattatgctgaacctggcctgcacagatctg
ctgtatctgaccagcctccccttcctgattcactactatgccagtggc
gaaaactggatctttggagatttcatgtgtaagtttatccgcttcagc
ttccatttcaacctgtatagcagcatcctcttcctcacctgtttcagc
atcttccgctactgtgtgatcattcacccaatgagctgcttttccatt
cacaaaactcgatgtgcagttgtagcctgtgctgtggtgtggatcatt
tcactggtagctgtcattccgatgaccttcttgatcacatcaaccaac
aggaccaacagatcagcctgtctcgacctcaccagttcggatgaactc
aatactattaagtggtacaacctgatttgactgcaactactttctgc
ctccccttggtgatagtgacactttgctataccacgattatccacact
ctgacccatggactgcaaactgacagctgccttaagcagaaagcacga
aggctaaccattctgctactccttgcatttacgtatgtttttaccc
ttccatatcttgagggtcattcggatcgaatctcgcctgctttcaatc
agttgttccattgagaatcagatccatgaagcttacatcgtttctaga
ccattagctgctctgaacacctttggtaacctgttactatatgtggtg
gtcagcgacaactttcagcaggctgtctgctcaacagtgagatgcaaa
gtaagcgggaaccttgagcaagcaaagaaaattagttactcaaacaac
ccttga

**Fig. 6**

| | | | |
|---|---|---|---|
| MTEVLWPAVP | NGTDAAFLAG | PGSSWGNSTV | ASTAAVSSSF |
| KCALTKTGFQ | FYYLPAVYIL | VFIIGFLGNS | VAIWMFVFHM |
| KPWSGISVYM | FNLALADFLY | VLTLPALIFY | YFNKTDWIFG |
| DAMCKLQRFI | FHVNLYGSIL | FLTCISAHRY | SGVVYPLKSL |
| GRLKKKNAIC | ISVLVWLIVV | VAISPILFYS | GTGVRKNKTI |
| TCYDTTSDEY | LRSYFIYSMC | TTVAMFCVPL | VLILGCYGLI |
| VRALIYKDLD | NSPLRRKSIY | LVIIVLTVFA | VSYIPFHVMK |
| TMNLRARLDF | QTPAMCAFND | RVYATYQVTR | GLASLNSCVD |
| PILYFLAGDT | FRRRLSRATR | KASRRSEANL | QSKSEDMTLN |
| ILPEFKQNGD | TSL | | |

**FIG. 7**

BLASTP - alignment of GPCR_p2Ylike_Protein against swiss|P49650|P2YR_MOUSE

P2Y PURINOCEPTOR 1 (ATP RECEPTOR) (P2Y1) (PURINERGIC RECEPTOR).//:trembl|U22829|MM22829_1 product: "P2Y purinoceptor"; Mus musculus P2Y purinoceptor mRNA, complete cds. //:trembl|AJ245636|MMU245636_1 gene: "P2Y1"; product: "P2Y1 receptor"; Mus musculus P2Y1 gene for P2Y1 receptor //:gp|U22829|767871 product: "P2Y purinoceptor"; Mus musculus P2Y purinoceptor mRNA, complete cds. //:gp|AJ245636|6013075 gene: "P2Y1"; product: "P2Y1 receptor"; Mus musculus P2Y1 gene for P2Y1 receptor.

```
This hit is scoring at : 2e-58 (expectation value)
Alignment length (overlap) : 299
Identities : 36 %
Scoring matrix : BLOSUM62 (used to infer consensus pattern)
Database searched : nrdb

Q:   24 CTDENIPLKMHYLPVIYGIIFLVGFPGNAVVISTYIFKMRPWKSSTIIMLNLACTDLLYL
        C. ... .:.:YLP.:Y ::F::GF GN:V.I .::F.M:PW.. ::.M.NLA..D.LY:
H:   42 CALTKTGFQFYYLPAVYILVFIIGFLGNSVAIWMFVFHMKPWSGISVYMFNLALADFLYV
```

FIG. 7 (continued)

```
                           *
TSLPFLIHYYASGENWIFGDFMCKFIRFSFHFNLYSSILFLTCFSIFRYCVIIHPMSCFS
.:LP LI.YY :  .:WIFGD MCK. RF FH.NLY.SILFLTC.S..RY.  :::P:....
LTLPALIFYYFNKTDWIFGDAMCKLQRFIFHVNLYGSILFLTCISAHRYSGVVYPLKSLG

                                                *
IHKTRCAVVACAVVWIISLVAVIPMTFLITSTNRTNRS-ACLDLTSSDELNTIKWYNLIL
  K.: A:....:VW:I :VA: P:.F.   : .R.N:: .C.D.TS:D L.:.  Y::..
RLKKKNAIYVSVLVWLIVVVAISPILFYSGTGTRKNKTVTCYDTTSNDYLRSYFIYSMCT

TATTFCLPLVIVTLCYTTIIHTLTHGLQTDSCLKQKARRLTILLLLAFYVCFLPFHILRV
T...FC:PLV::. CY .I:..L.:.   .:S L::K:   L.I::L..F V.::PFH:::.
TVAMFCIPLVLILGCYGLIVKALIYNDLDNSPLRRKSIYLVIIVLTVFAVSYIPFHVMKT

     *               *
IRIESRL---LSISCSIENQIHEAYIVSRPLAALNTFGNLLLYVVVSDNFQQAVCSTVR     318
:.:.:RL    ..  .C..:::::..Y V:R LA:LN:   : :LY.:..D.F::.:....R  
MNLRARLDFQTPEMCDFNDRVYATYQVTRGLASLNSCVDPILYFLAGDTFRRRLSRATR     340
```

Fig. 8

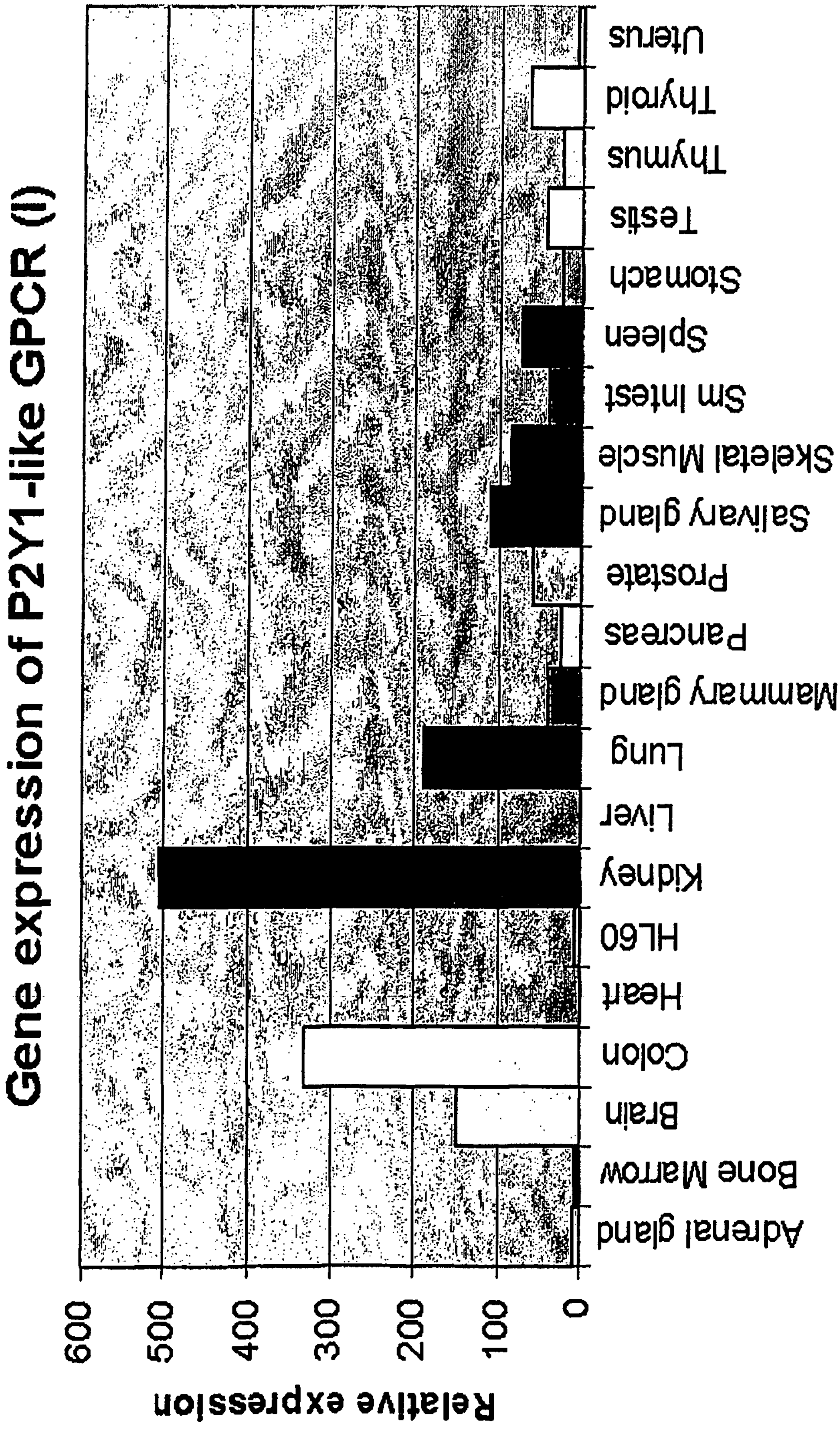

Gene expression of P2Y1-like GPCR (I)
Relative expression
0
100
200
300
400
500
600
Adrenal gland
Bone Marrow
Brain
Colon
Heart
HL60
Kidney
Liver
Lung
Mammary gland
Pancreas
Prostate
Salivary gland
Skeletal Muscle
Sm Intest
Spleen
Stomach
Testis
Thymus
Thyroid
Uterus

Fig. 9

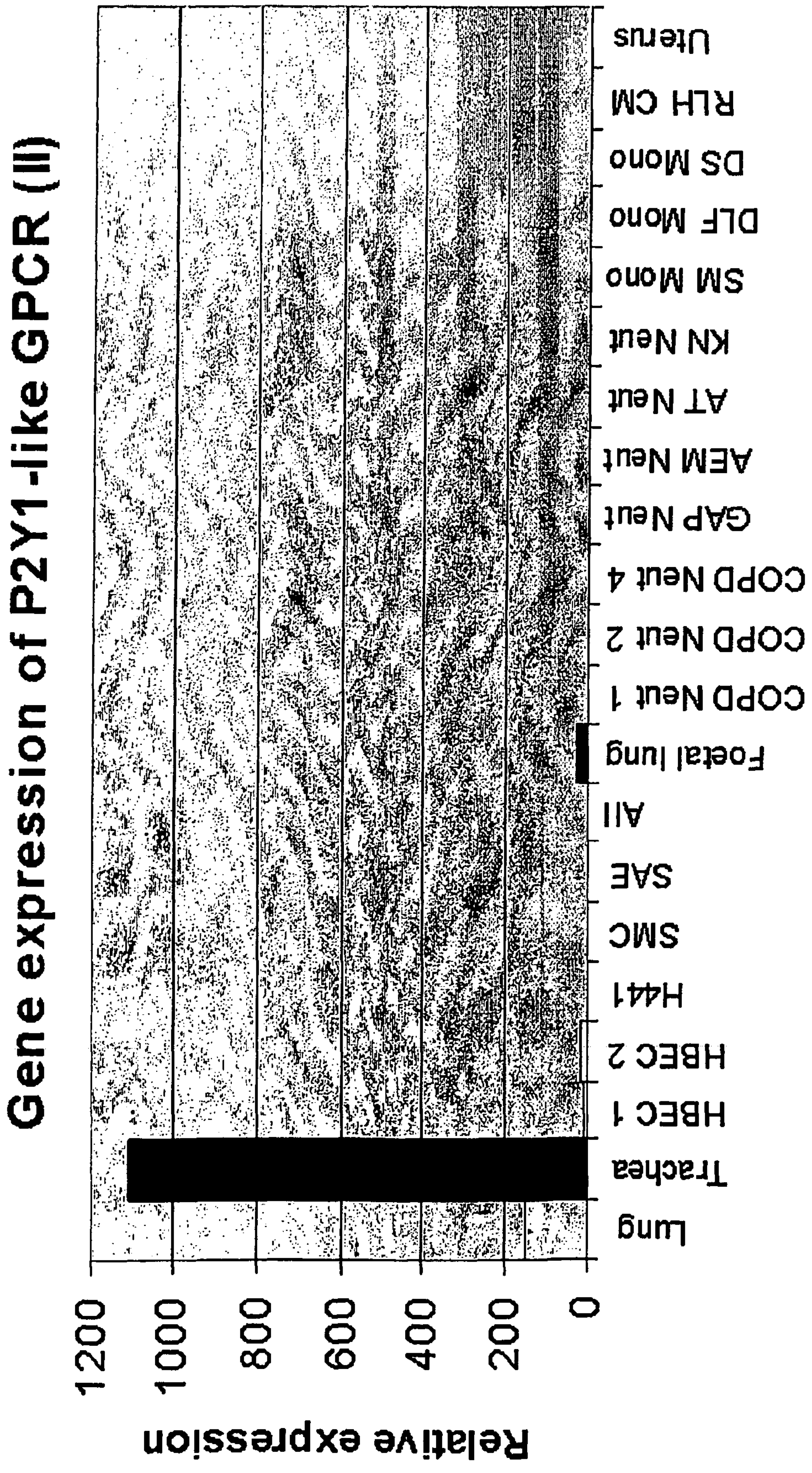
Gene expression of P2Y1-like GPCR (II)
Relative expression
0
200
400
600
800
1000
1200
Lung
Trachea
HBEC 1
HBEC 2
H441
SMC
SAE
AII
Foetal lung
COPD Neut 1
COPD Neut 2
COPD Neut 4
GAP Neut
AEM Neut
AT Neut
KN Neut
SM Mono
DLF Mono
DS Mono
RLH CM
Uterus

Fig. 10

## Gene expression of P2Y1-like GPCR (III)

| Sample | Cell Name | Abs. # | Norm. # |
|---|---|---|---|
| Sample 1 | Brain | 51.01 | 51 |
| Sample 2 | Heart | 1 | 5 |
| Sample 3 | Kidney | 212.7 | 354 |
| Sample 4 | Liver | 0 | 0 |
| Sample 5 | Lung | 59.88 | 53 |
| Sample 6 | Trachea | 272.4 | 537 |
| Sample 7 | Bone Marrow | 1 | 0 |
| Sample 8 | Colon | 142.6 | 127 |
| Sample 9 | Small intestine | 27.2 | 41 |
| Sample 10 | Spleen | 1 | 0 |
| Sample 11 | Stomach | 6.617 | 9 |
| Sample 12 | Thymus | 3.628 | 4 |
| Sample 13 | Mammary gland | 27.2 | 46 |
| Sample 14 | Prostate | 16.75 | 17 |
| Sample 15 | Skeletal muscle | 4.585 | 3 |
| Sample 16 | Testis | 44.51 | 15 |
| Sample 17 | Uterus | 25.33 | 32 |
| Sample 18 | Cerebellum | 6.984 | 7 |
| Sample 19 | Fetal Brain | 214 | 153 |
| Sample 20 | Fetal Liver | .00431 | 0 |
| Sample 21 | Spinal cord | 30.92 | 38 |
| Sample 22 | Placenta | 49.56 | 70 |
| Sample 23 | Adrenal gland | 2.974 | 3 |
| Sample 24 | Pancreas | 4.253 | 41 |
| Sample 25 | Salivery gland | 161.8 | 292 |
| Sample 26 | Thyroid | 15.37 | 36 |

Fig. 11

Gene expression of P2Y1-like GPCR (IV)

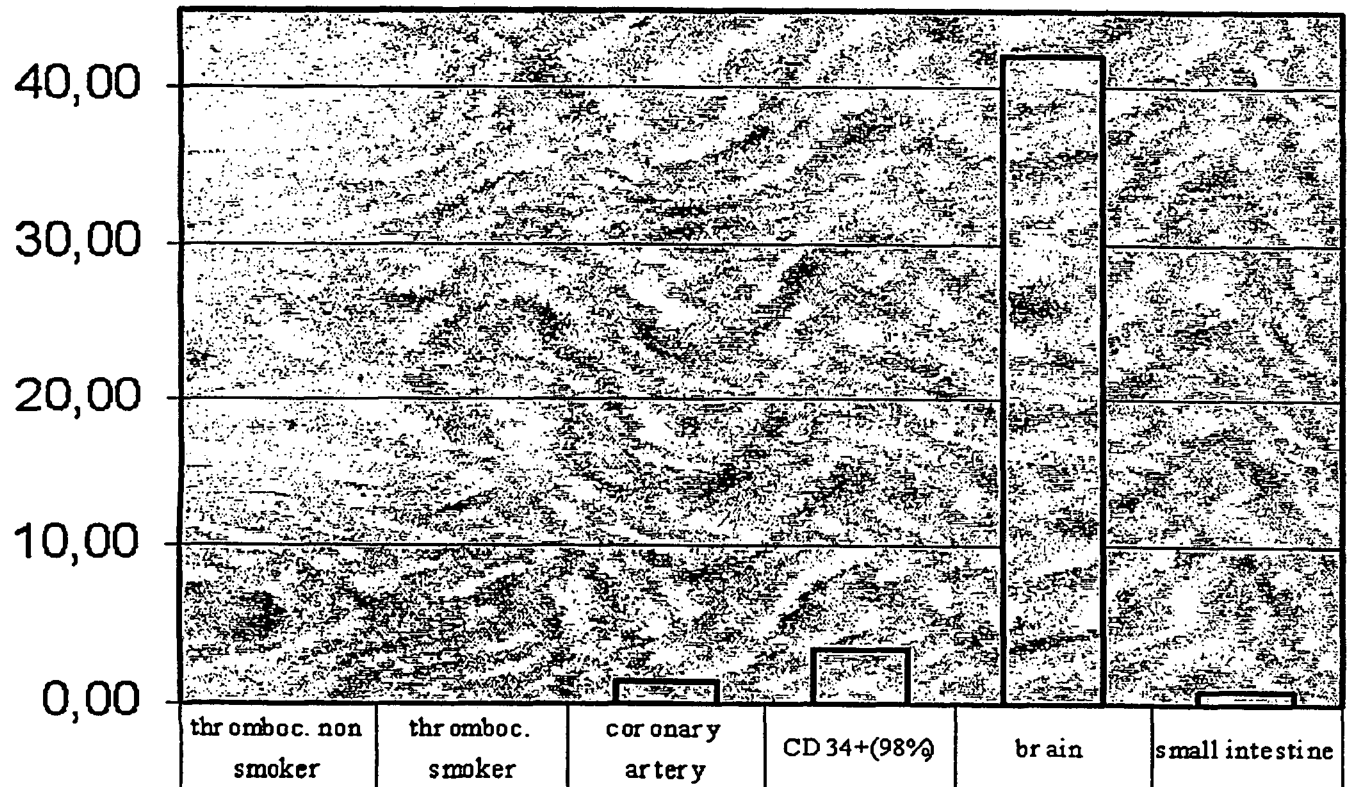


| | thromboc. non smoker | thromboc. smoker | coronary artery | CD34+(98%) | brain | small intestine |
|---|---|---|---|---|---|---|
| rel. expr. | 0,12 | 0,06 | 1,46 | 3,42 | 42,13 | 1,00 |
| Ct | 37,27 | 37,36 | 35,80 | 32,23 | 30,05 | 36,47 |
| dCt | 21,21 | 22,27 | 17,58 | 16,35 | 12,73 | 18,12 |

SEQUENCE LISTING

<110> Bayer AG

<120> REGULATION OF HUMAN P2Y1-LIKE G PROTEIN-COUPLED RECEPTOR

<130> LIO131 Foreign Countries

<150> US 60/224,989
<151> 2000-08-14

<160> 6

<170> PatentIn version 3.1

<210> 1
<211> 593
<212> DNA
<213> Homo sapiens

<400> 1
tggcttttga gtcagaaaaa tgcgagtgtg gatcccagct aagcccctag atgtgttacc 60

agagacgagc cgcaaaacat ttctcagtct tagtctcttg taaaatagag gtaataagaa 120

acacttttca gtattttgtg acatgtagaa gtaagtgatg gtggcatgca tcacacttgg 180

ttaatagtag gtcctgttgt taagtctcta atggcgatac cctatggctt ctccaaatgg 240

tgaccttgcc aaattgtttt ccaaagcgac atgtggcttt tttctcccaa tccctcattt 300

taactctcat ggtaatttaa cttttatatt tttattagat gcatttagta acttgcctca 360

tagtcatttt cttggaaatt caatttcttc tccacagggt ctcttttgag attaaagaga 420

gagaagtggc aaatttagga tgttagaata attttcattt aaaagtagat ccttgttttt 480

attaccctat cattaatgtt ttctgttttc ctttatcagc gagttactgc tcatttgatt 540

catattgcca aactgaactc tcttgttttc ttgcaagatg aaaggagaca acc 593

<210> 2
<211> 337
<212> PRT
<213> Homo sapiens

<400> 2

Met Asn Glu Pro Leu Asp Tyr Leu Ala Asn Ala Ser Asp Phe Pro Asp
1 5 10 15

Tyr Ala Ala Ala Phe Gly Asn Cys Thr Asp Glu Asn Ile Pro Leu Lys
20 25 30

Met His Tyr Leu Pro Val Ile Tyr Gly Ile Ile Phe Leu Val Gly Phe
35 40 45

Pro Gly Asn Ala Val Val Ile Ser Thr Tyr Ile Phe Lys Met Arg Pro
50 55 60

Trp Lys Ser Ser Thr Ile Ile Met Leu Asn Leu Ala Cys Thr Asp Leu
65 70 75 80

Leu Tyr Leu Thr Ser Leu Pro Phe Leu Ile His Tyr Tyr Ala Ser Gly
85 90 95

Glu Asn Trp Ile Phe Gly Asp Phe Met Cys Lys Phe Ile Arg Phe Ser
100 105 110

Phe His Phe Asn Leu Tyr Ser Ser Ile Leu Phe Leu Thr Cys Phe Ser
115 120 125

Ile Phe Arg Tyr Cys Val Ile Ile His Pro Met Ser Cys Phe Ser Ile
130 135 140

His Lys Thr Arg Cys Ala Val Val Ala Cys Ala Val Val Trp Ile Ile
145 150 155 160

Ser Leu Val Ala Val Ile Pro Met Thr Phe Leu Ile Thr Ser Thr Asn
165 170 175

Arg Thr Asn Arg Ser Ala Cys Leu Asp Leu Thr Ser Ser Asp Glu Leu
180 185 190

Asn Thr Ile Lys Trp Tyr Asn Leu Ile Leu Thr Ala Thr Thr Phe Cys
195 200 205

Leu Pro Leu Val Ile Val Thr Leu Cys Tyr Thr Thr Ile Ile His Thr
210 215 220

Leu Thr His Gly Leu Gln Thr Asp Ser Cys Leu Lys Gln Lys Ala Arg
225 230 235 240

Arg Leu Thr Ile Leu Leu Leu Leu Ala Phe Tyr Val Cys Phe Leu Pro
245 250 255

Phe His Ile Leu Arg Val Ile Arg Ile Glu Ser Arg Leu Leu Ser Ile
260 265 270

Ser Cys Ser Ile Glu Asn Gln Ile His Glu Ala Tyr Ile Val Ser Arg
275 280 285

Pro Leu Ala Ala Leu Asn Thr Phe Gly Asn Leu Leu Leu Tyr Val Val
290 295 300

Val Ser Asp Asn Phe Gln Gln Ala Val Cys Ser Thr Val Arg Cys Lys

305 310 315 320

Val Ser Gly Asn Leu Glu Gln Ala Lys Lys Ile Ser Tyr Ser Asn Asn
325 330 335

Pro

<210> 3
<211> 673
<212> DNA
<213> Homo sapiens

<400> 3
aatatttcat ttacttaacc aaaaacaaat acttgctgat actttaccta gcatcctaag 60

atgttcagga tgtctccctc aatggaactc ctggtaaata ctgtgtattc aagtaatcat 120

gtgccaaagc cagggcagag cttctagttc tttgcaatcc ctttattgag ctcctccact 180

ggggagatat aagaatggga tgcatgtata tcagcaaagt attcagacat agtattacaa 240

gctattggaa ctcagaggca tcttagagaa catctgttcc caccaactta ctatatatac 300

acggaaacca atttcttacc cttgccctag attgctcagt aaatttgtgc caagatagga 360

gaaaaccaat cttttcactc atcatttcat gcttctctgc actctgggcc tatttgtatt 420

gaaccattag acaattcaaa ccactacttg tatctttctt aatatttatt ttttacatct 480

cagagctcta caatttgttt ccttcaagct taactttgag attataaaac tgggtttagc 540

cagttctgta tattacttca agccagtaag ataccttga aataatccaa ggacgtccat 600

gcaaatagtt gaaattagta cctgcaatat atttggagta ttatgtcttt attgttgtta 660

aaaagttttt att 673

<210> 4
<211> 2245
<212> DNA
<213> Homo sapiens

<220>
<221> misc_feature
<222> (1914)..(1916)
<223> start codon

<220>
<221> promoter
<222> (1112)..(1117)
<223> TATA box

<400> 4
aacaagactg ccacagtcgt atcctggctc acccctaact agcggtttga ctttgagcac 60

atcactgcct cctccgtgcc tcagtttcct cttctgttga atggggatga tcacagcact 120

acctcaccat ttgttgtgag gattaaatag attcaaatat gtaagacact tacagtaatg 180
ctttgtacac agaaggcact attatttttt attaatccct atttttcctt cccaacttca 240
tctcccaata tcccataacc atgctgattt ccttacaact cccccagacc tcctggacta 300
agtgagattt ggaagagtat actcagggca gtcaagaagg actgattctg ctaatttagg 360
atttgtcaag ttgggttagt ttgaaatata cctctgtact cctctcagga aatgctggat 420
agtgtagatt tagtatcaga taatagtaat aatggtggca gactctgatg tttcctcctt 480
cccttgtgat ctcagtagtc ttgtaccatt catacatcaa atgacttagc aactaacaat 540
cacgtaatat tttttaagct tttcttatgt gcccagtagt gagccaagag ttttttttcc 600
tatttttaac atactattga cagctccatg aggtgggttt atattttgtt aaaataattt 660
gccatgtcca cttccccagt agactgtgac agtggcatat acatctgtgt gtgttattca 720
gtttgtatct ctaggacttg acagaggacc caatacagag tgggtgctcc agaaatgtgg 780
acttggatgc atttcaccaa cagctattta catccaaata ataagctaat tataactctt 840
cttctagatg tacttttctc attgcaaaaa tcaccgtcaa aatttttaaa aacactaata 900
agcaagaaga aggaagttaa aattgcactc cttccagccc tagagagctt aatcaacttg 960
cttaggaaca cgctactaaa tggctaatct gagcaccaaa atctggccat ctggcttcag 1020
ggctcagcct cctaatggcc gcattatacc attgatcccg ccccatccac cccaacacac 1080
acactcccct gcacaaacaa attatgttag ctattaaaag gaattggaag agtggaaatg 1140
tgcaattaaa tctaccacaa atacaatgtt gcaatacgtg caatctatat ttttaaaatt 1200
tacagtattc agcattttcc tgtcttcaca cttgttccct ccgttattta cggcaatcat 1260
gacgaaaatc aacaaaatac agattgagag gcagcactga gaggtaatgt agaaagggaa 1320
tggcttttga gtcagaaaaa tgcgagtgtg gatcccagct aagcccctag atgtgttacc 1380
agagacgagc cgcaaaacat ttctcagtct tagtctcttg taaaatagag gtaataagaa 1440
acacttttca gtattttgtg acatgtagaa gtaagtgatg gtggcatgca tcacacttgg 1500
ttaatagtag gtcctgttgt taagtctcta atggcgatac cctatggctt ctccaaatgg 1560
tgaccttgcc aaattgtttt ccaaagcgac atgtggcttt tttctcccaa tccctcattt 1620
taactctcat ggtaatttaa cttttatatt tttattagat gcatttagta acttgcctca 1680
tagtcatttt cttggaaatt caatttcttc tccacagggt ctcttttgag attaaagaga 1740
gagaagtggc aaatttagga tgttagaata attttcattt aaaagtagat ccttgttttt 1800
attaccctat cattaatgtt ttctgttttc ctttatcagc gagttactgc tcatttgatt 1860
catattgcca aactgaactc tcttgttttc ttgcaagatg aaaggagaca accatgaatg 1920
agccactaga ctatttagca aatgcttctg atttccccga ttatgcagct gcttttggaa 1980

attgcactga tgaaaacatc ccactcaaga tgcactacct ccctgttatt tatggcatta 2040

tcttcctcgt gggatttcca ggcaatgcag tagtgatatc cacttacatt ttcaaaatga 2100

gaccttggaa gagcagcacc atcattatgc tgaacctggc ctgcacagat ctgctgtatc 2160

tgaccagcct ccccttcctg attcactact atgccagtgg cgaaaactgg atctttggag 2220

atttcatgtg taagtttatc cgctt 2245

<210> 5
<211> 1014
<212> DNA
<213> Homo sapiens

<400> 5
atgaatgagc cactagacta tttagcaaat gcttctgatt tcccccgatta tgcagctgct 60

tttggaaatt gcactgatga aaacatccca ctcaagatgc actacctccc tgttatttat 120

ggcattatct tcctcgtggg atttccaggc aatgcagtag tgatatccac ttacattttc 180

aaaatgagac cttggaagag cagcaccatc attatgctga acctggcctg cacagatctg 240

ctgtatctga ccagcctccc cttcctgatt cactactatg ccagtggcga aaactggatc 300

tttggagatt tcatgtgtaa gtttatccgc ttcagcttcc atttcaacct gtatagcagc 360

atcctcttcc tcacctgttt cagcatcttc cgctactgtg tgatcattca cccaatgagc 420

tgcttttcca ttcacaaaac tcgatgtgca gttgtagcct gtgctgtggt gtggatcatt 480

tcactggtag ctgtcattcc gatgaccttc ttgatcacat caaccaacag gaccaacaga 540

tcagcctgtc tcgacctcac cagttcggat gaactcaata ctattaagtg gtacaacctg 600

attttgactg caactacttt ctgcctcccc ttggtgatag tgacactttg ctataccacg 660

attatccaca ctctgaccca tggactgcaa actgacagct gccttaagca gaaagcacga 720

aggctaacca ttctgctact ccttgcattt tacgtatgtt ttttaccctt ccatatcttg 780

agggtcattc ggatcgaatc tcgcctgctt tcaatcagtt gttccattga gaatcagatc 840

catgaagctt acatcgtttc tagaccatta gctgctctga acacctttgg taacctgtta 900

ctatatgtgg tggtcagcga caactttcag caggctgtct gctcaacagt gagatgcaaa 960

gtaagcggga accttgagca agcaaagaaa attagttact caaacaaccc ttga 1014

<210> 6
<211> 373
<212> PRT
<213> Homo sapiens

<400> 6

Met Thr Glu Val Leu Trp Pro Ala Val Pro Asn Gly Thr Asp Ala Ala
1 5 10 15

```
Phe Leu Ala Gly Pro Gly Ser Ser Trp Gly Asn Ser Thr Val Ala Ser
            20                  25                  30

Thr Ala Ala Val Ser Ser Ser Phe Lys Cys Ala Leu Thr Lys Thr Gly
        35                  40                  45

Phe Gln Phe Tyr Tyr Leu Pro Ala Val Tyr Ile Leu Val Phe Ile Ile
    50                  55                  60

Gly Phe Leu Gly Asn Ser Val Ala Ile Trp Met Phe Val Phe His Met
65                  70                  75                  80

Lys Pro Trp Ser Gly Ile Ser Val Tyr Met Phe Asn Leu Ala Leu Ala
                85                  90                  95

Asp Phe Leu Tyr Val Leu Thr Leu Pro Ala Leu Ile Phe Tyr Tyr Phe
            100                 105                 110

Asn Lys Thr Asp Trp Ile Phe Gly Asp Ala Met Cys Lys Leu Gln Arg
        115                 120                 125

Phe Ile Phe His Val Asn Leu Tyr Gly Ser Ile Leu Phe Leu Thr Cys
    130                 135                 140

Ile Ser Ala His Arg Tyr Ser Gly Val Val Tyr Pro Leu Lys Ser Leu
145                 150                 155                 160

Gly Arg Leu Lys Lys Lys Asn Ala Ile Cys Ile Ser Val Leu Val Trp
                165                 170                 175

Leu Ile Val Val Val Ala Ile Ser Pro Ile Leu Phe Tyr Ser Gly Thr
            180                 185                 190

Gly Val Arg Lys Asn Lys Thr Ile Thr Cys Tyr Asp Thr Thr Ser Asp
        195                 200                 205

Glu Tyr Leu Arg Ser Tyr Phe Ile Tyr Ser Met Cys Thr Thr Val Ala
    210                 215                 220

Met Phe Cys Val Pro Leu Val Leu Ile Leu Gly Cys Tyr Gly Leu Ile
225                 230                 235                 240

Val Arg Ala Leu Ile Tyr Lys Asp Leu Asp Asn Ser Pro Leu Arg Arg
                245                 250                 255

Lys Ser Ile Tyr Leu Val Ile Ile Val Leu Thr Val Phe Ala Val Ser
            260                 265                 270
```

Tyr Ile Pro Phe His Val Met Lys Thr Met Asn Leu Arg Ala Arg Leu
275 280 285

Asp Phe Gln Thr Pro Ala Met Cys Ala Phe Asn Asp Arg Val Tyr Ala
290 295 300

Thr Tyr Gln Val Thr Arg Gly Leu Ala Ser Leu Asn Ser Cys Val Asp
305 310 315 320

Pro Ile Leu Tyr Phe Leu Ala Gly Asp Thr Phe Arg Arg Arg Leu Ser
325 330 335

Arg Ala Thr Arg Lys Ala Ser Arg Arg Ser Glu Ala Asn Leu Gln Ser
340 345 350

Lys Ser Glu Asp Met Thr Leu Asn Ile Leu Pro Glu Phe Lys Gln Asn
355 360 365

Gly Asp Thr Ser Leu
370

SEQUENCE LISTING

<110> Actimis Pharmaceuticals, Inc.

<120> REGULATION OF HUMAN P2Y1-LIKE G PROTEIN-COUPLED RECEPTOR

<130> LI0131 European

<150> US 60/224,989
<151> 2000-08-14

<160> 6

<170> PatentIn version 3.1

<210> 1
<211> 593
<212> DNA
<213> Homo sapiens

<400> 1

```
tggcttttga gtcagaaaaa tgcgagtgtg gatcccagct aagcccctag atgtgttacc      60
agagacgagc cgcaaaacat ttctcagtct tagtctcttg taaaatagag gtaataagaa     120
acacttttca gtattttgtg acatgtagaa gtaagtgatg gtggcatgca tcacacttgg     180
ttaatagtag gtcctgttgt taagtctcta atggcgatac cctatggctt ctccaaatgg     240
tgaccttgcc aaattgtttt ccaaagcgac atgtggcttt tttctcccaa tccctcattt     300
taactctcat ggtaatttaa cttttatatt tttattagat gcatttagta acttgcctca     360
tagtcatttt cttggaaatt caatttcttc tccacagggt ctcttttgag attaaagaga     420
gagaagtggc aaatttagga tgttagaata attttcattt aaaagtagat ccttgttttt     480
attaccctat cattaatgtt ttctgttttc ctttatcagc gagttactgc tcatttgatt     540
catattgcca aactgaactc tcttgttttc ttgcaagatg aaaggagaca acc            593
```

<210> 2
<211> 337
<212> PRT
<213> Homo sapiens

<400> 2

```
Met Asn Glu Pro Leu Asp Tyr Leu Ala Asn Ala Ser Asp Phe Pro Asp
1               5                   10                  15
```

```
Tyr Ala Ala Ala Phe Gly Asn Cys Thr Asp Glu Asn Ile Pro Leu Lys
            20                  25                  30

Met His Tyr Leu Pro Val Ile Tyr Gly Ile Ile Phe Leu Val Gly Phe
        35                  40                  45

Pro Gly Asn Ala Val Val Ile Ser Thr Tyr Ile Phe Lys Met Arg Pro
    50                  55                  60

Trp Lys Ser Ser Thr Ile Ile Met Leu Asn Leu Ala Cys Thr Asp Leu
65                  70                  75                  80

Leu Tyr Leu Thr Ser Leu Pro Phe Leu Ile His Tyr Tyr Ala Ser Gly
                85                  90                  95

Glu Asn Trp Ile Phe Gly Asp Phe Met Cys Lys Phe Ile Arg Phe Ser
            100                 105                 110

Phe His Phe Asn Leu Tyr Ser Ser Ile Leu Phe Leu Thr Cys Phe Ser
        115                 120                 125

Ile Phe Arg Tyr Cys Val Ile Ile His Pro Met Ser Cys Phe Ser Ile
    130                 135                 140

His Lys Thr Arg Cys Ala Val Val Ala Cys Ala Val Val Trp Ile Ile
145                 150                 155                 160

Ser Leu Val Ala Val Ile Pro Met Thr Phe Leu Ile Thr Ser Thr Asn
                165                 170                 175

Arg Thr Asn Arg Ser Ala Cys Leu Asp Leu Thr Ser Ser Asp Glu Leu
            180                 185                 190

Asn Thr Ile Lys Trp Tyr Asn Leu Ile Leu Thr Ala Thr Thr Phe Cys
        195                 200                 205

Leu Pro Leu Val Ile Val Thr Leu Cys Tyr Thr Thr Ile Ile His Thr
    210                 215                 220

Leu Thr His Gly Leu Gln Thr Asp Ser Cys Leu Lys Gln Lys Ala Arg
225                 230                 235                 240

Arg Leu Thr Ile Leu Leu Leu Leu Ala Phe Tyr Val Cys Phe Leu Pro
                245                 250                 255

Phe His Ile Leu Arg Val Ile Arg Ile Glu Ser Arg Leu Leu Ser Ile
            260                 265                 270

Ser Cys Ser Ile Glu Asn Gln Ile His Glu Ala Tyr Ile Val Ser Arg
        275                 280                 285

Pro Leu Ala Ala Leu Asn Thr Phe Gly Asn Leu Leu Leu Tyr Val Val
    290                 295                 300

Val Ser Asp Asn Phe Gln Gln Ala Val Cys Ser Thr Val Arg Cys Lys
305                 310                 315                 320
```

```
Val Ser Gly Asn Leu Glu Gln Ala Lys Lys Ile Ser Tyr Ser Asn Asn
                325                 330                 335

Pro


<210>  3
<211>  673
<212>  DNA
<213>  Homo sapiens


<400>  3
aatatttcat ttacttaacc aaaaacaaat acttgctgat actttaccta gcatcctaag      60
atgttcagga tgtctccctc aatggaactc ctggtaaata ctgtgtattc aagtaatcat     120
gtgccaaagc cagggcagag cttctagttc tttgcaatcc ctttattgag ctcctccact     180
ggggagatat aagaatggga tgcatgtata tcagcaaagt attcagacat agtattacaa     240
gctattggaa ctcagaggca tcttagagaa catctgttcc caccaactta ctatatatac     300
acggaaacca atttcttacc cttgccctag attgctcagt aaatttgtgc caagatagga     360
gaaaaccaat cttttcactc atcatttcat gcttctctgc actctgggcc tatttgtatt     420
gaaccattag acaattcaaa ccactacttg tatctttctt aatatttatt ttttacatct     480
cagagctcta caatttgttt ccttcaagct taactttgag attataaaac tgggtttagc     540
cagttctgta tattacttca agccagtaag atacccttga aataatccaa ggacgtccat     600
gcaaatagtt gaaattagta cctgcaatat atttggagta ttatgtcttt attgttgtta     660
aaaagttttt att                                                        673

<210>  4
<211>  2245
<212>  DNA
<213>  Homo sapiens


<220>
<221>  misc_feature
<222>  (1914)..(1916)
<223>  start codon


<220>
<221>  promoter
<222>  (1112)..(1117)
<223>  TATA box
```

<400> 4

aacaagactg ccacagtcgt atcctggctc acccctaact agcggtttga ctttgagcac 60
atcactgcct cctccgtgcc tcagtttcct cttctgttga atggggatga tcacagcact 120
acctcaccat ttgttgtgag gattaaatag attcaaatat gtaagacact tacagtaatg 180
ctttgtacac agaaggcact attatttttt attaatccct atttttcctt cccaacttca 240
tctcccaata tcccataacc atgctgattt ccttacaact cccccagacc tcctggacta 300
agtgagattt ggaagagtat actcagggca gtcaagaagg actgattctg ctaatttagg 360
atttgtcaag ttgggttagt ttgaaatata cctctgtact cctctcagga aatgctggat 420
agtgtagatt tagtatcaga taatagtaat aatggtggca gactctgatg tttcctcctt 480
cccttgtgat ctcagtagtc ttgtaccatt catacatcaa atgacttagc aactaacaat 540
cacgtaatat tttttaagct tttcttatgt gcccagtagt gagccaagag ttttttttcc 600
tatttttaac atactattga cagctccatg aggtgggttt atattttgtt aaaataattt 660
gccatgtcca cttccccagt agactgtgac agtggcatat acatctgtgt gtgttattca 720
gtttgtatct ctaggacttg acagaggacc caatacagag tgggtgctcc agaaatgtgg 780
acttggatgc atttcaccaa cagctattta catccaaata ataagctaat tataactctt 840
cttctagatg tacttttctc attgcaaaaa tcaccgtcaa aatttttaaa aacactaata 900
agcaagaaga aggaagttaa aattgcactc cttccagccc tagagagctt aatcaacttg 960
cttaggaaca cgctactaaa tggctaatct gagcaccaaa atctggccat ctggcttcag 1020
ggctcagcct cctaatggcc gcattatacc attgatcccg ccccatccac cccaacacac 1080
acactcccct gcacaaacaa attatgttag ctattaaaag gaattggaag agtggaaatg 1140
tgcaattaaa tctaccacaa atacaatgtt gcaatacgtg caatctatat ttttaaaatt 1200
tacagtattc agcattttcc tgtcttcaca cttgttccct ccgttattta cggcaatcat 1260
gacgaaaatc aacaaaatac agattgagag gcagcactga gaggtaatgt agaaagggaa 1320
tggcttttga gtcagaaaaa tgcgagtgtg gatcccagct aagcccctag atgtgttacc 1380
agagacgagc cgcaaaacat ttctcagtct tagtctcttg taaaatagag gtaataagaa 1440
acacttttca gtattttgtg acatgtagaa gtaagtgatg gtggcatgca tcacacttgg 1500
ttaatagtag gtcctgttgt taagtctcta atggcgatac cctatggctt ctccaaatgg 1560
tgaccttgcc aaattgtttt ccaaagcgac atgtggcttt tttctcccaa tccctcattt 1620
taactctcat ggtaatttaa cttttatatt tttattagat gcatttagta acttgcctca 1680
tagtcatttt cttggaaatt caatttcttc tccacagggt ctcttttgag attaaagaga 1740
gagaagtggc aaatttagga tgttagaata attttcattt aaaagtagat ccttgttttt 1800
attaccctat cattaatgtt ttctgttttc ctttatcagc gagttactgc tcatttgatt 1860
catattgcca aactgaactc tcttgttttc ttgcaagatg aaaggagaca accatgaatg 1920
agccactaga ctatttagca aatgcttctg atttccccga ttatgcagct gcttttggaa 1980
attgcactga tgaaaacatc ccactcaaga tgcactacct ccctgttatt tatggcatta 2040
tcttcctcgt gggatttcca ggcaatgcag tagtgatatc cacttacatt ttcaaaatga 2100
gaccttggaa gagcagcacc atcattatgc tgaacctggc ctgcacagat ctgctgtatc 2160
tgaccagcct ccccttcctg attcactact atgccagtgg cgaaaactgg atctttggag 2220
atttcatgtg taagtttatc cgctt 2245

<210> 5

<211> 1014

<212> DNA

<213> Homo sapiens

<400> 5

```
atgaatgagc cactagacta tttagcaaat gcttctgatt tccccgatta tgcagctgct      60
tttggaaatt gcactgatga aaacatccca ctcaagatgc actacctccc tgttatttat     120
ggcattatct tcctcgtggg atttccaggc aatgcagtag tgatatccac ttacattttc     180
aaaatgagac cttggaagag cagcaccatc attatgctga acctggcctg cacagatctg     240
ctgtatctga ccagcctccc cttcctgatt cactactatg ccagtggcga aaactggatc     300
tttggagatt tcatgtgtaa gtttatccgc ttcagcttcc atttcaacct gtatagcagc     360
atcctcttcc tcacctgttt cagcatcttc cgctactgtg tgatcattca cccaatgagc     420
tgcttttcca ttcacaaaac tcgatgtgca gttgtagcct gtgctgtggt gtggatcatt     480
tcactggtag ctgtcattcc gatgaccttc ttgatcacat caaccaacag gaccaacaga     540
tcagcctgtc tcgacctcac cagttcggat gaactcaata ctattaagtg gtacaacctg     600
attttgactg caactacttt ctgcctcccc ttggtgatag tgacactttg ctataccacg     660
attatccaca ctctgaccca tggactgcaa actgacagct gccttaagca gaaagcacga     720
aggctaacca ttctgctact ccttgcattt tacgtatgtt ttttaccctt ccatatcttg     780
agggtcattc ggatcgaatc tcgcctgctt tcaatcagtt gttccattga gaatcagatc     840
catgaagctt acatcgtttc tagaccatta gctgctctga acacctttgg taacctgtta     900
ctatatgtgg tggtcagcga caactttcag caggctgtct gctcaacagt gagatgcaaa     960
gtaagcggga accttgagca agcaaagaaa attagttact caaacaaccc ttga          1014
```

<210> 6

<211> 373

<212> PRT

<213> Homo sapiens

<400> 6

```
Met Thr Glu Val Leu Trp Pro Ala Val Pro Asn Gly Thr Asp Ala Ala
1               5                   10                  15

Phe Leu Ala Gly Pro Gly Ser Ser Trp Gly Asn Ser Thr Val Ala Ser
            20                  25                  30

Thr Ala Ala Val Ser Ser Ser Phe Lys Cys Ala Leu Thr Lys Thr Gly
        35                  40                  45

Phe Gln Phe Tyr Tyr Leu Pro Ala Val Tyr Ile Leu Val Phe Ile Ile
    50                  55                  60
```

```
Gly Phe Leu Gly Asn Ser Val Ala Ile Trp Met Phe Val Phe His Met
65                  70                  75                  80

Lys Pro Trp Ser Gly Ile Ser Val Tyr Met Phe Asn Leu Ala Leu Ala
                85                  90                  95

Asp Phe Leu Tyr Val Leu Thr Leu Pro Ala Leu Ile Phe Tyr Tyr Phe
            100                 105                 110

Asn Lys Thr Asp Trp Ile Phe Gly Asp Ala Met Cys Lys Leu Gln Arg
        115                 120                 125

Phe Ile Phe His Val Asn Leu Tyr Gly Ser Ile Leu Phe Leu Thr Cys
    130                 135                 140

Ile Ser Ala His Arg Tyr Ser Gly Val Val Tyr Pro Leu Lys Ser Leu
145                 150                 155                 160

Gly Arg Leu Lys Lys Lys Asn Ala Ile Cys Ile Ser Val Leu Val Trp
                165                 170                 175

Leu Ile Val Val Val Ala Ile Ser Pro Ile Leu Phe Tyr Ser Gly Thr
            180                 185                 190

Gly Val Arg Lys Asn Lys Thr Ile Thr Cys Tyr Asp Thr Thr Ser Asp
        195                 200                 205

Glu Tyr Leu Arg Ser Tyr Phe Ile Tyr Ser Met Cys Thr Thr Val Ala
    210                 215                 220

Met Phe Cys Val Pro Leu Val Leu Ile Leu Gly Cys Tyr Gly Leu Ile
225                 230                 235                 240

Val Arg Ala Leu Ile Tyr Lys Asp Leu Asp Asn Ser Pro Leu Arg Arg
                245                 250                 255

Lys Ser Ile Tyr Leu Val Ile Ile Val Leu Thr Val Phe Ala Val Ser
            260                 265                 270

Tyr Ile Pro Phe His Val Met Lys Thr Met Asn Leu Arg Ala Arg Leu
        275                 280                 285

Asp Phe Gln Thr Pro Ala Met Cys Ala Phe Asn Asp Arg Val Tyr Ala
    290                 295                 300

Thr Tyr Gln Val Thr Arg Gly Leu Ala Ser Leu Asn Ser Cys Val Asp
305                 310                 315                 320

Pro Ile Leu Tyr Phe Leu Ala Gly Asp Thr Phe Arg Arg Arg Leu Ser
                325                 330                 335

Arg Ala Thr Arg Lys Ala Ser Arg Arg Ser Glu Ala Asn Leu Gln Ser
            340                 345                 350

Lys Ser Glu Asp Met Thr Leu Asn Ile Leu Pro Glu Phe Lys Gln Asn
        355                 360                 365
```

```
Gly Asp Thr Ser Leu
    370
```